(19) **Europäisches Patentamt / European Patent Office / Office européen des brevets**

(11) **EP 3 978 110 A1**

(12) **EUROPEAN PATENT APPLICATION**
published in accordance with Art. 153(4) EPC

(43) Date of publication:
06.04.2022 Bulletin 2022/14

(21) Application number: 20812766.2

(22) Date of filing: 27.05.2020

(51) International Patent Classification (IPC):
$B01J\ 20/08$ (2006.01)    $B01J\ 20/14$ (2006.01)
$B01J\ 20/16$ (2006.01)    $B01D\ 15/22$ (2006.01)
$G01N\ 3/02$ (2006.01)

(52) Cooperative Patent Classification (CPC):
B01D 15/22; B01J 20/08; B01J 20/14; B01J 20/16;
G01N 3/02

(86) International application number:
PCT/CN2020/092590

(87) International publication number:
WO 2020/238953 (03.12.2020 Gazette 2020/49)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 31.05.2019  CN 201910472990
31.05.2019  CN 201910472985
31.05.2019  CN 201910471632

(71) Applicants:
• **China Petroleum & Chemical Corporation**
**Beijing 100728 (CN)**

• **Research Institute Of Petroleum Processing,**
**Sinopec**
**Beijing 100083 (CN)**

(72) Inventors:
• **XU, Guangtong**
**Beijing 100083 (CN)**
• **LIU, Zelong**
**Beijing 100083 (CN)**
• **SHI, Yanqiang**
**Beijing 100083 (CN)**
• **WANG, Naixin**
**Beijing 100083 (CN)**

(74) Representative: **Hoffmann Eitle**
**Patent- und Rechtsanwälte PartmbB**
**Arabellastraße 30**
**81925 München (DE)**

(54) **REVERSIBLE ENRICHMENT MATERIAL, PREPARATION THEREFOR, AND APPLICATION THEREOF**

(57) Disclosed is a reversible enrichment material, its preparation and application thereof, wherein the reversible enrichment material comprises: an inorganic carrier; and an active metal salt, a first metal salt promoter and a second metal salt promoter supported on the inorganic carrier; wherein the active metal salt is selected from the group consisting of a soluble silver salt, a soluble copper salt, or a combination thereof, the first metal salt promoter is one or more selected from the group consisting of soluble salts of Group IA, Group IIA and Group IIIA metals, and the second metal salt promoter is one or more selected from the group consisting of soluble salts of transition metals other than Group IB metals. The reversible enrichment material can realize effective separation of saturated hydrocarbon from unsaturated hydrocarbon and has good reversibility.

Fig. 1

**Description**

Cross Reference to Related Applications

The present applicantion claims the priority of a patent application No. 201910471632.9, titled "a method for separating and analyzing hydrocarbon components of hydrocarbon fuels", filed on May 31, 2019 before the Chinese Patent Office; the priority of a patent application No. 201910472985.0, titled "reversible enrichment material for aromatic component in hydrocarbon fuels and preparation thereof", filed on May 31, 2019 before the Chinese Patent Office; and the priority of a patent application No. 201910472990.1, titled "a packed column and a system for separating and analyzing hydrocarbon components of hydrocarbon fuels", filed on May 31, 2019 before the Chinese Patent Office, the contents of which are incorporated herein by reference in their entirety.

Technical Field

The present application relates to the field of adsorption materials, particularly to a reversible enrichment material suitable for use in the separation of saturated hydrocarbons from unsaturated hydrocarbons (also referred to as "non-saturated hydrocarbons"), its preparation and application thereof.

Background Art

Since the hydrocarbon composition of hydrocarbon fuel is closely related to its combustion performance and service performance, and is related to the environmental problems which are increasingly regarded as important, how to optimize the hydrocarbon composition of hydrocarbon fuel, improve the combustion performance thereof, and reduce the emission of harmful substances is a major concern in the petrochemical industry. Currently, the three major refined oil products (gasoline, aviation fuel and diesel oil) have different requirements on the hydrocarbon composition of hydrocarbon fuels due to differences of working principles of engines, working environments and the like; for gasoline, more isoparaffins and less olefins and aromatics are desired, for aviation fuel, more naphthenes is desired, while the content of aromatics should be strictly controlled, and for diesel oil, more n-paraffins and less branched isoparaffins and n-olefins are desired, while the content of polycyclic aromatic hydrocarbons with short side chains should be strictly controlled. Therefore, detailed hydrocarbon composition analysis is of great significance to product quality control of hydrocarbon fuels, related process development and catalyst development.

In current detailed hydrocarbon composition analysis of hydrocarbon fuels, especially aviation fuel and diesel oil, the devices used mainly include standard wall glass tube adsorption column, high performance liquid chromatograph and gas chromatograph-mass spectrometer. The standard wall glass tube adsorption column is used in the fluorescent indicator adsorption method GB/T 11132-2008 (corresponding to ASTM D1319), and its application in the hydrocarbon composition analysis of hydrocarbon fuels involoes the preparation of silica gel for the adsorption column, the filling of the adsorption column, the addition of a color developing agent, the leaching in the separation process, the measurement after the leaching and the like, so that the analysis period is long, the result reproducibility tolerance range is wide, the precision is poor, and this device can only give the content information of saturated hydrocarbon, olefin and aromatic hydrocarbon of the hydrocarbon fuel while cannot give detailed hydrocarbon composition information; the high performance liquid chromatograph is used in SH/T0806-2008 and ASTM D6379-11 and other methods for determining the types of aromatic hydrocarbons of aviation fuel and middle distillate, and when compared with a standard wall glass tube adsorption column, this device has the advantages that the separation speed and the separation efficiency are significantly improved, the content information of saturated hydrocarbon, monocyclic aromatic hydrocarbon and bicyclic aromatic hydrocarbon can be given, but due to the fact that a differential refraction detector is used, the difference responses between different types of aromatic hydrocarbons is large, and thus the quantitative error of different types of aromatic hydrocarbons is large, and detailed hydrocarbon composition of saturated hydrocarbons and olefin information cannot be given.

With the increasing maturity of gas chromatography-mass spectrometry technology, gas chromatograph-mass spectrometer is widely used in the field of petrochemical industry, but due to insufficient capacity of the chromatographic column, online separation and detailed hydrocarbon composition analysis of aviation fuels and middle distillates by gas chromatography-mass spectrometry cannot be realized. This device is used in the SH/T0606-2005 method for the analysis of detailed hydrocarbon composition of hydrocarbon fuels, but in the gas chromatograph-mass spectrometer used in the SH/T0606-2005 method, the chromatographic column in the oven is an empty quartz capillary column which does not have separation capacity, the separation is accomplished by the solid phase extraction separation performed before a sample enters the gas chromatograph-mass spectrometer, where the hydrocarbon fuel is separated into saturated hydrocarbons and aromatic hydrocarbons which then enter the gas chromatograph-mass spectrometer separately for analysis. When using this device, the time for analyzing the detailed hydrocarbon composition of hydrocarbon fuels is long, the olefin component is entrained in the saturated hydrocarbon component during the solid phase extraction separation process, and the gas chromatograph-mass spectrometer cannot distinguish naphthenes from olefins in the hydrocarbon fuel.

There remains an urgent need in the art for new material, device and method suitable for hydrocarbon component separation and analysis of hydrocarbon-based materials, particularly hydrocarbon fuels, containing saturated and unsaturated hydrocarbons.

Summary of the Invention

An object of the present application is to provide a reversible enrichment material suitable for use in the separation of saturated hydrocarbons from unsaturated hydrocarbons, particularly in the separation of aromatic hydrocarbons from saturated hydrocarbons in hydrocarbon fuels, its preparation and application thereof.

To achieve the above object, in an aspect, the present application provides a reversible enrichment material comprising:

an inorganic carrier; and
an active metal salt, a first metal salt promoter and a second metal salt promoter that are supported on the inorganic carrier;
wherein the active metal salt is selected from the group consisting of soluble silver salts, soluble copper salts, or combinations thereof, the first metal salt promoter is one or more selected from the group consisting of soluble salts of Group IA, Group IIA, and Group IIIA metals, and the second metal salt promoter is one or more selected from the group consisting of soluble salts of transition metals other than Group IB metals,
in the reversible enrichment material, based on the total amount of the inorganic carrier, the active metal salt is present in an amount of about 0.1-80% by mass, preferably about 0.5-50% by mass; the first metal salt promoter is present in an amount of about 0.1-80% by mass, preferably about 0.2-40% by mass; the second metal salt promoter is present in an amount of about 0.5-80% by mass, preferably about 0.5-30% by mass.

Preferably, the inorganic carrier has been subjected to a modification comprising sequentially a calcination treatment, an acid-washing and/or alkali-washing treatment and a drying treatment.

In another aspect, the present application provides a method for preparing the reversible enrichment material, comprising the steps of:

1) providing an inorganic carrier;
2) loading the active metal salt, the first metal salt promoter and the second metal salt promoter onto the inorganic carrier; and
3) optionally, drying the material obtained in step 2).

Preferably, the step 1) further comprises subjecting an inorganic starting material to a modification to obtain the inorganic carrier, wherein the modification comprises sequentially a calcination treatment, an acid-washing and/or alkali-washing treatment, and a drying treatment.

In yet another aspect, the present application provides a packed column comprising a column tube and an enrichment filler packed in the column tube, wherein the enrichment filler is a reversible enrichment material according to the present application.

Preferably, the inner surface of the column tube has been subjected to a passivation treatment, and the passivation treatment includes an alkali treatment and an acid treatment.

In yet another aspect, the present application provides a separation and analysis system comprising a packed column according to the present application.

Preferably, the system further comprises a gas chromatograph, wherein the packed column is connected between a sample inlet and a detector of the gas chromatograph.

Preferably, the system further comprises a gas chromatograph-mass spectrometer, wherein the gas chromatograph-mass spectrometer comprises a sample inlet and an analysis module, and the two ends of the packed column are respectively communicated with the sample inlet and an inlet of the analysis module;
the analysis module comprises a mass spectrometry unit and a gas chromatography unit, and inlets of the mass spectrometry unit and the gas chromatography unit are respectively communicated with the inlet of the analysis module through a spilitter.

In yet another aspect, the present application provides a method for performing hydrocarbon component separation and analysis on a hydrocarbon-based material comprising saturated hydrocarbon and unsaturated hydrocarbon, comprising the steps of:

S1) feeding the hydrocarbon-based material into a packed column from a first end thereof by purging with a carrier gas under a first separation condition for enrichment and separation, to obtain a first effluent comprising saturated hydrocarbon from a second end of the packed column;

S2) purging the packed column from the second end thereof with the carrier gas under a second separation condition, to obtain a second effluent comprising unsaturated hydrocarbon from the first end of the packed column;

wherein the packed column is a packed column according to the present application and the temperature of the first separation condition is lower than the temperature of the second separation condition.

Preferably, the method further comprises: S3) after step S1, passing the first effluent to a gas chromatograph and a mass spectrometer to perform separation and analysis on saturated hydrocarbon component in the first effluent; and/or,

S4) after step S2, passing the second effluent to a gas chromatograph and a mass spectrometer to perform separation and analysis on unsaturated hydrocarbon component in the second effluent.

The reversible enrichment material according to the present application has a reversible enrichment capacity for unsaturated hydrocarbons, especially aromatic hydrocarbons, is capable of realizing an effective separation of saturated hydrocarbons from unsaturated hydrocarbons, especially aromatic hydrocarbons, has good saturated hydrocarbon passing capacity and strong unsaturated hydrocarbon enrichment capacity, and thus can be used to realize reversible enrichment of unsaturated hydrocarbon component in hydrocarbon-based materials. The enrichment material can be used for hydrocarbon composition analysis of petroleum products, especially the hydrocarbon composition analysis of aviation fuel and diesel oil, allowing a convenient and quick acquirement of accurate hydrocarbon composition data of the petroleum products.

After being coupled with a gas chromatograph or a gas chromatograph-mass spectrometer, the packed column according to the present application can be used for the online separation of saturated hydrocarbon component from unsaturated hydrocarbon component in hydrocarbon-based materials. The system according to the present application can realize a stable coupling of packed column system and gas chromatograph-mass spectrometer, and therefore can realize an online separation of saturated hydrocarbon and unsaturated hydrocarbon in hydrocarbon-based materials, especially middle distillates such as aviation fuel, diesel oil and the like, improve the repeatability and accuracy of the separation and analysis, accurately provide the olefin and naphthene information of a hydrocarbon fuel, and greatly save the time for detailed hydrocarbon composition analysis of middle distillates such as aviation fuel, diesel oil and the like. The system can be used for quick and accurate determination of detailed hydrocarbon composition of hydrocarbon fuels, especially aviation fuels and diesel oils.

The method according to the present application can realize a quick online separation of saturated hydrocarbon and unsaturated hydrocarbon in hydrocarbon-based materials, especially middle distillates, such as aviation fuel, diesel oil and the like, improve the repeatability, accuracy and efficiency of detailed hydrocarbon composition separation and analysis of hydrocarbon-based materials, solve the problem of existing methods that can not distinguish olefin from naphthene in hydrocarbon-based materials, and greatly save the time for detailed hydrocarbon composition analysis of hydrocarbon-based materials, especially middle distillates such as aviation fuel, diesel oil and the like, while providing more detailed hydrocarbon composition information.

Additional characteristics and advantages of the present application will be described in detail in the Detailed Description section below.

Brief Description of the Drawings

The drawings, forming a part of the present description, are provided to help the understanding of the present application, and should not be considered to be limiting. The present application can be interpreted with reference to the drawings in combination with the detailed description hereinbelow. In the drawings:

Fig. 1 is a schematic diagram of an embodiment of the system and method according to the present application;
Fig. 2 is a schematic diagram of another embodiment of the system and method according to the present application;
Figs. 3-19 show experimental results of the separation and analysis of unsaturated hydrocarbons and saturated hydrocarbons performed in Examples 11-10 to 11-26 of the present application, respectively;
Figs. 20-32 show experimental results of the separation and analysis of unsaturated hydrocarbons and saturated hydrocarbons performed in Examples III-1 to 111-13 of the present application, respectively.

Detailed Description of the Invention

The present application will be further described hereinafter in detail with reference to particular embodiments thereof and the accompanying drawings. It should be noted that the particular embodiments of the present application are provided for illustration purpose only, and are not intended to be limiting in any manner.

Any specific numerical value, including the endpoints of a numerical range, described in the context of the present application is not restricted to the exact value thereof, but should be interpreted to further encompass all values close to said exact value. Moreover, regarding any numerical range described herein, arbitrary combinations can be made between the endpoints of the range, between each endpoint and any specific value within the range, or between any two specific values within the range, to provide one or more new numerical range(s), where said new numerical range(s) should also be deemed to have been specifically described in the present application.

Unless otherwise stated, the terms used herein have the same meaning as commonly understood by the person skilled in the art; and if the terms are defined herein and their definitions are different from the ordinary understanding in the art, the definition provided herein shall prevail.

In the context of the present application, in addition to those matters explicitly stated, any matter or matters not mentioned are considered to be the same as those known in the art without any change. Moreover, any of the embodiments described herein can be freely combined with another one or more embodiments described herein, and the technical solutions or ideas thus obtained are considered as part of the original disclosure or original description of the present application, and should not be considered to be a new matter that has not been disclosed or anticipated herein, unless it is clear to the person skilled in the art that such a combination is obviously unreasonable.

All publications, patent applications, patents, and other references cited herein are hereby incorporated by reference in their entirety.

In the present application, the term "hydrocarbon-based material" refers to a material comprising hydrocarbons as a main component, for example, a material having a hydrocarbon content of about 80 wt% or more, preferably about 90 wt% or more, more preferably about 95 wt% or more, especially preferably about 99 wt% or more, and most preferably consisting essentially of hydrocarbons.

In the present application, the term "hydrocarbon fuel" refers to a liquid hydrocarbon-based material typically used in engines, which comprises liquid hydrocarbons having 5 to 25 carbon atoms or mixtures thereof as a main component, and may additionally comprisies a minor amount (e.g., less than about 10 wt%, or less than about 5 wt%) of other materials, such as sulfur-containing compounds.

In the present application, the term "enrichment material" refers to a material that is capable of adsorbing a target component in a multi-component mixture under certain conditions so that the concentration of the target component on said material is higher than that in the bulk phase of the multi-component mixture.

In the present application, the term "reversible" means that the adsorption of the target component by the enrichment material is reversible, i.e. the adsorbed target component can be desorbed from the enrichment material by adjusting the operating conditions, and during the adsorption and desorption process, the type, composition and amount of the target component is essentially unchanged.

In a first aspect, the present application provides a reversible enrichment material suitable for use in the separation of saturated hydrocarbons from unsaturated hydrocarbons, comprising:

an inorganic carrier; and
an active metal salt, a first metal salt promoter and a second metal salt promoter that are supported on the inorganic carrier;
wherein the active metal salt is selected from the group consisting of a soluble silver salt, a soluble copper salt, or a combination thereof, the first metal salt promoter is one or more selected from the group consisting of soluble salts of Group IA, Group IIA and Group IIIA metals, and the second metal salt promoter is one or more selected from the group consisting of soluble salts of transition metals other than Group IB metals.

In a preferred embodiment, the inorganic carrier has been subjected to a modification comprising a calcination treatment, an acid-washing and/or alkali-washing treatment, and a drying treatment performed sequentially.

By loading the active metal salt and two specific types of metal salt promoters on the inorganic carrier, the enrichment material according to the present application can effectively improve the enriching capacity of a single active metal salt on unsaturated hydrocarbons, especially aromatic hydrocarbon component, through the synergistic effect of the metal promoters, and thus is suitable for use in the column for separating saturated hydrocarbon component from unsaturated hydrocarbon component in gas chromatography or gas chromatography-mass spectrometry. Compared with other types of enrichment materials, the reversible enrichment material according to the present application has better passing capacity for saturated hydrocarbons and better enriching capacity for unsaturated hydrocarbons, especially aromatic hydrocarbons. The reversible enrichment material has obvious advantages in passing capacity for saturated hydrocarbons and enriching capacity for low-carbon-number aromatic hydrocarbons in aviation fuel, can stably enrich low-carbon-number aromatic hydrocarbon component (such as ethylbenzene) at a temperature as high as 170 °C, has no retainability for long-chain alkane (such as n-hexadecane) in saturated hydrocarbons, can realize a complete separation of saturated hydrocarbons and low-carbon-number aromatic hydrocarbons in aviation fuel, can realize a complete desorption of aromatic hydrocarbons (such as 1-methylnaphthalene) adsorbed by the enrichment material at a temperature of 208 °C, can be well used for hydrocarbon composition analysis of aviation fuel, and can improve the accuracy and repeatability of hydrocarbon composition analysis of aviation fuel.

According to the present application, the inorganic carrier may be any porous inorganic material well known to those skilled in the art as a carrier, for example, the inorganic carrier may be one or more selected from the group consisting of diatomite carrier, alumina carrier, titania carrier, zirconia carrier, mesoporous molecular sieve carrier, amorphous silica-alumina carrier, silica gel carrier, and controlled porous glass carrier, preferably a diatomite carrier.

In a preferred embodiment, the inorganic carrier can be obtained by subjecting an inorganic starting material to a modifiaction, for example, by subjecting the inorganic starting material to a calcination treatment, an acid-washing and/or alkali-washing treatment, and a drying treatment sequentially.

In the present application, the inorganic starting materials may be those well known to those skilled in the art and are commercially available, and may be, for example, one or more selected from the group consisting of a diatomite starting material, alumina, titania, zirconia, mesoporous molecular sieves, amorphous silica-alumina, silica gel, and controlled porous glass. According to the application, through the modification, the passing capacity of the inorganic starting material for long-chain alkanes can be improved, so that the performance of the enrichment material for separating saturated hydrocarbons from unsaturated hydrocarbons can be further improved.

In a preferred embodiment, the conditions of the calcination treatment may include: a temperature of about 500-950 °C, preferably about 850-950 °C, and a period of about 4-16 hours; the conditions of the acid-washing treatment may include: a temperature of about 100-200 °C, a period of about 10-40 hours, and a mass ratio of the starting material to be treated on a dry basis to the acid solution of about 1 : (1-20), wherein the acid solution used for the acid-washing treatment is one or more selected from the group consisting of nitric acid, hydrochloric acid and sulfuric acid, and the concentration of the acid solution used for the acid-washing treatment is about 1-90% by mass; the conditions of the alkali-washing treatment may include: a temperature of about 100-200 °C, a period of about 10-40 hours, and a mass ratio of the starting material to be treated on a dry basis to the alkali solution of about 1 (1-20), wherein the alkali solution used for the alkali-washing treatment is one or more selected from the group consisting of sodium hydroxide solution, potassium hydroxide solution and ammonia water, and the concentration of the alkali solution used for the alkali-washing treatment is about 1-90% by mass, preferably about 10-50% by mass; the conditions of the drying treatment may include: a temperature of about 100-200 °C, preferably about 150-180 °C, and a period of about 3-6 hours.

In a preferred embodiment, the inorganic carrier may have a specific surface area in a range of from about 1 $m^2/g$ to about 600 $m^2/g$, preferably from about 1 $m^2/g$ to about 370 $m^2/g$, more preferably from about 1 $m^2/g$ to about 100 $m^2/g$, still more preferably from about 2 $m^2/g$ to about 50 $m^2/g$, a pore size in a range of from about 1 nm to about 1000 nm, preferably from about 1 nm to about 500 nm, and a particle size in a range of from about 80 $\mu$m to about 800 $\mu$m, preferably from about 100 $\mu$m to about 600 $\mu$m.

In a preferred embodiment, based on the total amount of the inorganic carrier, in the reversible enrichment material, the active metal salt may be present in an amount of about 0.1-80% by mass, preferably about 0.5-50% by mass, more preferably about 1-30% by mass or about 0.8-15% by mass; the first metal salt promoter may be present in an amount of about 0.1-80% by mass, preferably about 0.2-40% by mass; the second metal salt promoter may be present in an amount of about 0.5-80% by mass, preferably about 0.5-30% by mass or about 0.7-12% by mass. Further, the ratio of the mass content of the first metal salt promoter to the mass content of the second metal salt promoter, calculated as metal oxide, may be about 1 : (0.05-50), for example about 1 : (0.05-0.15) or about 1 : (0.5-50), preferably about 1 : (0.5-40).

In a preferred embodiment, the soluble silver salt may be silver nitrate and the soluble copper salt may be selected from the group consisting of copper nitrate, copper sulfate or a combination thereof, preferably copper nitrate.

In a preferred embodiment, in the first metal salt promoter, the Group IA metal may be one or more selected from the group consisting of lithium, sodium and potassium, the Group IIA metal may be one or more selected from the group consisting of beryllium, magnesium, calcium and barium, the Group IIIA metal may be selected from the group consisting of aluminum, gallium or a combination thereof, and in the second metal salt promoter, the transition metal may be one or more selected from the group consisting of zinc (IIB), cadmium (IIB), vanadium, chromium (VIB), molybdenum (VIB), tungsten (VIB), manganese (VIII), iron (VIII), cobalt (VIII), nickel (VIII), ruthenium (VIII), platinum (VIII), rhodium (VIII) and palladium (VIII). In a further preferred embodiment, the metal salt promoters may be a soluble nitrate or sulphate of the metal promoter.

In a particularly preferred embodiment, the reversible enrichment material comprises one first metal salt promoter and two second metal salt promoters, the first metal salt promoter may be selected from the group consisting of soluble salts of Group IA, Group IIA and Group IIIA metals, preferably from soluble salts of Group IA-IIA metals; the two second metal salt promoters may be selected from the group consisting of soluble salts of transition metals other than Group IB metals. In a further preferred embodiment, the second metal salt promoter may comprise two soluble transition metal salts selected from the group consisting of soluble salts of Group IIB, group VIB and group VIII transition metals, preferably a first soluble transition metal salt selected from soluble salts of Group VIII transition metals and a second soluble transition metal salt selected from soluble salts of Group IIB and Group VIB transition metals. Preferably, the first metal salt promoter may be present in an amount of about 0.1-80% by mass, preferably about 0.2-40% by mass, for example about 0.5-20% by mass or about 0.3-18% by mass, calculated as metal oxide; the two soluble transition metal salts may be present in an amount of about 0.5-80% by mass (preferably about 0.5-30% by mass, for example, about 1.5-22% by mass or about 0.8-16% by mass) and about 0.5-80% by mass (preferably about 0.5-30% by mass, for example, about 1.2-25% by mass or about 0.75-20% by mass), respectively, calculated as metal oxide. Further preferably, the ratio of the mass content of the first soluble transition metal salt to the mass content of the second soluble transition metal salt, calculated as metal oxide, may be about 1 : (0.05-40), for example, about 1 : (0.05-0.15) or about 1 : (0.5-40), preferably about 1 : (0.5-30). In a still further preferred embodiment, the first soluble transition metal salt may be selected from soluble salts of Group VIII transition metals and the second soluble transition metal salt may be selected from soluble salts of Group IIB transition metals, wherein the ratio of the mass content of the first soluble transition metal salt to the mass content of the second soluble transition metal salt, calculated as metal oxide, may be about 1 : (0.5-30), for example about 1 : (0.7-30), about 1 : (1.2-26) or about 1 : (1.5-25), preferably about 1 : (3-20) or about 1 : (2.5-15).

In a second aspect, there is provided a method for preparing a reversible enrichment material according to the present application, comprising the steps of:

1) providing an inorganic carrier;
2) loading the active metal salt, the first metal salt promoter and the second metal salt promoter onto the inorganic carrier; and
3) optionally, drying the material obtained in step 2).

In a preferred embodiment, the step 1) may further comprise subjecting an inorganic starting material to a modification to obtain the inorganic carrier, wherein the modification comprises a calcination treatment, an acid-washing and/or alkali-washing treatment, and a drying treatment performed sequentially. The details of the inorganic carrier, the inorganic starting material, and the modification are as described above and are omitted here for brevity.

According to the application, the drying step in the preparation of the reversible enrichment material and the drying treatment in the modification of the inorganic carrier may be the same or different, and can be selected by those skilled in the art in view of the need.

The reversible enrichment material is particularly suitable for use as a filler in a separation column used in gas chromatography analysis of aromatic hydrocarbon content in aviation fuel and detailed hydrocarbon composition analysis of aviation fuel by gas chromatography-mass spectrometry. The enrichment material of the present application can be loaded into a chromatographic column to make a separation chromatographic column required for gas chromatography or gas chromatography-mass spectrometry analysis of the composition of aviation fuel, to separate saturated hydrocarbon component from aromatic hydrocarbon component in aviation fuel, and the separation process may be as follows: after passing through a separation column at a lower temperature, saturated hydrocarbons in the aviation fuel are fed into a gas chromatograph or a gas chromatograph-mass spectrometer for analyzing the components thereof and their contents, and then the temperature of the column box is raised and the separation column is subjected to back flushing, so that aromatic hydrocarbons are fed into the gas chromatograph or gas chromatograph-mass spectrometer for analyzing the components thereof and their contents, so that hydrocarbon composition or detailed hydrocarbon composition analysis data of the aviation fuel is conveniently and quickly obtained.

In a third aspect, the present application provides a packed column suitable for use in the separation of saturated hydrocarbons from unsaturated hydrocarbons, comprising a column tube and an enrichment filler packed in the column tube, wherein the enrichment filler is a reversible enrichment material according to the present application.

The packed column can be stably coupled with a gas chromatograph or a gas chromatograph-mass spectrometer, and allows a full passing of saturated hydrocarbons and a reversible enrichment of unsaturated hydrocarbons in hydrocarbon-based materials, so that an online separation of unsaturated hydrocarbon component from saturated hydrocarbon component in the hydrocarbon-based materials can be realized. The packed column is suitable for use in the online separation of unsaturated hydrocarbons from saturated hydrocarbons in hydrocarbon-based materials, especially hydrocarbon fuels, by gas chromatography or gas chromatography-mass spectrometry, can significantly improve the repeatability and accuracy of hydrocarbon composition analysis and detailed hydrocarbon composition analysis of hydrocarbon fuels, and is simple, convenient and quick to operate.

According to the present application, to avoid the influence of impurities on the surface of the column tube on the analysis result, in a preferred embodiment, the inner surface of the column tube has been subjected to a passivation treatment. Further preferably, the passivation treatment may include an alkali treatment and an acid treatment. For example, the passivation treatment may include: soaking the column tube in an alkali solution having a concentration of 5-20% by mass at 20-100 °C for 30-120 minutes, washing with water to neutrality, soaking in an acid solution having a concentration of 5-20% by mass at 30-120 °C for 30-100 minutes, washing with water to neutrality, and drying.

In a preferred embodiment, the column tube may be U-shaped or spiral-shaped.

In a preferred embodiment, the column tube may have an inner diameter of about 0.1-12 mm, preferably about 1-6 mm, more preferably 1.5-4.5 mm; a length of about 20-5000 mm, preferably about 50-1000 mm, and more preferably 100-600 mm.

In a preferred embodiment, the column tube may be at least one selected from the group consisting of a stainless steel column tube, a glass column tube, and a quartz column tube, and is more preferably a stainless steel column tube.

In a preferred embodiment, the packed column may be used at a temperature ranging from about 80 °C to about 400 °C, more preferably from about 100 °C to about 350 °C, and even more preferably from about 110 °C to about 300 °C.

In a preferred embodiment, the packed column of the present application may be used as a separation column in a gas chromatograph or a gas chromatograph-mass spectrometer, wherein after the separation column is connected in the gas chromatograph or gas chromatograph-mass spectrometer system, a separation experiment of saturated hydrocarbons and unsaturated hydrocarbons in a hydrocarbon-based material is started under a low temperature of the column box; after all of the saturated hydrocarbon component in the hydrocarbon-based material flow out of the separation column, the temperature of the column box is raised and the flow direction of the carrier gas in the separation column is reversed at the same time, and after the temperature is raised to a relatively high temperature, unsaturated hydrocarbon component enriched in the separation column flows out and are fed to an instrument detector for detection, so that the contents of saturated and unsaturated hydrocarbons in the hydrocarbon-based material and detailed hydrocarbon composition information can be conveniently and quickly obtained depending on the type of detector equipped in the instrument.

In a fourth aspect, the present application provides a system suitable for use in hydrocarbon component separation and analysis of a hydrocarbon-based material comprising saturated and unsaturated hydrocarbons, comprising a packed column according to the present application.

In some preferred embodiments, the system may comprise a packed column of the present application and a gas chromatograph, wherein the packed column may be connected between a sample inlet and a detector inlet of the gas chromatograph.

To further analyze the components and their contents in the hydrocarbon component of the hydrocarbon-based material, in other preferred embodiments, the system may further comprise a gas chromatograph-mass spectrometer, which may be of a type commonly used in the art.

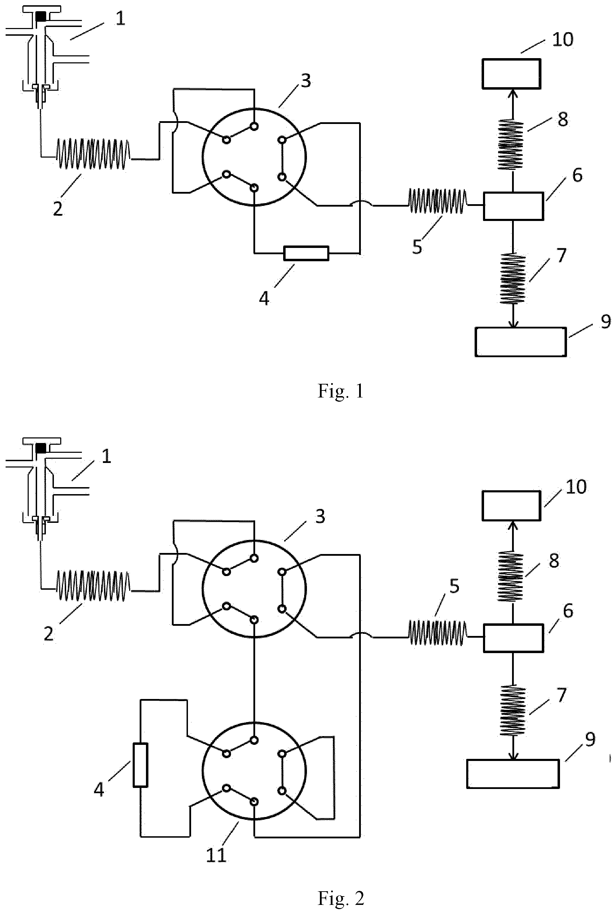

In some further preferred embodiments, as shown in Fig. 1, the gas chromatograph-mass spectrometer may comprise a sample inlet 1 and an analysis module, and the two ends of a packed column 4 may be respectively communicated with the sample inlet 1 and an inlet of the analysis module. The analysis module may comprise a mass spectrometry unit 10 and a gas chromatography unit, the gas chromatography unit may, for example, comprise a flame ionization detector 9, the inlets of the mass spectrometry unit 10 and the gas chromatography unit may be in communication with the inlet of the analysis module via a splitter 6, respectively. In this embodiment, after the packed column is connected in the gas chromatograph or gas chromatograph-mass spectrometer system, a separation experiment of saturated hydrocarbons and unsaturated hydrocarbons in a hydrocarbon-based material is started under a low temperature of the column box; after all of the saturated hydrocarbon component in the hydrocarbon-based material flows out of the separation column, the temperature of the column box is raised and the flow direction of the carrier gas in the separation column is reversed at the same time, and after the temperature is raised to a relatively high temperature, unsaturated hydrocarbon component enriched in the separation column flows out and is fed to the detector of the instrument for detection, so that the contents of saturated and unsaturated hydrocarbons in the hydrocarbon-based material and detailed hydrocarbon composition information can be conveniently and quickly obtained depending on the type of detector equipped in the instrument.

In a further preferred embodiment, to help the maintainance of a vacuum state in the mass spectrometer and ensure a stable coupling of the packed column and the gas chromatograph-mass spectrometer, as shown in Fig. 1, a first capillary column 2 may be connected between the sample inlet 1 and the packed column 4, and a second capillary column 5 may be connected between the packed column 4 and the inlet of the analysis module, wherein the quartz capillary column 2 preferably has an inner diameter of about 0.1-0.6 mm and a length of about 0.1-60 m, more preferably an inner diameter of about 0.2-0.5 mm and a length of about 2-50 m; further more preferably an inner diameter of about 0.2-0.4 mm and a length of about 5-30 m. The quartz capillary column 5 preferably has an inner diameter of about 0.1-0.6 mm and a length of about 0.2-30 m, and more preferably an inner diameter of about 0.2-0.4 mm and a length of about 0.5-10 m.

In a further preferred embodiment, to facilitate the control of the purging with the carrier gas and the flow direction of the effluent within the packed column, the system may further comprise a multi-port valve through which the packed column may be switchably coupled to the gas chromatograph-mass spectrometer.

In a further preferred embodiment, as shown in Fig. 1, the multi-port valve may have a plurality of working ports, and have a first working position and a second working position; in the first working position, a first end (also referred to as "inlet") of the packed column 4 may be in communication with the sample inlet 1 through at least one working port, and a second end (also referred to as "outlet") of the packed column 4 is in communication with the inlet of the analysis module through at least one working port, such that at least a portion of the hydrocarbon fuel flows in the packed column 4 from the first end to the second end (i.e., from left to right in Fig. 1); in the second working position, the second end of the packed column may be in communication with the sample inlet through at least one working port, and the first end of the packed column is in communication with the inlet of the analysis module through at least one working port, such that at least a portion of the hydrocarbon fuel flows in the packed column from the second end to the first end. In this embodiment, the multi-port valve can be first set to the first working position under a lower temperature, and the filler in the packed column can adsorb and enrich unsaturated hydrocarbons, so that a first effluent comprising saturated hydrocarbons flows out from the second end of the packed column and enters the analysis module for further analysis; the multi-port valve can then be set to the second working position under an elevated temperature, and the filler in the packed column releases the adsorbed unsaturated hydrocarbons, so that a second effluent comprising unsaturated hydrocarbons flows out from the first end of the packed column and enters the analysis module for further analysis.

In the present application, the multi-port valve may be a hand-operated rotary valve and/or a pneumatic rotary valve well known to those skilled in the art that have a plurality of working ports for connecting and controlling multiple pipelines, for example, a six-way valve. The number of the multi-port valves can be one or more. The manner in which the multi-port valve is switched between the first working position and the second working position may be well known to those skilled in the art, for example by turning a handle and/or by pneumatic actuation.

In a particularly preferred embodiment, as shown in Fig. 1, the system may comprise a gas chromatograph-mass spectrometer; a packed column 4 according to the present application; a six-way valve 3 and quartz capillary column tubes 2 and 5; the gas chromatograph-mass spectrometer may further comprise a sample inlet 1, a splitter 6, quartz capillary columns 7 and 8, a mass spectrometer unit 10 and a gas chromatograph unit having a flame ionization detector 9. The six-way valve 3 and the dividing/undividing sample inlet 1 can be connected through the quartz capillary column 2, the splitter 6 and the six-way valve 3 can be connected through the quartz capillary column 5, so that the mass spectrometer can be kept in a vacuum state, a stable coupling of the packed column and the gas chromatograph-mass spectrometer can be ensured, the splitter 6 is connected with the mass spectrometry unit 10 through a quartz capillary column 8, and the splitter 6 is connected with the flame ionization detector 9 through a quartz capillary column 7.

In still further preferred embodiments, as shown in Fig. 2, the system may further comprise a multi-port valve set having a first multi-port valve 3 and a second multi-port valve 11, the first multi-port valve 3 and the second multi-port valve 11 may be communicated with each other in a switchable manner, and the packed column 4 may be connected into the gas chromatograph-mass spectrometer through the second multi-port valve 11; the multi-port valve set has a switch-in working position for connecting the packed column into the system and a switch-out working position for removing the packed column out of the system; in the switch-in working position, the sample inlet 1, the first multi-port valve 3, the packed column 4 connected through the second multi-port valve 11 and the inlet of the analysis module are communicated sequentially, so that at least a portion of the hydrocarbon fuel flows through the sample inlet, the first multi-port valve, the packed column and the analysis module sequentially; and in the switch-out working position, the packed column is not connected into the system, and the sample inlet, the first multi-port valve, the second multi-port valve and the inlet of the analysis module are communicated sequentially, so that at least a portion of the hydrocarbon fuel flows through the sample inlet, the first multi-port valve, the second multi-port valve and the analysis module sequentially. In this embodiment, the packed column is connected into the system during enrichment and separation process, when saturated hydrocarbon component and unsaturated hydrocarbon component respectively flow out of the packed column, the packed column can be switched out of the system by switching the multi-port valve set, and at that moment, fluid in the system does not flow through the packed column, so that the service life of the packed column can be remarkably prolonged.

The packed column and system of the present application may be used for separating and analyzing hydrocarbon components in hydrocarbon-based materials, especially hydrocarbon fuels, which may be at least one of gasoline, aviation fuel and diesel oil, preferably selected from the group consisting of aviation fuel, diesel oil or a combination thereof. The unsaturated hydrocarbon may be selected from the group consisting of aromatic hydrocarbons, olefins, or combinations thereof, preferably aromatic hydrocarbons.

In some preferred embodiments, as shown in Fig. 1, the separation and analysis of hydrocarbon component in hydrocarbon fuels using the system of the present application may include the following: after being vaporized and entering the device through the dividing/undividing sample inlet 1, the hydrocarbon fuel to be analyzed firstly flows through the packed column 4, the unsaturated hydrocarbons in the hydrocarbon fuel is selectively enriched by the packed column at a low temperature, while the saturated hydrocarbon component can smoothly pass through the packed column, the saturated hydrocarbon component is divided by the splitter 6 and respectively passed to the mass spectrometry unit 10 and the flame ionization detector 9, the mass spectrometry unit 10 carries out detailed hydrocarbon composition analysis on the incoming saturated hydrocarbon component, the saturated hydrocarbon component is classified into paraffin, monocyclic naphthene, dicyclic naphthene and the like according to characteristic ion peaks, and the saturated hydrocarbon component passed to the flame ionization detector 9 is used for calculating the content by percentage of the saturated hydrocarbon in the hydrocarbon fuel; after the saturated hydrocarbon component completely passes through the packed column and enters the analysis module, the six-way valve 3 is rotated to change the flow direction of carrier gas in the device, and the temperature of the packed column is raised at the same time to release enriched unsaturated hydrocarbon, so that a complete separation of the unsaturated hydrocarbon and the saturated hydrocarbon in the hydrocarbon fuel can be realized, the unsaturated hydrocarbon component respectively enters the mass spectrometry unit 10 and the flame ionization detector 9 for detection, the unsaturated hydrocarbons can be classified in the mass spectrometry unit 10 into olefin, alkylbenzene, naphthalene and the like according to the detected characteristic ion peaks, and the unsaturated hydrocarbon data detected by the flame ionization detector 9 is used for calculating the content by percentage of the unsaturated hydrocarbon in the hydrocarbon fuel.

In other preferred embodiments, as shown in Fig. 2, the system may comprise a double six-way valve configuration (i.e., a multi-port valve set of two six-way valves), so that the service life of the packed column 4 can be significantly prolonged.

In a fifth aspect, the present application provides a method for performing hydrocarbon component separation and analysis on a hydrocarbon-based material comprising saturated hydrocarbon and unsaturated hydrocarbon, comprising the steps of:

S1) feeding the hydrocarbon-based material into a packed column from a first end thereof by purging with a carrier gas under a first separation condition for enrichment and separation, to obtain a first effluent comprising saturated hydrocarbon from a second end of the packed column; and

S2) purging the packed column from the second end thereof with the carrier gas under a second separation condition, to obtain a second effluent comprising unsaturated hydrocarbon from the first end of the packed column;

wherein the packed column is a packed column according to the present application and the temperature of the first separation condition is lower than the temperature of the second separation condition.

In the method according to the present application, the packed column has a relatively larger enrichment capacity and good releasability for unsaturated hydrocarbons, and can reversibly enrich and release unsaturated hydrocarbons in hydrocarbon-based materials, so that an online separation of unsaturated hydrocarbons from saturated hydrocarbons in hydrocarbon-based materials, especially hydrocarbon fuels, and an accurate determination of detailed hydrocarbon composition thereof can be realized, olefins and naphthenes and the like can be respectively determined qualitatively and quantitatively, the whole process can be performed online, the repeatability and the accuracy of the detailed hydrocarbon composition analysis of hydrocarbon-based materials can be significantly improved, and the time for the detailed hydrocarbon composition analysis of hydrocarbon-based materials can be shortened. This method can be used for quick and accurate determination of detailed hydrocarbon composition of hydrocarbon-based materials, especially hydrocarbon fuels such as aviation fuel and diesel oil, including biomass-derived aviation fuel and biomass-derived diesel oil.

In the method according to the present application, the hydrocarbon-based material may comprise saturated hydrocarbon and unsaturated hydrocarbon, the enrichment material in the packed column can enrich unsaturated hydrocarbon component, the material may adsorb unsaturated hydrocarbon component under the first separation condition, so that saturated hydrocarbon component passes through the packed column, and after all of the saturated hydrocarbon component flows out, the unsaturated hydrocarbon component may be desorbed from the enrichment material under the second separation condition and flows out of the packed column under the action of the carrier gas, so that a separation of the saturated hydrocarbon component from the unsaturated hydrocarbon component can be realized. After all of the saturated hydrocarbon component flows out, to increase the outflow rate of the unsaturated hydrocarbon component, a back flushing with the carrier gas may be performed along a direction opposite to flushing direction under the first separation condition.

In a preferred embodiment, the hydrocarbon-based material is a hydrocarbon fuel, preferably selected from the group consisting of aviation fuel, diesel fuel or a combination thereof; the unsaturated hydrocarbon component is selected from the group consisting of aromatic hydrocarbons, olefins, or combinations thereof, preferably aromatic hydrocarbons.

In the method according to the present application, the first separation condition may vary over a wide range, for example the first separation condition may include: a temperature of about 80-180 °C, and a flow rate of the carrier gas of about 1-60 mL/min; to further improve the separation efficiency, it is preferred that the first separation is performed at a temperature of about 115-170 °C and a flow rate of the carrier gas of about 2-45 mL/min; the conditions for the second separation may also vary over a wide range, for example the second separation condition may include: a temperature of about 190-400 °C, and a flow rate of the carrier gas of about 1-60 mL/min; further, in order to facilitate the release of the unsaturated hydrocarbon component from the enrichment material and increase the outflow rate, the second separation is preferably performed at a temperature of about 200-350 °C and a carrier gas flow rate of about 2-45 mL/min. Further preferably, the temperature of the second separation condition may differ from the temperature of the first separation condition by about 20-200 °C, preferably about 30-100 °C.

In a preferred embodiment of the method according to the present application, the first separation condition may be first maintained to perform step S1; when all of the saturated hydrocarbon component flows out under the first separation condition, the temperature may be raised to the second separation condition at a rate that may be, for example, about 10-250 °C/min, preferably about 30-150 °C/min. Under the first separation condition, to ensure that all of the saturated hydrocarbon component flows out, the first separation condition may be maintained for a time period of, for example, about 1-50 minutes, preferably about 2-30 minutes. Further, after the temperature is raised to the second separation condition, it may be maintained for, for example, about 0.5-10 minutes, preferably about 1-8 minutes, to perform step S2, so as to ensure that all of the unsaturated hydrocarbons flow out.

In the method according to the present application, the carrier gas may be any gas that does not react with the components of the hydrocarbon-based material under the first separation condition and the second separation condition, such as an inert gas, preferably selected from the group consisting of helium, nitrogen, hydrogen, or a combination thereof.

In the method according to the present application, to ensure the efficiency and accuracy of the analysis, the sample amount of the hydrocarbon-based material is preferably about 0.01-2 μL, and more preferably about 0.02-1 μL.

To further analyze the composition of the first and second effluents, in some preferred embodiments, the method may further comprise: S3) after the step S1, passing the first effluent to a gas chromatograph and a mass spectrometer to analyze the kind and content of the saturated hydrocarbon component in the first effluent; and/or S4) after the step S2, passing the second effluent to a gas chromatograph and a mass spectrometer to perform separation and analysis on unsaturated hydrocarbon component in the second effluent.

In a further preferred embodiment, to ensure the analytical accuracy of the gas chromatograph and mass spectrometer, the mass ratio (divide ratio) between the portion of the first effluent entering the gas chromatograph and the portion of the first effluent entering the mass spectrometer may be about (1-40) : 1, preferably about (5-30) : 1; the mass ratio between the portion of the second effluent entering the gas chromatograph to the portion of the second effluent entering the mass spectrometer may be about (1-40) : 1, preferably about (5-30) : 1.

In the method according to the present application, the operating conditions of the gas chromatography may vary within a wide range, and preferably, the operating conditions of the gas chromatography may include: a temperature of the flame ionization detector of about 200-350 °C, preferably about 230-300 °C; operating conditions of the mass spectrometry may include: a temperature of the mass spectrometer transmission line of about 180-400 °C, preferably about 200-300 °C.

In some preferred embodiments of the method according to the present application, the packed column can be connected between a sample inlet and an analysis module of a gas chromatograph-mass spectrometer to conveniently and quickly perform the method according to the present application using existing gas chromatograph-mass spectrometer; in this embodiment, the hydrocarbon-based material may sequentially pass through the sample inlet, and the packed column for separating out saturated hydrocarbon component and unsaturated hydrocarbon component, which are then fed to the analysis module, respectively, to further analyze the detailed composition thereof.

Further, to facilitate the control of the operation, in some preferred embodiments, the method according to the present application is carried out using a device as shown in Fig. 1, wherein the packed column is connected into the gas chromatograph-mass spectrometer via a multi-port valve, which can be configured to have a first working position and a second working position; and in the first working position, the first end of the packed column can be communicated with the sample inlet through at least one working port of the multi-port valve, and the second end of the packed column can be communicated with the inlet of the analysis module through at least one working port, so that at least a portion of the hydrocarbon fuel flows in the packed column from the first end to the second end; in the second working position, the second end of the packed column can be communicated with the sample inlet via at least one working port, and the first end of the packed column may be communicated with the inlet of the analysis module via at least one working port, such that at least a portion of the hydrocarbon fuel flows in the packed column from the second end to the first end. In this embodiment, the multi-port valve may be set in the first working position under the first separation condition, allowing the first effluent to flow out from the second end of the packed column and enter the analysis module for further analysis; then, the multi-port valve may be set in the second working position under the second separation condition, allowing the second effluent to flow out from the first end of the packed column and enter the analysis module for further analysis.

In other preferred embodiments, the method according to the present application is carried out using a device as shown in Fig. 2, wherein a multi-port valve set having a first multi-port valve and a second multi-port valve is used to connect the packed column into a gas chromatograph-mass spectrometer, the multi-port valve set is configured to have a switch-in working position for connecting the packed column into the system and a switch-out working position for removing the packed column out from the system; in the switch-in working position, as shown in Fig. 2, the sample inlet, the first multi-port valve, the packed column connected via the second multi-port valve, and the inlet of the analysis module may be communicated sequentially, so that at least a portion of the hydrocarbon fuel sequentially flows through the sample inlet, the first multi-port valve, the packed column, and the analysis module sequetially; and in the switch-out working position, the packed column is not connected into the system, and the sample inlet, the first multi-port valve, the second multi-port valve and the inlet of the analysis module may be communicated sequentially, so that at least a portion of the hydrocarbon fuel flows through the sample inlet, the first multi-port valve, the second multi-port valve and the analysis module sequentially. In this embodiment, the multi-port valve can be set in the switch-in working position under the first separation condition and the second separation condition, where the packed column is connected into the system, and the sample to be measured flows into the packed column through the multi-port valve; after the first effluent and the second effluent have entered the analysis module for separation and analysis, the multi-port valve can be set in the switch-out working position, where the packed column is not connected into the system, and the fluid coming from the sample inlet or the working port of the first multi-port valve only flows through the second multi-port valve while does not flow through the packed column.

In some preferred embodiments, as shown in Fig. 1, the method according to the present application may be as follows: the hydrocarbon fuel to be analyzed is fed directly without pre-separation, and is vaporized through a dividing/undividing sample inlet 1, then flows through a packed column 4, the unsaturated hydrocarbon in the hydrocarbon fuel is selectively enriched by the packed column at low temperature, while the saturated hydrocarbon component smoothly passes through, the saturated hydrocarbon component is divided by a splitter 6 and then enters a mass spectrometry unit 10 and a flame ionization detector 9 respectively, the mass spectrometry unit 10 performs detailed hydrocarbon composition analysis on the incoming saturated hydrocarbon component, and classifies the saturated hydrocarbon component into alkane, monocyclic naphthene, bicyclic naphthene, tricyclic naphthene and the like, and the saturated hydrocarbon component entering the flame ionization detector 9 is used for calculating the percentage content of saturated hydrocarbon in the hydrocarbon fuel; after all of the saturated hydrocarbon component passes through the packed column and enters the analysis module, the six-way valve 3 rotates to change the flow direction of carrier gas in the device, and the temperature of the packed column is raised at the same time to release enriched unsaturated hydrocarbon, so that a complete online separation of the unsaturated hydrocarbon and the saturated hydrocarbon in the hydrocarbon fuel can be realized, the unsaturated hydrocarbon enters the mass spectrometry unit 10 and the flame ionization detector 9, respectively, for detection, the mass spectrometry unit 10 performs detailed hydrocarbon composition analysis on the incoming unsaturated hydrocarbon component and classifies the unsaturated hydrocarbon component into olefin, alkylbenzene, indane or tetralin, indene, naphthalene, acenaphthene, acenaphthylene, tricyclic aromatic hydrocarbon and the like, and the unsaturated hydrocarbon component entering the flame ionization detector 9 is used for calculating the percentage content of the unsaturated hydrocarbon in the hydrocarbon fuel.

In some preferred embodiments, as shown in Fig. 1, a six-way valve 3 is used to achieve the back flushing of the gas circuit in the method; in other preferred embodiments, as shown in Fig. 2, two six-way valves are adopted, wherein the six-way valve 3 is used for realizing the back flushing of the gas circuit in the method, and the six-way valve 11 is used for realizing the connection or disconnection of the packed column 4 in the gas circuit, so that the service life of the packed column is further prolonged.

In some preferred embodiments, the present application provides the following technical solutions:

A1. A reversible enrichment material for aromatic hydrocarbon component in hydrocarbon fuels, characterized in that the reversible enrichment material comprises:

an inorganic carrier; and
an active metal salt, a first metal salt promoter and a second metal salt promoter supported on the inorganic carrier;
wherein the active metal salt is selected from the group consisting of a soluble silver salt, a soluble copper salt, or a combination thereof, the first metal salt promoter is one or more selected from the group consisting of soluble salts of Group IA, Group IIA and Group IIIA metals, and the second metal salt promoter is one or more selected from the group consisting of soluble salts of transition metals other than Group IB metals;
the inorganic carrier is obtained by subjecting an inorganic starting material to a calcination treatment, an acid-washing and/or alkali-washing treatment and a drying treatment sequentially.

A2. The reversible enrichment material of Item A1, wherein the conditions of the calcination treatment include: a temperature of about 500-950 °C and a period of about 4-16 hours.

A3. The reversible enrichment material of Item A1, wherein the conditions of the acid-washing treatment include: a temperature of about 100-200 °C, a period of about 10-40 hours, and a mass ratio of the starting material to be treated on a dry basis to the acid solution of about 1 : (1-20), wherein the acid solution used for the acid-washing treatment is one or more selected from the group consisting of nitric acid, hydrochloric acid and sulfuric acid, and the concentration of the acid solution used for the acid-washing treatment is about 1-90% by mass;
the conditions of the alkali-washing treatment include: a temperature of about 100-200 °C, a period of about 10-40 hours, and a mass ratio of the starting material to be treated on a dry basis to the alkali solution of about 1 : (1-20), wherein the alkali solution used for the alkali-washing treatment is one or more selected from sodium hydroxide solution, potassium hydroxide solution and ammonia water, and the concentration of the alkali solution used for the alkali-washing treatment is about 1-90% by mass.

A4. The reversible enrichment material of Item A1, wherein the conditions of the drying treatment include: a temperature of about 100-200 °C and a period of about 3-6 hours.

A5. The reversible enrichment material of Item A1, wherein the inorganic carrier has a specific surface area of about 1-600 $m^2$/g, a pore size in a range of about 1-1000 nm, and a particle size in a range of about 80-800 $\mu$m.

A6. The reversible enrichment material of Item A1 or A5, wherein the inorganic carrier is one or more selected from the group consisting of diatomite carrier, alumina carrier, titania carrier, zirconia carrier, mesoporous molecular sieve carrier, amorphous silica-alumina carrier, silica gel carrier, and controlled porous glass carrier.

A7. The reversible enrichment material of Item A1, wherein, in the reversible enrichment material, the active metal salt is present in an amount of about 0.1-80% by mass, preferably about 0.5-50% by mass; the first metal salt promoter is present in an amount of about 0.1-80% by mass, preferably about 0.2-40% by mass; and the second metal salt promoter is present in an amount of about 0.5-80% by mass, preferably about 0.5-30% by mass.

A8. The reversible enrichment material of Item A1, wherein the ratio of the mass content of the first metal salt promoter to the mass content of the second metal salt promoter, calculated as metal oxide, is about 1 : (0.05-0.15) or about 1 : (0.5-50).

A9. The reversible enrichment material of Item A1, wherein the soluble silver salt is silver nitrate and the soluble copper salt is selected from the group consisting of copper nitrate, copper sulfate or a combination thereof.

A10. The reversible enrichment material of Item A1, wherein, in the first metal salt promoter, the Group IA metal is one or more selected from the group consisting of lithium, sodium and potassium, the Group IIA metal is one or more selected from the group consisting of beryllium, magnesium, calcium and barium, and the Group IIIA metal is selected from the group consisting of aluminum, gallium or a combination thereof; in the second metal salt promoter, the transition metal is one or more selected from the group consisting of zinc, cadmium, vanadium, chromium, molybdenum, tungsten, manganese, iron, cobalt, nickel, ruthenium, platinum, rhodium and palladium.

A11. The reversible enrichment material of Item A1, wherein the second metal salt promoter comprises two soluble transition metal salts selected from soluble salts of Group VIII transition metals and soluble salts of Group IIB transition metals; the ratio of the mass content of the first soluble transition metal salt to the mass content of the second soluble transition metal salt, calculated as metal oxide, is about 1 : (0.05-0.15) or about 1 : (0.5-40).

A12. A method for preparing a reversible enrichment material according to any one of Items A1 to A11, characterized in that the method comprises: loading the active metal salt, the first metal salt promoter and the second metal salt promoter onto the inorganic carrier, and then carrying out a drying treatment.

B1. A packed column for reversible enrichment of unsaturated hydrocarbon component in hydrocarbon fuels, characterized in that the packed column comprises a column tube and an enrichment filler filled in the column tube;

the enrichment filler comprises an inorganic carrier, and an active metal salt, a first metal salt promoter and a second metal salt promoter supported on the inorganic carrier;
wherein the active metal salt is selected from the group consisting of soluble silver salts, soluble copper salts, or a combination thereof, the first metal salt promoter is one or more selected from the group consisting of soluble salts of Group IA, Group IIA and Group IIIA metals, and the second metal salt promoter is one or more selected from the group consisting of soluble salts of transition metals other than Group IB metals;
the inorganic carrier is obtained by subjecting an inorganic starting material to a calcination treatment, an acid-washing and/or alkali-washing treatment and a drying treatment sequentially.

B2. The packed column of Item B1, wherein the inorganic carrier is one or more selected from the group consisting of diatomite carrier, alumina carrier, titania carrier, zirconia carrier, mesoporous molecular sieve carrier, amorphous silica-alumina carrier, silica gel carrier, and controlled porous glass carrier.

B3. The packed column of Item B2, wherein the inner surface of the column tube has been subjected to a passivation treatment comprising alkali treatment and acid treatment; and the column tube is a stainless steel column tube, a glass column tube or a quartz column tube.

B4. The packed column of Item B2 or B3, wherein the passivation treatment comprises: soaking the column tube in an alkali solution having a contration of about 5-20% by mass at 20-100 °C for 30-120 minutes, washing with water to neutrality, soaking in an acid solution have a concentration of 5-20% by mass at 30-120 °C for 30-100 minutes, washing with water to neutrality, and drying.

B5. The packed column of Item B1, wherein the column tube is U-shaped or spiral-shaped.

B6. The packed column of Item B1, wherein the column tube has an inner diameter of about 0.1-12 mm and a length of about 20-5000 mm.

B7. The packed column of Item B6, wherein the column tube has an inner diameter of about 1-6 mm and a length of about 50-1000 mm.

B8. The packed column of Item B1, wherein the inorganic carrier has a specific surface area of about 1-600 $m^2/g$, a pore size in a range of about 1-1000 nm, and a particle size in a range of about 80-800 $\mu$m.

B9. The packed column of Item B1, wherein, in the enrichment filler, the active metal salt is present in an amount of about 0.1-80% by mass, preferably about 0.5-50% by mass; the first metal salt promoter is present in an amount of about 0.1-80% by mass, preferably about 0.2-40% by mass; and the second metal salt promoter is present in an amount of about 0.5-80% by mass, preferably about 0.5-30% by mass.

B10. the packed column of Item B1, wherein the ratio of the mass contents of the first metal salt promoter to the mass content of the second metal salt promoter, calculated as metal oxide, is about 1 : (0.05-0.15) or about 1 : (0.5-50).

B11. The packed column of Item B1, wherein the soluble silver salt is silver nitrate and the soluble copper salt is selected from the group consisting of copper nitrate, copper sulfate, or a combination thereof;

in the first metal salt promoter, the Group IA metal is one or more selected from the group consisting of lithium, sodium and potassium, the Group IIA metal is one or more selected from the group consisting of beryllium, magnesium, calcium and barium, and the Group IIIA metal is selected from the group consisting of aluminum, gallium or a combination thereof; in the second metal salt promoter, the transition metal is one or more selected from the group consisting of zinc, cadmium, vanadium, chromium, molybdenum, tungsten, manganese, iron, cobalt, nickel, ruthenium, platinum, rhodium and palladium;

the second metal salt promoter is two selected from the group consisting of soluble salts of transition metals other than Group IB metals, preferably soluble salts of Group VIII and Group IIB transition metals.

B12. The packed column of Item B10, wherein the second metal salt promoter comprises a first one of the second metal salt promoter selected from soluble salts of Group VIII transition metals and a second one of the second metal salt promoter selected from soluble salts of Group IIB and/or Group VIB transition metals; the ratio of the mass content of the first soluble transition metal salt to the mass content of the second soluble transition metal salt, calculated as metal oxide, is about 1 : (0.05-0.15) or about 1 (0.5-40).

B13. The packed column of Item B1, wherein the conditions of the calcination treatment include: a temperature of about 500-950 °C and a period of about 4-16 hours;

the conditions of the acid-washing treatment include: a temperature of about 100-200 °C, a period of about 10-40 hours, and a mass ratio of the starting material to be treated on a dry basis to the acid solution of about 1 : (1-20), wherein the acid solution used for the acid-washing treatment is one or more selected from the group consisting of nitric acid, hydrochloric acid and sulfuric acid, and the concentration of the acid solution used for the acid-washing treatment is about 1-90% by mass;

the conditions of the alkali-washing treatment include: a temperature of about 100-200 °C, a period of about 10-40 hours, and a mass ratio of the starting material to be treated on a dry basis to the alkali solution of about 1 : (1-20), wherein the alkali solution used for the alkali-washing treatment is one or more selected from the group consisting of sodium hydroxide solution, potassium hydroxide solution and ammonia water, and the concentration of the alkali solution used for the alkali-washing treatment is about 1-90% by mass;

the conditions of the drying treatment include: a temperature of about 100-200 °C and a period of about 3-6 hours.

B14. The packed column of Item B1, wherein the enrichment filler is prepared by a method comprising: loading the active metal salt, the first metal salt promoter and the second metal salt promoter onto the inorganic carrier, and then carrying out a drying treatment.

B15. The packed column of Item B1, wherein the hydrocarbon fuel is selected from the group consisting of aviation fuel, diesel fuel, or a combination thereof; the unsaturated hydrocarbon is selected from aromatic hydrocarbon, olefin, or a combination thereof.

B16. A system for separating and analyzing hydrocarbon components of hydrocarbon fuels, characterized in that the system comprises a packed column according to any one of Items B1 to B 15.

B17. The system of Item B16, wherein the system further comprises a gas chromatograph-mass spectrometer; the gas chromatograph-mass spectrometer comprises a sample inlet and an analysis module, wherein two ends of the packed column are respectively communicated with the sample inlet and the inlet of the analysis module;

the analysis module comprises a mass spectrometry unit and a gas chromatography unit, and inlets of the mass spectrometry unit and the gas chromatography unit are respectively communicated with the inlet of the analysis module through a splitter.

B18. The system of Item B17, wherein a first capillary column is connected between the sample inlet and the packed column, and a second capillary column is connected between the packed column and the inlet of the analysis module.

B19. The system of Item B17, wherein the system further comprises a multi-port valve through which the packed column is switchably connected into the gas chromatograph-mass spectrometer.

B20. The system of Item B17, wherein the multi-port valve has a plurality of working ports and has a first working position and a second working position;

in the first working position, the first end of the packed column is communicated with the sample inlet through at least one working port, and the second end of the packed column is communicated with the inlet of the analysis module through at least one working port;

in the second working position, the second end of the packed column is communicated with the sample inlet through at least one working port, and the first end of the packed column is communicated with the inlet of the analysis module through at least one working port.

B21. The system of Item B20, wherein the system further comprises a multi-port valve set comprising a first multi-port valve and a second multi-port valve, the first multi-port valve and the second multi-port valve are connected in a switchable manner, and the packed column is connected into the gas chromatograph-mass spectrometer through the second multi-port valve;

the multi-port valve set has a switch-in working position for connecting the packed column into the system and a switch-out working position for removing the packed column out from the system;

in the switch-in working position, the sample inlet, the first multi-port valve, the packed column connected via the second multi-port valve and the inlet of the analysis module are communicated sequentially;

in the switch-out working position, the packed column is not connected into the system, and the sample inlet, the first multi-port valve, the second multi-port valve and the inlet of the analysis module are communicated sequentially.

B22. The system of Item B20, wherein the multi-port valve is a six-way valve having 6 work ports.

C1. A method for separating and analyzing hydrocarbon components of hydrocarbon fuels, characteried in that the method comprises the steps of:

S1) feeding a hydrocarbon fuel into an enrichment column from a first end thereof under a first separation condition by purging with a carrier gas for enrichment and separation, to obtain a first effluent comprising saturated hydrocarbon from a second end of the enrichment column;

S2) purging the enrichment column from the second end with the carrier gas under a second separation condition, to obtain a second effluent comprising unsaturated hydrocarbon from the first end of the enrichment column;

wherein the temperature of the first separation condition is lower than the temperature of the second separation condition;

the enrichment column comprises a column tube and an enrichment material filled in the column tube; the enrichment material comprises an inorganic carrier, and an active metal salt, a first metal salt promoter and a second metal salt promoter supported on the inorganic carrier; wherein the active metal salt is selected from the group consisting of soluble silver salts, soluble copper salts, or a combination thereof, the first metal salt promoter is one or more selected from the group consisting of soluble salts of Group IA, Group IIA and Group IIIA metals, and the second metal salt promoter is one or more selected from the group consisting of soluble salts of transition metals other than Group IB metals; the inorganic carrier is obtained by subjecting an inorganic starting material to a calcination treatment, an acid-washing and/or alkali-washing treatment and a drying treatment sequentially.

C2. The method of Item C1, wherein the first separation condition include: a temperature of about 80-180 °C, and a flow rate of the carrier gas of about 1-60 mL/min; the second separation condition include: a temperature of about 190-400 °C, and a flow rate of the carrier gas of about 1-60 mL/min; a difference between the temperature of the second separation condition and the temperature of the first separation condition of about 20-200 °C.

C3. The method of Item C1, wherein the method further comprises: maintaining the first separation condition for about 1-50 minutes to perform step S1, and then raising the temperature to about 190-400 °C and maintaining the temperature for about 0.5-10 minutes to perform step S2, wherein the temperature raising rate is about 10-250 °C/min.

C4. The method of Item C1, wherein the carrier gas is at least one of helium, nitrogen, and hydrogen.

C5. The method of Item C1, wherein the hydrocarbon fuel is fed in an amount of about 0.01-2 $\mu$L.

C6. The method of Item C1, wherein the method further comprises feeding the first effluent to a gas chromatograph and a mass spectrometer to separate and analyze saturated hydrocarbon component therein; and/or, feeding the second effluent to a gas chromatograph and a mass spectrometer to separate and analyze unsaturated hydrocarbon component therein.

C7. The method of Item C1, in which the mass ratio of the first effluent fed into the gas chromatograph and that fed into the mass spectrometer is about (1-40) : 1; the mass ratio of the second effluent fed into the gas chromatograph and that fed into the mass spectrometer is about (1-40) : 1.

C8. The method of Item C1, wherein the operating conditions of the gas chromatograph include: a temperature of the flame ionization detector of about 200-350 °C; the operating conditions of the mass spectrometer include: a temperature of the mass spectrometer transmission line of about 180-400 °C.

C9. The method of Item C1, wherein the method comprises: connecting the enrichment column between a sample inlet and an analysis module of a gas chromatograph-mass spectrometer; passing the hydrocarbon fuel sequentially through the sample inlet, the reversible enrichment column, and the analysis module.

C10. The method of Item C1, wherein the method further comprises: connecting the enrichment column into a gas chromatograph-mass spectrometer via a multi-port valve configured to have a first working position and a second working position; and

in the first working position, the first end of the enrichment column is communicated with the sample inlet through at least one working port of the multi-port valve, and the second end of the enrichment column is communicated with an inlet of the analysis module through at least one working port, such that at least a portion of the hydrocarbon fuel flows in the enrichment column from the first end to the second end;

in a second working position, the second end of the packed column is communicated with the sample inlet through at least one working port, and an inlet of the enrichment column is communicated with an inlet of the analysis module through at least one working port, such that at least a portion of the hydrocarbon fuel flows in the enrichment column from the second end to the first end;

the method comprises: setting the multi-port valve in the first working position under the first separation condition; and setting the multi-port valve in the second working position under the second separation condition.

C11. The method of Item C1, wherein the method further comprises: connecting the enrichment column into a gas chromatograph-mass spectrometer through a multi-port valve set comprising a first multi-port valve and a second multi-port valve, wherein the multi-port valve set is configured to have a switch-in working position for connecting the enrichment column into the system and a switch-out working position for removing the enrichment column out from the system;

in the switch-in working position, the sample inlet, the first multi-port valve, the enrichment column connected via the second multi-port valve, and an inlet of the analysis module are communicated sequentially so that at least a portion of the hydrocarbon fuel flows through the sample inlet, the first multi-port valve, the enrichment column, and the analysis module sequentially;

in the switch-out working position, the packed column is not connected into the system, and the sample inlet, the first multi-port valve, the second multi-port valve and the inlet of the analysis module are communicated sequentially, so that at least a portion of the hydrocarbon fuel flows through the sample inlet, the first multi-port valve, the second multi-port valve and the analysis module sequentially;

the method comprises: setting the multi-port valve in the switch-in working position under the first and second separation conditions; and setting the multi-port valve in the switch-out working position after the first effluent and the second effluent enter the analysis module for separation and analysis.

C12. The method of Item C1, wherein the method comprises subjecting the column tube to a passivation treatment and then loading the enrichment material into the passivated column tube to obtain the enrichment column; the passivation treatment comprises: soaking the column tube in an alkali solution having a concentration of 5-20% by mass at 20-100 °C for 30-120 minutes, washing with water to neutrality, soaking in an acid solution having a concentration of 5-20% by mass at 30-120 °C for 30-100 minutes, washing with water to neutrality, and then drying; the column tube is U-shaped or spiral-shaped, the column tube has an inner diameter of about 0.1-12 mm, and a length of about 20-5000 mm; the column tube is at least one of stainless steel column tube, glass column tube and quartz column tube.

C13. The method of Item C1, wherein, in the reversible enrichment material, the active metal salt is present in an amount of about 0.1-80% by mass, preferably about 0.5-50% by mass; the first metal salt promoter is present in an amount of about 0.1-80% by mass, preferably about 0.2-40% by mass; and the second metal salt promoter is present in an amount of about 0.5-80% by mass, preferably about 0.5-30% by mass.

C14. The method of Item C1, wherein the ratio of the mass content of the first metal salt promoter to the mass content of the second metal salt promoter, calculated as metal oxide, is about 1 : (0.05-0.15) or 1 : (0.5-50).

C15. The method of Item C1, wherein the soluble silver salt is silver nitrate and the soluble copper salt is selected from the group consisting of copper nitrate, copper sulfate, or a combination thereof;

in the first metal salt promoter, the Group IA metal is one or more selected from the group consisting of lithium, sodium and potassium, the Group IIA metal is one or more selected from the group consisting of beryllium, magnesium, calcium and barium, and the Group IIIA metal is selected from the group consisting of aluminum, gallium or a combination thereof; in the second metal salt promoter, the transition metal is one or more selected from the group consisting of zinc, cadmium, vanadium, chromium, molybdenum, tungsten, manganese, iron, cobalt, nickel, ruthenium, platinum, rhodium and palladium;

the second metal salt promoter is two selected from the group consisting of soluble salts of transition metals other than Group IB metals, preferably soluble salts of Group VIII and Group IIB transition metals.

C16. The method of Item C1, wherein the second metal salt promoter comprises a first one of the second metal salt promoter selected from soluble salts of Group VIII transition metals and a second one of the second metal salt promoter selected from soluble salts of Group IIB and/or group VIB transition metals; the ratio of the mass content of the first soluble transition metal salt to the mass content of the second soluble transition metal salt, calculated as metal oxide, is about 1 : (0.05-0.15) or about 1 : (0.5-40).

C17. The method of Item C1, wherein the conditions of the calcination treatment include: a temperature of about 500-950 °C and a period of about 4-16 hours;

the conditions of the acid-washing treatment include: a temperature of about 100-200 °C, a period of about 10-40 hours, and a mass ratio of the starting material to be treated on a dry basis to the acid solution of about 1 : (1-20), wherein the acid solution used for the acid-washing treatment is one or more selected from the group consisting of nitric acid, hydrochloric acid and sulfuric acid, and the concentration of the acid solution used for the acid-washing treatment is about 1-90% by mass;

the conditions of the alkali-washing treatment include: a temperature of about 100-200 °C, a period of about 10-40 hours, and a mass ratio of the starting material to be treated on a dry basis to the alkali solution of about 1 : (1-20), wherein the alkali solution used for the alkali-washing treatment is one or more selected from the group consisting of sodium hydroxide solution, potassium hydroxide solution and ammonia water, and the concentration of the alkali solution used for the alkali-washing treatment is about 1-90% by mass;

the conditions of the drying treatment include: a temperature of about 100-200 °C and a period of about 3-6 hours.

C18. The method of Item C17, wherein the inorganic carrier is one or more selected from the group consisting of diatomite carrier, alumina carrier, titania carrier, zirconia carrier, mesoporous molecular sieve carrier, amorphous silica-alumina carrier, silica gel carrier, and controlled porous glass carrier; the inorganic carrier has a specific surface area of about 1-600 $m^2/g$, a pore size in a range of about 1-1000 nm, and a particle size in a range of about 80-800 $\mu m$.

C19. The method of Item C1, wherein the method further comprises: loading the active metal salt, the first metal salt promoter and the second metal salt promoter onto the inorganic carrier, and then drying to obtain an enrichment material.

C20. The method according to any of Items C1-C19, wherein the hydrocarbon fuel is selected from the group consisting of aviation fuel, diesel fuel, or a combination thereof; the unsaturated hydrocarbon component is selected from aromatic hydrocarbon, olefin, or a combination thereof.

Examples

The present application will be further described in detail with reference to examples, but the present application is not limited thereto.

In the following examples and comparative examples, the diatomite starting material was purchased from Suzhou Jiaye Biotechnology Limited, under the trade name Diatomite; other inorganic starting materials were purchased from Sinopharm Chemical Reagent Co., Ltd., unless otherwise stated.

Carrier Preparation Example I-1

Diatomite starting material and water were mixed at a mass ratio of 1 : 0.8, slurried and calcined at 850 °C for 8 hours, the calcined diatomite was subjected to an acid-washing treatment for 3 times, wherein the acid used was sulfuric acid, the mass concentration of the acid solution was 20%, the temperature of each acid-washing was 135 °C, the time of each acid-washing was 8 hours, and the mass ratio of the diatomite starting material on a dry basis to the acid solution was 1 : 5, the acid-washed diatomite was filtered, and dried at 150 °C for 3 hours. The contents of each component of the diatomite before and after acid-washing are shown in Table 1-1.

Table I-1 Change in the composition of the diatomite before and after acid-washing

|  | Before treatment | After treatment |
|---|---|---|
| $SiO_2$ | 73.1 | 81.4 |
| $Al_2O_3$ | 9.7 | 8.7 |
| $Fe_2O_3$ | 5.2 | 4.6 |
| CaO | 3.6 | 1.3 |
| MgO | 3.1 | 1.8 |
| $K_2O$ | 2.5 | 1.1 |
| MnO | 1.2 | 0.9 |
| $P_2O_5$ | 1.6 | 0.2 |

The diatomite carrier product obtained was ground and screened for later use, designated as diatomite carrier Z1, which has a pore diameter mainly distributed in a range of 50-1000 nm as measured by mercury intrusion method, and a specific surface area of 13 $m^2$/g as measured by nitrogen physical adsorption method.

Carrier Preparation Example 1-2

Controlled porous glass was calcined at 600 °C for 10 hours, the calcined controlled porous glass was subjected to acid-washing treatment for 1 time, wherein the acid used was nitric acid, the mass concentration of the acid solution was 30%, the temperature of the acid-washing was 150 °C, the time of the acid-washing was 10 hours, and the mass ratio of the controlled porous glass starting material on a dry basis to the acid solution was 1 : 10, the acid-washed controlled porous glass was filtered, and dried at 150 °C for 3 hours.
The controlled porous glass carrier product obtained was screened for later use, designated as controlled porous glass carrier Z2, which has a pore diameter mainly distributed in a range of 2-50 nm as measured by static nitrogen physical adsorption method, and a specific surface area of 367 $m^2$/g as measured by nitrogen physical adsorption method.

Carrier Preparation Example 1-3

Diatomite starting material and water were mixed at a mass ratio of 1 : 1, slurried and calcined at a high temperature of 950 °C for 15 hours, the calcined diatomite was subjected to alkali-washing treatment for 2 times, wherein the alkali used was sodium hydroxide, the mass concentration of the alkali solution was 15%, the temperature of alkali-washing was 105 °C, the time of each alkali-washing was 9 hours, and the mass ratio of the diatomite starting material on a dry basis to the alkali solution was 1 : 12, the alkali-washed diatomite was filtered, and dried at 120 °C for 3 hours.
The diatomite carrier product obtained was ground and screened for later use, designated as diatomite carrier Z3, which has a pore diameter mainly distributed in a range of 50-1000 nm, and a specific surface area of 9 $m^2$/g as measured by nitrogen physical adsorption method.

Carrier Preparation Example 1-4

Diatomite starting material and water were mixed at a mass ratio of 1 : 1.5, slurried and calcined at a high temperature of 700 °C for 6 hours, the calcined diatomite was subjected to an acid-washing treatment for 2 times, the acid used was hydrochloric acid, the mass concentration of the acid solution was 5%, the temperature of acid-washing was 110 °C, the time of each acid-washing was 3 hours, and the mass ratio of the diatomite starting material on a dry basis to the acid solution was 1 : 30, the acid-washed diatomite was filtered, and dried at 110 °C for 1.5 hours.
The diatomite carrier product obtained was ground and screened for later use, designated as diatomite carrier Z4, which has a pore diameter mainly distributed in a range of 50-1000 nm, and a specific surface area of 4 $m^2$/g as measured by nitrogen physical adsorption method.

Carrier Preparation Example 1-5

Diatomite starting material and water were mixed at a mass ratio of 1 : 0.8, slurried and calcined at a high temperature of 850 °C for 8 hours. The calcined diatomite was ground and screened for later use, designated as diatomite carrier D1, which has a pore diameter mainly distributed in a range of 50-1000 nm, and a specific surface area of 2 m$^2$/g as measured by nitrogen physical adsorption method.

Carrier Preparation Example 1-6

Diatomite starting material was subjected to acid-washing treatment for 3 times, wherein the acid used was sulfuric acid, the mass concentration of the acid solution was 20%, the temperature of each acid-washing was 135 °C, the time of each acid-washing was 8 hours, and the mass ratio of the diatomite starting material on a dry basis to the acid solution was 1 : 5, the acid-washed diatomite was filtered, and dried at 150 °C for 3 hours.

The acid-washed diatomite was screened for later use, designated as diatomite carrier D2, which has a pore diameter mainly distributed in a range of 50-1000 nm, and a specific surface area of 3 m$^2$/g as measured by nitrogen physical adsorption method.

The following examples show the effect of the reversible enrichment material of the present application when used in the separation of saturated hydrocarbon and unsaturated hydrocarbon components of hydrocarbon-based materials, in which the reversible enrichment material of the present application was filled in a chromatographic column of a gas chromatograph, and the appearance time of ethylbenzene peak and the time at which n-hexadecane completely flowed out (i.e. completely passed through the chromatographic column) at the temperature for measurement were taken as evaluation criteria, and where the appearance time of ethylbenzene peak was greater than the time at which n-hexadecane completely flowed out at the temperature for measurement, it indicated that the reversible enrichment material had good separation performance.

Example I-1

5.2125g of diatomite carrier Z1 with a particle size of 150-400 $\mu$m was taken, immersed in 10 mL of a solution containing 0.25% by mass of silver nitrate, 0.5% by mass of potassium nitrate and 3% by mass of cadmium nitrate, naturally evaporated to remove water, and dried in an oven at 150 °C for 1 h to obtain an enrichment material having a mass content of silver nitrate of 0.5%, a mass content of potassium nitrate of 1% and a mass content of cadmium nitrate of 6%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.618 minutes and the appearance time of ethylbenzene peak was 5.336 minutes; and when measured at a column box temperature of 170 °C, the time at which n-hexadecane completely flowed out was 2.537 minutes and the appearance time of ethylbenzene peak was 3.824 minutes.

Example 1-2

5.0525g of controlled porous glass carrier Z2 with a particle size of 150-400 $\mu$m was taken, immersed in 10 mL of a solution containing 0.5% by mass of silver nitrate, 0.5% by mass of gallium nitrate and 10% by mass of palladium nitrate, naturally evaporated to remove water, and then dried in an oven at 150 °C for 1 h to obtain an enrichment material having a mass content of silver nitrate of 1%, a mass content of gallium nitrate of 1% and a mass content of palladium nitrate of 20%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 4.215 minutes and the appearance time of ethylbenzene peak was 5.725 minutes; and when measured at a column box temperature of 170 °C, the time at which n-hexadecane completely flowed out was 3.284 minutes and the appearance time of ethylbenzene peak was 4.307 minutes.

Example 1-3

5.0210 g of diatomite carrier Z3 with a particle size of 150-400 $\mu$m was taken, immersed in 10mL solution containing 0.5% by mass of silver nitrate, 5% by mass of beryllium nitrate and 1% by mass of zinc nitrate, naturally evaporated to remove water, and dried in an oven at 150 °C for 1 h to obtain an enrichment material having a mass content of silver nitrate of 1%, a mass content of beryllium nitrate of 10% and a mass content of zinc nitrate of 2%, relative to the total amount of the carrier.

The enrichment material obtained was filled into a packed column with an inner diameter of 2 mm and a length of 200 mm, the chromatographic column was connected into a gas chromatograph, and when measured at a column box temperature of 150 °C, the time at which n-hexadecane completely flowed out was 3.618 minutes and the appearance time of ethylbenzene peak was 5.674 minutes.

Example 1-4

5.2100 g of diatomite carrier Z4 with a particle size of 150-400 μm was taken, immersed in 10 mL of a solution containing 0.5% by mass of copper nitrate, 3% by mass of sodium chloride and 1% by mass of nickel nitrate, naturally evaporated to remove water, and then dried in an oven at 100 °C for 1 h to obtain an enrichment material having a mass content of copper nitrate of 1%, a mass content of sodium chloride of 6% and a mass content of nickel nitrate of 2%, relative to the total amount of the carrier.

The enrichment material obtained was filled into a packed column with an inner diameter of 2 mm and a length of 200 mm, the chromatographic column was connected into a gas chromatograph, and when measured at a column box temperature of 150 °C, the time at which n-hexadecane completely flowed out was 3.602 minutes and the appearance time of ethylbenzene peak was 6.025 minutes.

Example 1-5

6.2564g of diatomite carrier Z1 with a particle size of 150-400 μm was taken, immersed in 20 mL of a solution containing 1.25% by mass of copper sulfate, 1% by mass of magnesium nitrate and 5% by mass of platinum nitrate, naturally evaporated to remove water, and then dried in an oven at 150 °C for 1 h to obtain an enrichment material having a mass content of copper sulfate of 4%, a mass content of magnesium nitrate of 3.2% and a mass content of platinum nitrate of 16%, relative to the total amount of the carrier.

The enrichment material obtained was filled into a packed column with an inner diameter of 2 mm and a length of 200 mm, the chromatographic column was connected into a gas chromatograph, and when measured at a column box temperature of 150 °C, the time at which n-hexadecane completely flowed out was 3.602 minutes and the appearance time of ethylbenzene peak was 6.323 minutes.

Example 1-6

5.5587g of diatomite carrier Z1 with a particle size of 150-400 μm was taken, immersed in 20mL of a solution containing 0.125% by mass of copper nitrate, 2.5% by mass of magnesium nitrate and 0.25% by mass of ferric nitrate, naturally evaporated to remove water, and then dried in an oven at 100 °C for 1 h to obtain an enrichment material having a mass content of copper nitrate of 0.5%, a mass content of magnesium nitrate of 10% and a mass content of ferric nitrate of 1%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.602 minutes and the appearance time of ethylbenzene peak was 6.284 minutes; and when measured at a column box temperature of 170 °C, the time at which n-hexadecane completely flowed out was 2.163 minutes and the appearance time of ethylbenzene peak was 4.821 minutes.

Example 1-7

4.9532g of diatomite carrier Z1 with a particle size of 150-400 μm was taken, immersed in 10 mL of a solution containing 5% by mass of copper nitrate, 10% by mass of potassium nitrate and 2.5% by mass of cobalt nitrate, naturally evaporated to remove water, and then dried in an oven at 150 °C for 1 h to obtain an enrichment material having a mass content of copper nitrate of 10%, a mass content of potassium nitrate of 20% and a mass content of cobalt nitrate of 5%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.602 minutes and the appearance time of ethylbenzene peak was 6.886 minutes; and when measured at a column box temperature of 170 °C, the time at which n-hexadecane completely flowed out was 2.205 minutes and the appearance time of ethylbenzene peak was 4.974 minutes.

Example 1-8

4.9865g of diatomite carrier Z1 with a particle size of 150-400 $\mu$m was taken, immersed in 15 mL of a solution containing 5% by mass of copper nitrate, 4% by mass of potassium nitrate, 1% by mass of ferric nitrate and 0.5% by mass of rhodium nitrate, naturally evaporated to remove water, and then dried in an oven at 150 °C for 1 h to obtain an enrichment material having a mass content of copper nitrate of 15%, a mass content of potassium nitrate of 12%, a mass content of ferric nitrate of 3% and a mass content of rhodium nitrate of 1.5%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.602 minutes and the appearance time of ethylbenzene peak was 8.897 minutes; and when measured at a column box temperature of 170 °C, the time at which n-hexadecane completely flowed out was 2.163 minutes and the appearance time of ethylbenzene peak was 5.462 minutes.

Example 1-9

4.8765g of diatomite carrier Z1 with a particle size of 150-400 $\mu$m was taken, immersed in 15mL of a solution containing 10% by mass of silver nitrate, 2% by mass of aluminum nitrate, 5% by mass of cadmium nitrate and 0.5% by mass of ferric nitrate, naturally evaporated to remove water, and then dried in an oven at 150 °C for 1 h to obtain an enrichment material having a mass content of silver nitrate of 30%, a mass content of aluminum nitrate of 6%, a mass content of cadmium nitrate of 15% and a mass content of ferric nitrate of 1.5%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.618 minutes and the appearance time of ethylbenzene peak was 9.215 minutes; and when measured at a column box temperature of 170 °C, the time at which n-hexadecane completely flowed out was 2.257 minutes and the appearance time of ethylbenzene peak was 5.847 minutes.

Example I-10

5.0615g of diatomite carrier Z1 with a particle size of 150-400 $\mu$m was taken, immersed in 20mL of a solution containing 2% by mass of silver nitrate, 1% by mass of lithium nitrate, 3% by mass of ammonium tungstate and 0.2% by mass of ruthenium nitrate, naturally evaporated to remove water, and then dried in an oven at 150 °C for 1 h to obtain an enrichment material having a mass content of silver nitrate of 8%, a mass content of lithium nitrate of 4%, a mass content of ammonium tungstate of 12% and a mass content of ruthenium nitrate of 0.8%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.618 minutes and the appearance time of ethylbenzene peak was 8.867 minutes; and when measured at a column box temperature of 170 °C, the time at which n-hexadecane completely flowed out was 2.313 minutes and the appearance time of ethylbenzene peak was 4.885 minutes.

Example I-11

10.2684g of diatomite carrier Z1 with a particle size of 150-400 $\mu$m was taken, immersed in 20mL of a solution containing 2% by mass of silver nitrate, 1% by mass of ferric nitrate, 0.5% by mass of ammonium tungstate and 1% by mass of potassium nitrate, naturally evaporated to remove water, and then dried in an oven at 150 °C for 1 h to obtain an enrichment material having a mass content of silver nitrate of 3.9%, a mass content of ferric nitrate of 1.9%, a mass content of ammonium tungstate of 1.9% and a mass content of potassium nitrate of 1.9%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.467 minutes and the appearance time of ethylbenzene peak was 5.844 minutes; and when measured at a column box temperature of 170 °C, the time at which n-hexadecane completely flowed out was 2.445 minutes and the appearance time of ethylbenzene peak was 4.157 minutes.

Example 1-12

4.8765g of diatomite carrier Z1 with a particle size of 150-400 $\mu$m was taken, immersed in 15mL of a solution containing 10% by mass of silver nitrate, 2% by mass of aluminum nitrate, 20% by mass of cadmium nitrate and 0.1% by mass of ferric nitrate, naturally evaporated to remove water, and then dried in an oven at 150 °C for 1 h to obtain an enrichment material having a mass content of silver nitrate of 30%, a mass content of aluminum nitrate of 6%, a mass content of cadmium nitrate of 60% and a mass content of ferric nitrate of 0.3%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.341 minutes and the appearance time of ethylbenzene peak was 6.535 minutes; and when measured at a column box temperature of 170 °C, the time at which n-hexadecane completely flowed out was 2.363 minutes and the appearance time of ethylbenzene peak was 4.031 minutes.

Example 1-13

An experiment was conducted substantially as described in Example 1-9, except that the diatomite carrier Z1 was replaced with the diatomite carrier Z4.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.704 minutes and the appearance time of ethylbenzene peak was 7.657 minutes; and when measured at a column box temperature of 170 °C, the time at which n-hexadecane completely flowed out was 2.481 minutes and the appearance time of ethylbenzene peak was 4.882 minutes.

Example 1-14

An experiment was conducted substantially as described in Example 1-9, except that the diatomite carrier Z1 was replaced with silica gel (produced by Qingdao Haiyang Chemical Co., Ltd.) having a particle size of 150-400 $\mu$m, a specific surface area of $418m^2/g$ and an average pore diameter of 20.8 nm.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 5.932 minutes and the appearance time of ethylbenzene peak was 6.948 minutes; when measured at a column box temperature of 170 °C, the time at which n-hexadecane completely flowed out was 4.128 minutes and the appearance time of ethylbenzene peak was 4.815 minutes.

Example 1-15

An experiment was conducted substantially as described in Example 1-9, except that the diatomite carrier Z1 was replaced with $\gamma$-$Al_2O_3$ having a particle size of 150-400 $\mu$m, a specific surface area of 270 $m^2/g$, and an average pore diameter of 12.1 nm.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 5.027 minutes and the appearance time of ethylbenzene peak was 6.316 minutes; and when measured at a column box temperature of 170 °C, the time at which n-hexadecane completely flowed out was 3.894 minutes and the appearance time of ethylbenzene peak was 4.128 minutes.

Example 1-16

An experiment was conducted substantially as described in Example 1-9, except that the diatomite support Z1 was replaced with SBA-15 mesoporous molecular sieve having a particle size of 150-400 $\mu$m, a specific surface area of 582 $m^2/g$, and an average pore diameter of 9.8 nm.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 5.276 minutes and the appearance time of ethylbenzene peak was 6.371 minutes; and when measured at a column box temperature of 170 °C, the time at which n-hexadecane completely flowed out was 4.628 minutes and the appearance time of ethylbenzene peak was 5.312 minutes.

Example 1-17

An experiment was conducted substantially as described in Example 1-9, except that the diatomite carrier Z1 was replaced with zirconia having a particle size of 150-400 $\mu$m, a specific surface area of 92 $m^2$/g and an average pore diameter of 7.8 nm.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 5.034 minutes and the appearance time of ethylbenzene peak was 6.211 minutes; and when measured at a column box temperature of 170 °C, the time at which n-hexadecane completely flowed out was 4.314 minutes and the appearance time of ethylbenzene peak was 4.689 minutes.

Example 1-18

An experiment was conducted substantially as described in Example 1-9, except that the diatomite carrier Z1 was replaced with the diatomite carrier D1.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 5.315 minutes and the appearance time of ethylbenzene peak was 6.427 minutes; and when measured at a column box temperature of 170 °C, the time at which n-hexadecane completely flowed out was 4.612 minutes and the appearance time of ethylbenzene peak was 4.871 minutes.

Example 1-19

An experiment was conducted substantially as described in Example 1-9, except that the diatomite carrier Z1 was replaced with the diatomite carrier D2.

The enrichment material obtained was filled into a packed column with an inner diameter of 2 mm and a length of 200 mm, the chromatographic column was connected into a gas chromatograph, and when measured at a column box temperature of 150 °C, the time at which n-hexadecane completely flowed out was 3.364 minutes and the appearance time of ethylbenzene peak was 4.025 minutes.

Example 1-20

An experiment was conducted substantially as described in Example 1-9, except that the diatomite carrier Z1 was replaced with an untreated diatomite starting material.

The enrichment material obtained was filled into a packed column with an inner diameter of 2 mm and a length of 200 mm, the chromatographic column was connected into a gas chromatograph, and when measured at a column box temperature of 150 °C, the time at which n-hexadecane completely flowed out was 5.926 minutes and the appearance time of ethylbenzene peak was 6.323 minutes.

Example 1-21

This example shows the capacity of the reversible enrichment material of the present application for separating naphthenes from olefins, in which the enrichment material obtained in Example 1-9 was filled into a packed column with an inner diameter of 1.6 mm and a length of 300 mm, and the performance in the separation of naphthenes from olefins was evaluated using n-hexane as a solvent, pentylcyclohexane (having a boiling point of 202 °C) as a representative of naphthenes, and 1-octene (having a boiling point of 121 °C) as a representative of light olefins. The chromatographic column was connected into a gas chromatograph, and when measured at a column box temperature of 155 °C, the time at which pentylcyclohexane completely flowed out was 0.532 minutes and the appearance time of 1-octene peak was 8.936 minutes. The results show that the material has an excellent performance in the separation of naphthenes from olefins.

Comparative Example I-1

5.5362g of diatomite carrier Z1 with a particle size of 150-400 $\mu$m was taken, immersed in 30mL of a solution containing 4% by mass of silver nitrate and 12% by mass of calcium nitrate, naturally evaporated to remove water, dried in an oven at 150 °C for 1 h, to obtain an enrichment material having a mass content of silver nitrate of 20% and a mass content of calcium nitrate of 60%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.618 minutes and the appearance time of ethylbenzene peak was 3.827 minutes; and when measured at a column box temperature of 170 °C, the appearance of ethylbenzene peak was observed before n-hexadecane completely flowed out, which indicated that the separation of n-hexadecane from ethylbenzene failed.

Comparative Example 1-2

5.7805g of diatomite carrier Z1 with a particle size of 150-400 $\mu$m was taken, immersed in 12mL of a solution containing 0.5% by mass of silver nitrate and 15% by mass of manganese nitrate, naturally evaporated to remove water, dried in an oven at 150 °C for 1 h, to obtain an enrichment material having a mass content of silver nitrate of 1% and a mass content of manganese nitrate of 30%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.618 minutes and the appearance time of ethylbenzene peak was 3.758 minutes; and the separation of n-hexadecane from ethylbenzene failed at a column box temperature of 170 °C.

Comparative Example 1-3

5.0526g of diatomite carrier Z1 with a particle size of 150-400 $\mu$m was taken, immersed in 10 mL of a solution containing 0.25% by mass of silver nitrate and 1.25% by mass of copper nitrate, naturally evaporated to remove water, and then dried in an oven at 150 °C for 1 h to obtain an enrichment material having a mass content of silver nitrate of 0.5% and a mass content of copper nitrate of 2.5%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.618 minutes and the appearance time of ethylbenzene peak was 3.855 minutes; and the separation of n-hexadecane from ethylbenzene failed at a column box temperature of 170 °C.

Comparative Example 1-4

5.1527g of diatomite carrier Z1 with a particle size of 150-400 $\mu$m was taken, immersed in 10 mL of a solution containing 0.25% by mass of silver nitrate, 1.25% by mass of chromium nitrate and 0.5% by mass of palladium nitrate, naturally evaporated to remove water, and then dried in an oven at 150 °C for 1 h to obtain an enrichment material having a mass content of silver nitrate of 0.5%, a mass content of chromium nitrate of 2.5% and a mass content of palladium nitrate of 1%, relative to the total amount of the carrier.

The enrichment material obtained was filled into a packed column with an inner diameter of 2 mm and a length of 200 mm, the chromatographic column was connected into a gas chromatograph; when measured at a column box temperature of 150 °C, the time at which n-hexadecane completely flowed out was 3.784 minutes and the appearance time of ethylbenzene peak was 5.223 minutes; and when measured at a column box temperature of 170 °C, the time at which n-hexadecane completely flowed out was 2.645 minutes and the appearance time of ethylbenzene peak was 3.144 minutes.

Comparative Example 1-5

5.7943g of diatomite carrier Z1 with a particle size of 150-400 $\mu$m, immersed in 10 mL of a solution containing 1% by mass of potassium nitrate, 3% by mass of ferric nitrate and 0.8% by mass of chromium nitrate, naturally evaporated to remove water, and then dried in an oven at 120 °C for 4h, to obtain a material having a mass content of potassium nitrate of 1.7%, a mass content of ferric nitrate of 5.2% and a mass content of chromium nitrate of 1.4%, relative to the total amount of the carrier.

The enrichment material obtained was filled into a packed column with an inner diameter of 2 mm and a length of 200 mm, the chromatographic column was connected into a gas chromatograph, and when measured at a column box temperature of 150 °C, the appearance of ethylbenzene peak was observed before the appearance of n-hexadecane peak, which indicated that the material could not retain unsaturated hydrocarbons, and thus could not be used to differentiate saturated hydrocarbons from unsaturated hydrocarbons in a mixture in practical application.

It can be seen from the above examples and comparative examples that, where a same carrier was used, the interaction between the active metal and the metal salt promoter described in the present application can provide the reversible enrichment material obtained with better performance in the separation of saturated hydrocarbons from unsaturated hydrocarbons, and the use of a modified inorganic carrier, especially an inorganic carrier modified by calcining, acid-washing/alkali-washing and drying steps, can further improve the separation performance of the reversible enrichment material obtained.

Example 1-22

This example shows the unsaturated hydrocarbon desorption performance of the reversible enrichment material of the present application, in which the enrichment material obtained in Example 1-8 was used as a representative example to evaluate the unsaturated hydrocarbon desorption performance.

The enrichment material obtained in Example 1-8 was filled into a packed column with an inner diameter of 2 mm and a length of 200 mm to obtain a desired chromatographic column.

Cyclohexane solutions of ethylbenzene, isopropylbenzene, n-butylbenzene, n-pentylbenzene, phenylhexane, n-heptylbenzene, p-di-tert-butylbenzene, 1,3,5-triisopropylbenzene, tetrahydronaphthalene and 1-methylnaphthalene were respectively formulated as standard samples with mass concentrations of 1%, 2%, 5%, 10%, 8%, 5%, 3%, 2%, 2% and 2% respectively; and a mixed standard sample containing decane, n-undecane, n-dodecane, n-tetradecane, n-pentadecane, n-hexadecane, pentylcyclohexane, hexylcyclohexane, decahydronaphthalene, isopropylbenzene, butylbenzene, tetrahydronaphthalene and 1-methylnaphthalene was formulated, with the mass content of saturated hydrocarbons in the mixed standard sample being 82.9%, and the mass content of unsaturated hydrocarbons in the mixed standard sample being 17.1%.

The chromatographic column obtained was connected into a chromatographic column box, the flow rate of the carrier gas $N_2$ was set to be 25mL/min, and the initial temperature of the chromatographic column box was set to be 155 °C. The sample amount was 0.02 μL, after the cyclohexane completely flowed out of the chromatographic column, the peak area of the cyclohexane peak ($A_{CYH\text{-measured}}$) was measured, then the six-way valve was switched to perform a back flushing of the chromatographic column with the carrier gas, the temperature of the column box was raised to 208 °C at the same time, and the peak area of the unsaturated hydrocarbon peak ($A_{USH\text{-measured}}$) was measured; and for the mixed standard sample, after hexadecane completely passed through the chromatographic column, the six-way valve was switched to peformed a back flushing with the carrier gas, the temperature of the column box was raised to 208 °C at the same time, to release unsaturated hydrocarbon from the chromatographic column, and the peak areas of the saturated hydrocarbon peak and the unsaturated hydrocarbon peak were measured separately. The mass content of unsaturated hydrocarbon of each standard sample was calculated using normalization method, and compared with the formulation data, to calculate the recovery rate. The results are shown in Table 1-2.

$$\text{Recovery rate} = 100\% \text{ by mass} \times (A_{USH\text{-measured}}/A_{CYH\text{-measured}})/(m_{USH}/m_{CYH})$$

Table I-2 Experimental results of Example I-21

| Unsaturated hydrocarbons | Recovery rate, % by mass |
|---|---|
| Ethylbenzene | 100.2 |
| Isopropylbenzene | 100.6 |
| n-butylbenzene | 100.2 |
| n-pentylbenzene | 99.9 |
| Phenylhexane | 99.5 |
| n-heptylbenzene | 100.1 |
| p-di-tert-butyl benzene | 99.8 |

(continued)

| Unsaturated hydrocarbons | Recovery rate, % by mass |
|---|---|
| 1,3,5-triisopropylbenzene | 99.9 |
| Tetrahydronaphthalene | 99.3 |
| 1-methylnaphthalene | 98.7 |
| Mixed unsaturated hydrocarbons | 101.2 |

As can be seen from the data shown in Table 1-2, the unsaturated hydrocarbon desorption performance of the reversible enrichment material of the present application is good, the desorption recovery rate of the material for various unsaturated hydrocarbons after their adsorption is more than 98%, so that a reversible adsorption of unsaturated hydrocarbons, especially aromatic hydrocarbons, can be realized.

The following examples show the preparation of the reversible enrichment material and the packed column of the present application, and the effect thereof in the separation of saturated hydrocarbon and unsaturated hydrocarbon components of hydrocarbon-based materials, in which packed columns filled with the reversible enrichment material of the present application were used in gas chromatograph, and the appearance time of ethylbenzene peak and the time at which n-hexadecane completely flowed out (i.e. completely passed through the chromatographic column) at the temperature for measurement were taken as evaluation criteria, and where the appearance time of ethylbenzene peak was greater than the time at which n-hexadecane completely flowed out at the temperature for measurement, it indicated that the reversible enrichment material and the packed column had good separation performance.

Example II-1

Diatomite starting material was mixed with water, slurried and calcined at a high temperature of 800 °C for 9 hours, the calcined diatomite was subjected to acid-washing treatment for 2 times, the temperature of each acid-washing was 100 °C, the time of each acid-washing was 8 hours, the mass ratio of the diatomite starting material on a dry basis to the acid solution was 1:10, the acid used was nitric acid, the mass concentration of the acid solution was 15%, and the acid-washed diatomite was dried at a temperature of 170 °C for 3 hours. The change in the composition of the diatomite before and after the acid-washing treatment is shown in Table II-1.

The diatomite obtained (with a specific surface area of 9 $m^2/g$, and a pore diameter mainly distributed in 50-1000 nm) was ground and screened. 45.2461g of diatomite with a particle size of 125-630 $\mu$m was taken, immersed in 40mL of a solution containing 4.5% by mass of silver nitrate, 2% by mass of ferric nitrate, 1% by mass of ammonium tungstate and 2% by mass of potassium nitrate, naturally evaporated to remove water, and dried in an oven at 150 °C for 1 h to obtain a reversible enrichment filler having a mass content of silver nitrate of 3.9%, a mass content of ferric nitrate of 1.9%, a mass content of ammonium tungstate of 1% and a mass content of potassium nitrate of 1.9%, relative to the total amount of the carrier.

A U-shaped stainless steel column tube with an inner diameter of 2 mm and a length of 200 mm was subjected to a passivation treatment, in which the tube was soaked in a hot sodium hydroxide solution having a mass concentration of 10% for 30 minutes to remove oil stains on the tube wall, washed with deionised water to neutrality, further treated with a dilute hydrochloric acid aqueous solution having a concentration of 1 : 20, washed with water to neutrality, and dried for later use.

The enrichment filler obtained was filled into the passivated U-shaped stainless steel column tube with an inner diameter of 2 mm and a length of 200 mm to obtain a packed column for reversible adsorption of unsaturated hydrocarbon, the packed column was connected into a gas chromatograph, and when measured at a column box temperature of 150 °C, the time at which n-hexadecane completely flowed out was 3.441 minutes and the appearance time of ethylbenzene peak was 5.962 minutes.

Table II-1 Change in the composition of the diatomite before and after acid-washing

| | Before treatment | After treatment |
|---|---|---|
| $SiO_2$ | 73.1 | 79.6 |
| $Al_2O_3$ | 9.7 | 8.3 |
| $Fe_2O_3$ | 5.2 | 3.7 |
| CaO | 3.6 | 2.5 |

(continued)

|  | Before treatment | After treatment |
|---|---|---|
| MgO | 3.1 | 2.3 |
| $K_2O$ | 2.5 | 1.8 |
| MnO | 1.2 | 1.0 |
| $P_2O_5$ | 1.6 | 0.8 |

Example II-2

Zirconia with a particle size of 125-400 $\mu$m was calcined at a high temperature of 650 °C for 8 hours, the calcined zirconia was subjected to an alkali-washing treatment with a sodium hydroxide solution, in which the mass ratio of the zirconia starting material on a dry basis to the alkali solution was 1 : 20, the mass concentration of the alkali solution was 15%, the temperature of alkali-washing was 140 °C, the time of alkali-washing was 20 hours, the alkali-washed zirconia was dried at a temperature of 150 °C for 5 hours.

4.3164g of the zirconia obtained (with a specific surface area of 120 m$^2$/g, and a pore diameter mainly distributed in 2-20 nm) was taken and immersed in 10 mL of a solution containing 2% by mass of copper nitrate, 1% by mass of potassium nitrate, 1% by mass of nickel nitrate and 2% by mass of chromium nitrate, naturally evaporated to remove water, dired in an oven at 160 °C for 1.5h to obtain a reversible enrichment filler having a mass content of copper nitrate of 4.5%, a mass content of potassium nitrate of 2.3%, a mass content of nickel nitrate of 2.3% and a mass content of chromium nitrate of 4.5%, relative to the total amount of the carrier.

The enrichment filler obtained was taken, a U-shaped stainless steel column with an inner diameter of 3 mm and a length of 100 mm was passivated as described in Example II-1, the enrichment filler obtained was filled into the stainless steel column tube to obtain a packed column for reversible adsorption of unsaturated hydrocarbon, the packed column was connected into a gas chromatograph, and when measured at a column box temperature of 150 °C, the time at which n-hexadecane completely flowed out was 2.011 minutes and the appearance time of ethylbenzene peak was 2.997 minutes.

Example II-3

The enrichment filler obtained in Example II-1 was taken, a U-shaped stainless steel column with an inner diameter of 1 mm and a length of 300 mm was passivated as described in Example II-1, the enrichment filler obtained was filled into the stainless steel column tube to obtain a packed column for reversible adsorption of unsaturated hydrocarbon, the packed column was connected into a gas chromatograph, and when measured at a column box temperature of 150 °C, the time at which n-hexadecane completely flowed out was 3.582 minutes and the appearance time of ethylbenzene peak was 7.335 minutes.

Example II-4

The enrichment filler obtained in Example II-1 was taken, a U-shaped stainless steel column with an inner diameter of 1 mm and a length of 500 mm was passivated as described in Example II-1, the enrichment filler obtained was filled into the stainless steel column tube to obtain a packed column for reversible adsorption of unsaturated hydrocarbon, the packed column was connected into a gas chromatograph, and when measured at a column box temperature of 150 °C, the time at which n-hexadecane completely flowed out was 4.795 minutes, and the appearance time of ethylbenzene peak was 8.532 minutes.

Example II-5

The enrichment filler obtained in Example II-1 was taken, a U-shaped stainless steel column with an inner diameter of 1.5 mm and a length of 150 mm was passivated as described in Example II-1, the enrichment filler obtained was filled into the stainless steel column tube to obtain a packed column for reversible adsorption of unsaturated hydrocarbon, the packed column was connected into a gas chromatograph, and when measured at a column box temperature of 155 °C, the time at which n-hexadecane completely flowed out was 2.217 minutes, and the appearance time of ethylbenzene peak was 3.752 minutes.

Example II-6

Diatomite starting material was mixed with water, slurried and calcined at a high temperature of 950 °C for 5 hours, the calcined diatomite was subjected to acid-washing treatment for 2 times, the temperature of each acid-washing was 105 °C, the time of each acid-washing was 10 hours, the mass ratio of the diatomite starting material on a dry basis to the acid solution was 1 20, the acid used was hydrochloric acid, the mass concentration of the acid solution was 10%, the acid-washed diatomite was dried at 150 °C for 4 hours.

The diatomite obtained (with a specific surface area of 10 $m^2/g$, and a pore diameter mainly distributed in 50-1000 nm) was ground and screened. 25.2461g of diatomite with a particle size of 125-400 $\mu$m was taken, immersed in 20ml of a solution containing 2% by mass of silver nitrate, 3% by mass of potassium nitrate, 3% by mass of zinc nitrate and 1% by mass of nickel nitrate, naturally evaporated to remove water, and dried in an oven at 150 °C for 1 h to obtain an enrichment filler having a mass content of silver nitrate of 1.5%, a mass content of potassium nitrate of 2.2%, a mass content of zinc nitrate of 2.2% and a mass content of nickel nitrate of 0.8%, relative to the total amount of the carrier.

A spiral-shaped stainless steel column with an inner diameter of 2 mm and a length of 1000 mm was passivated as described in Example II-1, the enrichment filler obtained was filled into the stainless steel column tube to obtain a packed column for reversible adsorption of unsaturated hydrocarbon, the packed colume was connected into a gas chromatograph, and when measured at a column box temperature of 160 °C, the time at which n-hexadecane completely flowed out was 10.467 minutes and the appearance time of ethylbenzene peak was 15.632 minutes.

Example II-7

The enrichment filler obtained in Example II-6 was taken, a spiral-shaped stainless steel column with an inner diameter of 4 mm and a length of 1200 mm was passivated as described in Example II-1, the enrichment filler obtained was filled into the stainless steel column tube to obtain a packed column for reversible adsorption of unsaturated hydrocarbon, the packed column was connected into a gas chromatograph, and when measured at a column box temperature of 140 °C, the time at which n-hexadecane completely flowed out was 21.598 minutes and the appearance time of ethylbenzene peak was 34.634 minutes.

Example II-8

The diatomite of Example II-6 was ground and sieved, 2.3426g of diatomite having a particle size of 125-630 $\mu$m was taken, immersed in 10 mL of a solution containing 3.5% by mass of copper nitrate, 2.8% by mass of potassium nitrate, 0.7% by mass of iron nitrate and 0.35% by mass of rhodium nitrate, naturally evaporated to remove water, and dried in an oven at 150 °C for 1 h to obtain a reversible enrichment filler having a mass content of copper nitrate of 15%, a mass content of potassium nitrate of 12%, a mass content of iron nitrate of 3% and a mass content of rhodium nitrate of 1.5%, relative to the total amount of the carrier.

The enrichment filler obtained was filled into the stainless steel column tube of Example II-1 to obtain a packed column for reversible adsorption of unsaturated hydrocarbon, the packed column was connted into a gas chromatograph, and when measured at a column box temperature of 150 °C, the time at which n-hexadecane completely flowed out was 3.602 minutes and the appearance time of ethylbenzene peak was 8.897 minutes.

Example II-9

The diatomite of Example II-6 was ground and sieved, 2.8863g of diatomite with a particle size of 125-630 $\mu$m was taken, immersed in 10 mL of a solution containing 4% by mass of silver nitrate, 2% by mass of aluminum nitrate, 3% by mass of cadmium nitrate and 1% by mass of ferric nitrate, naturally evaporated to remove water, and dried in an oven at 150 °C for 1 h to obtain a reversible enrichment filler having a mass content of silver nitrate of 14%, a mass content of aluminum nitrate of 7%, a mass content of cadmium nitrate of 10% and a mass content of ferric nitrate of 3.5%, relative to the total amount of the carrier.

The enrichment filler obtained was filled into the stainless steel column tube of Example II-1 to obtain a packed column for reversible adsorption of unsaturated hydrocarbon, the packed column was connted into a gas chromatograph, and when measured at a column box temperature of 150 °C, the time at which n-hexadecane completely flowed out was 3.692 minutes and the appearance time of ethylbenzene peak was 9.113 minutes.

Comparative Example II-1

3.0032g of silica gel (produced by Qingdao Haiyang Chemical Co., Ltd.) with a particle size of 150-400 $\mu$m, a specific surface area of 418 $m^2/g$ and an average pore diameter of 20.8nm were taken. The silica gel was immersed in 15mL of a solution containing 0.1% by mass of silver nitrate and 2.0% by mass of nickel nitrate, naturally evaporated to remove water, and dried in an oven at 150 °C for 1 h to obtain an enrichment filler having a mass content of silver nitrate of 0.5% and a mass content of nickel nitrate of 10%, relative to the total amount of the carrier.

A U-shaped stainless steel column with an inner diameter of 2 mm and a length of 200 mm was passivated as described in Example II-1, the enrichment filler obtained was filled into the stainless steel column tube to obtain a packed column, the packed column was connected into a gas chromatograph, and when measured at a column box temperature of 160 °C, the time at which n-hexadecane completely flowed out was 3.562 minutes and the appearance time of ethylbenzene peak was 4.036 minutes.

Comparative Example II-2

4.256g of $\gamma$-$Al_2O_3$ with a particle size of 150-400 $\mu$m, a specific surface area of 270 $m^2$/g, and an average pore diameter of 12.1 nm was taken. The $\gamma$-$Al_2O_3$ was immersed in 10 mL of a solution containing 0.5% by mass of silver nitrate and 2.0% by mass of ferric nitrate, naturally evaporated to remove water, and dried in an oven at 150 °C for 1 h to obtain an enrichment filler having a mass content of silver nitrate of 1% and a mass content of ferric nitrate of 4%, relative to the total amount of the carrier.

A U-shaped stainless steel column tube with an inner diameter of 2 mm and a length of 200 mm was passivated as described in Example II-1, the enrichment filler obtained was filled into the stainless steel column tube to obtain a packed column, the packed column was connected into a gas chromatograph, and when measured at a column box temperature of 150 °C, the time at which n-hexadecane completely flowed out was 4.027 minutes and the appearance time of ethylbenzene peak was 4.536 minutes.

The following Examples II-10 to II-20 show the test results of the packed column of the present application for quantitative analysis of saturated and unsaturated hydrocarbons of hydrocarbon-based materials.

Example II-10

4.893g of the diatomite obtained in Example II-6 was taken and immersed in 10 mL of a solution containing 0.5% by mass of silver nitrate, 2.0% by mass of chromium nitrate, 1% by mass of palladium nitrate and 1.0% by mass of potassium nitrate, naturally evaporated to remove water, dried in an oven at 150 °C for 1 hour to obtain an enrichment filler having a mass content of silver nitrate of 1%, a mass content of chromium nitrate of 4%, a mass conent of palladium nitrate of 2% and a mass content of potassium nitrate of 2%, relative to the total amount of the carrier.

A U-shaped stainless steel column with an inner diameter of 2 mm and a length of 200 mm was passivated as described in Example II-1, and the enrichment filler obtained was filled into the stainless steel column tube to obtain a packed column for reversible adsorption of unsaturated hydrocarbon, which was connected into a gas chromatograph.

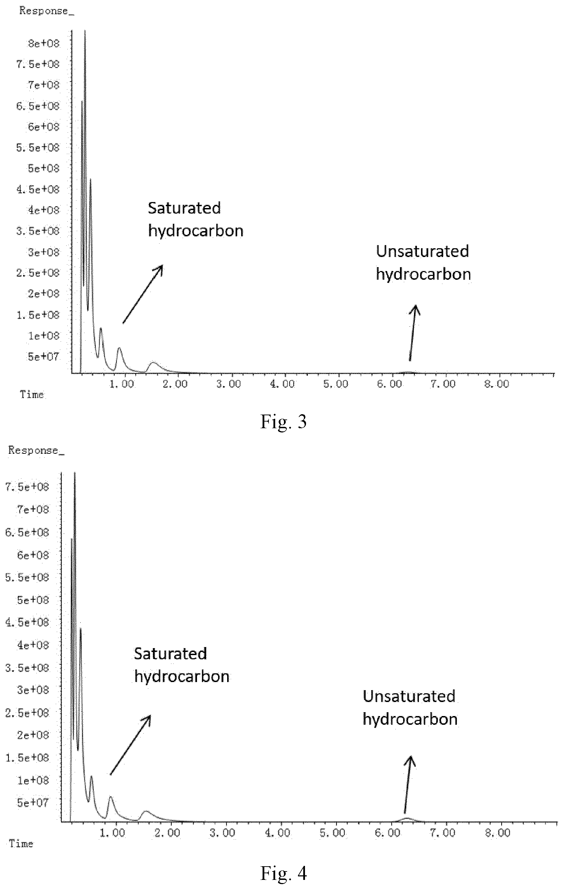

A mixed solution containing decane, n-undecane, n-dodecane, n-tridecane, n-tetradecane, n-pentadecane, hexylcyclohexane, pentylcyclohexane, decahydronaphthalene, isopropylbenzene, butylbenzene, tetrahydronaphthalene and 1-methylnaphthalene was formulated, with the mass content of saturated hydrocarbons being 99.44% and the mass content of unsaturated hydrocarbon being 0.56%. The temperature of the vaporizing chamber was 300 °C, the sample amount was 0.02 $\mu$L, the initial temperature of the column box was 155 °C and was maintained for 5 minutes, the six-way valve was switched at the time of 5 minutes to conduct a back flushing of the gas circuit in the packed column, the temperature of the column box was raised to 200 °C at a rate of 40 °C/min and maintained at 200 °C for 3 minutes. The time at which saturated hydrocarbon completely flowed out was 3 minutes as measured at a carrier gas flow rate of 25 mL/min. The chromatogram obtained is shown in Fig. 3, and the quantitative results are shown in Table II-2.

Table II-2 Quantitative results of the standard solution using the packed column of Example II-10

|  | Measured resul ts/wt% | Formulation data/wt% |
|---|---|---|
| Saturated hydrocarbons | 99.34 | 99.44 |
| Unsaturated hydrocarbons | 0.66 | 0.56 |

Example II-11

The packed column obtained in Example II-10 was used. The test conditions were the same as those employed in Example II-10, and the standard solution was formulated using the reagents described in Example II-10, with a difference that the mass content of saturated hydrocarbon in the formulation was 98.26% and the mass content of unsaturated hydrocarbon in the formulation was 1.74%. The time at which saturated hydrocarbon completely flowed out was 3 minutes as measured at a carrier gas flow rate of 25 mL/min. The chromatogram obtained is shown in Fig. 4, and the quantitative results are shown in Table II-3.

Table II-3 Quantitative results of the standard solution using the packed column of Example II-11

|  | Measured resul ts/wt% | Formulation data/wt% |
| --- | --- | --- |
| Saturated hydrocarbon | 98.12 | 98.26 |
| Unsaturated hydrocarbon | 1.88 | 1.74 |

Example II-12

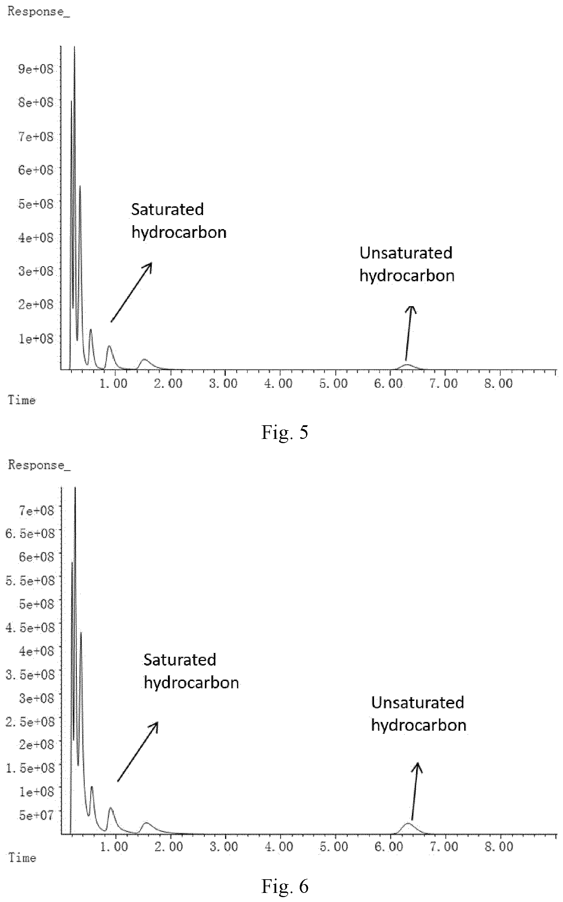

The packed column obtained in Example II-10 was used. The test conditions were the same as those employed in Example II-10, and the standard solution was formulated using the reagents described in Example II-10, with a difference that the mass content of saturated hydrocarbon in the formulation was 96.68% and the mass content of unsaturated hydrocarbon in the formulation was 3.32%. The time at which saturated hydrocarbon completely flowed out was 3 minutes as measured at a carrier gas flow rate of 25 mL/min. The chromatogram obtained is shown in Fig. 5, and the quantitative results are shown in Table II-4.

Table II-4 Quantitative results of the standard solution using the packed column of Example II-12

|  | Measured results/wt% | Formulation data/wt% |
| --- | --- | --- |
| Saturated hydrocarbon | 96.32 | 96.68 |
| Unsaturated hydrocarbon | 3.68 | 3.32 |

Example II-13

The packed column obtained in Example II-10 was used. The test conditions were the same as those employed in Example II-10, and the standard solution was formulated using the reagents described in Example II-10, with a difference that the mass content of saturated hydrocarbon in the formulation was 94.16% and the mass content of unsaturated hydrocarbon in the formulation was 5.84%. The time at which saturated hydrocarbon completely flowed out was 3 minutes as measured at a carrier gas flow rate of 25 mL/min. The chromatogram obtained is shown in Fig. 6, and the quantitative results are shown in Table II-5.

Table II-5 Quantitative results of the standard solution using the packed column of Example II-13

|  | Measured results/wt% | Formulation data/wt% |
| --- | --- | --- |
| Saturated hydrocarbon | 93.98 | 94.16 |
| Unsaturated hydrocarbon | 6.02 | 5.84 |

Example II-14

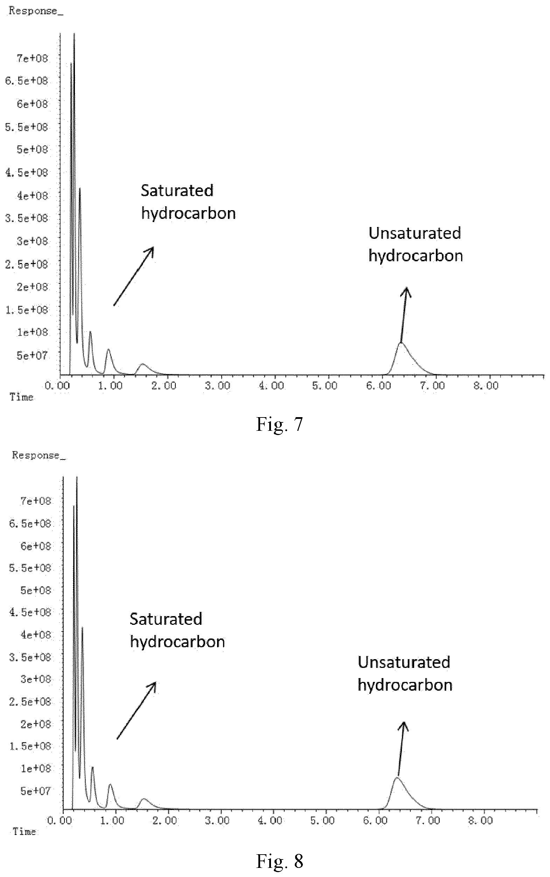

The packed column obtained in Example II-10 was used. The test conditions were the same as those employed in Example II-10, and the standard solution was formulated using the reagents described in Example II-10, with a difference that the mass content of saturated hydrocarbon in the formulation was 77.26% and the mass content of unsaturated hydrocarbon in the formulation was 22.74%. The time at which saturated hydrocarbon completely flowed out was 3 minutes as measured at a carrier gas flow rate of 25 mL/min. The chromatogram obtained is shown in Fig. 7, and the quantitative results are shown in Table II-6.

Table II-6 Quantitative results of the standard solution using the packed column of Example II-14

|  | Measured results/wt% | Formulation data/wt% |
| --- | --- | --- |
| Saturated hydrocarbon | 77.21 | 77.26 |
| Unsaturated hydrocarbon | 22.79 | 22.74 |

Example II-15

The packed column obtained in Example II-10 was used. The test conditions were the same as those employed in Example II-10, and the standard solution was formulated using the reagents described in Example II-10, with a difference that the mass content of saturated hydrocarbon in the formulation was 76.04% and the mass content of unsaturated hydrocarbon in the formulation was 23.96%. The time at which saturated hydrocarbon completely flowed out was 3 minutes as measured at a carrier gas flow rate of 25 mL/min. The chromatogram obtained is shown in Fig. 8, and the quantitative results are shown in Table II-7.

Table II-7 Quantitative results of the standard solution using the packed column of Example II-15

|  | Measured results/wt% | Formulation data/wt% |
|---|---|---|
| Saturated hydrocarbon | 75.91 | 76.04 |
| Unsaturated hydrocarbon | 24.09 | 23.96 |

Example II-16

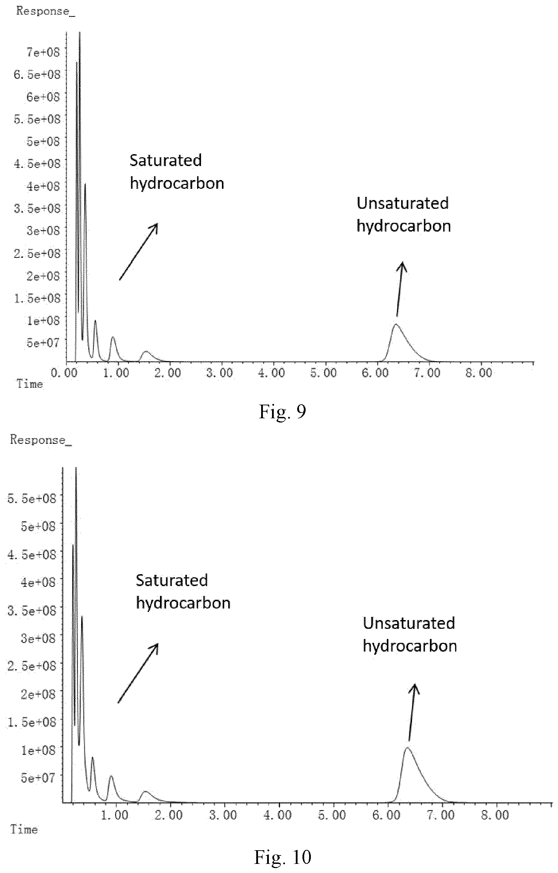

The packed column obtained in Example II-10 was used. The test conditions were the same as those employed in Example II-10, and the standard solution was formulated using the reagents described in Example II-10, with a difference that the mass content of saturated hydrocarbon in the formulation was 72.40% and the mass content of unsaturated hydrocarbon in the formulation was 27.60%. The time at which saturated hydrocarbon completely flowed out was 3 minutes as measured at a carrier gas flow rate of 25 mL/min. The chromatogram obtained is shown in Fig. 9, and the quantitative results are shown in Table II-8.

Table II-8 Quantitative results of the standard solution using the packed column of Example II-16

|  | Measured results/wt% | Formulation data/wt% |
|---|---|---|
| Saturated hydrocarbon | 72.08 | 72.40 |
| Unsaturated hydrocarbon | 27.92 | 27.60 |

Example II-17

The packed column obtained in Example II-10 was used. The test conditions were the same as those employed in Example II-10, and the standard solution was formulated using the reagents described in Example II-10, with a difference that the mass content of saturated hydrocarbon in the formulation was 67.32% and the mass content of unsaturated hydrocarbon in the formulation was 32.68%. The time at which saturated hydrocarbon completely flowed out was 3 minutes as measured at a carrier gas flow rate of 25 mL/min. The chromatogram obtained is shown in Fig. 10, and the quantitative results are shown in Table II-9.

Table II-9 Quantitative results of the standard solution using the packed column of Example II-17

|  | Measured results/wt% | Formulation data/wt% |
|---|---|---|
| Saturated hydrocarbon | 66.88 | 67.32 |
| Unsaturated hydrocarbon | 33.12 | 32.68 |

Example II-18

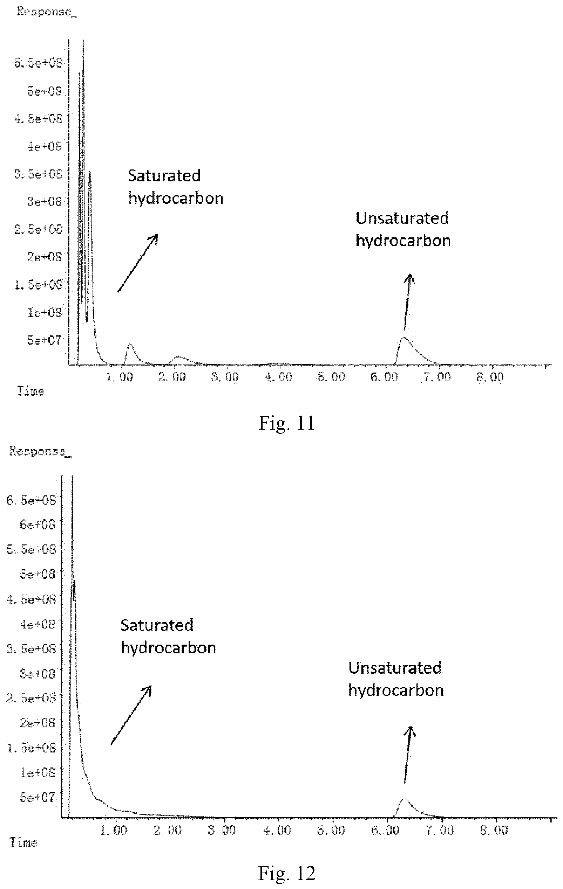

The packed column obtained in Example II-10 was used. A mixed solution containing decane, n-undecane, n-dodecane, n-tetradecane, n-hexadecane, hexylcyclohexane, pentylcyclohexane, decahydronaphthalene, isopropylbenzene, butylbenzene, tetrahydronaphthalene and 1-methylnaphthalene was formulated, in which the mass content of saturated hydrocarbon was 82.90%, and the mass content of unsaturated hydrocarbon was 17.10%. The test conditions were the same as those employed in Example II-10, and the time at which saturated hydrocarbon completely flowed out was 4.6 minutes as measured at a carrier gas flow rate of 25 mL/min. The chromatogram obtained is shown in Fig. 11, and the quantitative results are shown in Table II-10.

Table II-10 Quantitative results of the standard solution using the packed column of Example II-18

|  | Measured results/wt% | Formulation data/wt% |
|---|---|---|
| Saturated hydrocarbon | 82.44 | 82.90 |
| Unsaturated hydrocarbon | 17.56 | 17.10 |

Example II-19

The packed column obtained in Example II-10 was used, and the test conditions were the same as those employed in Example II-10, with a difference that a real aviation fuel sample II-A was used as the test sample, in which the aviation fuel A was an aviation kerosene fraction obtained by hydrotreating straight-run aviation kerosene, which had a distillation range of 160-240 °C, and the unsaturated hydrocarbon content of the aviation fuel A measured by a fluorescence chromatographic column is shown in Table II-11. The chromatogram obtained is shown in Fig. 12, and the volume content of the unsaturated hydrocarbon obtained by converting its mass content into volume content is shown in Table II-11.

Table II-11 Quantitative results of the sample using the packed column of Example II-19

|  | Measured results/v% | Fluorescence column chromatography resul ts/v% |
|---|---|---|
| Saturated hydrocarbon | 89.44 | 88.70 |
| Unsaturated hydrocarbon | 10.56 | 11.30 |

Example II-20

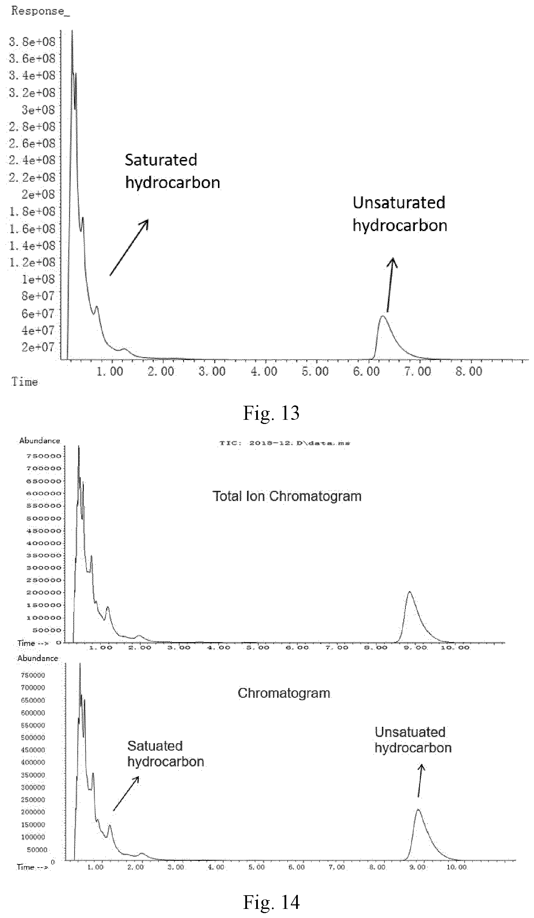

The packed column obtained in Example II-10 was used, and the test conditions were the same as those employed in Example II-10, with a difference that a real aviation fuel sample II-B was used as the test sample, in which the aviation fuel sample II-B was an aviation kerosene fraction obtained by hydrocracking a heavy oil, which had a distillation range of 160-280 °C, and the unsaturated hydrocarbon content of the aviation fuel sample II-B measured by a fluorescence chromatographic column is shown in Table II-12. The chromatogram obtained is shown in Fig. 13, and the volume content of the unsaturated hydrocarbon obtained by converting its mass content into volume content is shown in Table II-12.

Table II-12 Quantitative results of the sample using the packed column of Example II-20

|  | Measured results/v% | Fluorescence column chromatography resul ts/v% |
|---|---|---|
| Saturated hydrocarbon | 83.87 | 83.90 |
| Unsaturated hydrocarbon | 16.13 | 16.10 |

Example II-21

This example shows the effect of the packed column of the present application in hydrocarbon component analysis of hydrocarbon-based materials, in which the packed column obtained in Example 11-10 was used as a representative example to evaluate the performance in hydrocarbon component analysis.

The packed column obtained in Example 11-10 was used, and connected into a gas chromatograph-mass spectrometer, an empty capillary column with a length of 30m and an inner diameter of 0.25 mm was connected between a sample inlet and a six-way valve to buffer the vacuum degree of the system, in which the temperature of the sample inlet was 300 °C, and the split ratio of the sample inlet was 20 : 1. The component flowing out of the packed column was divided into two parts after passing through the splitter, one part entered the flame ionization detector, the other part entered the mass spectrometer, and the split ratio of the two parts was 10 : 1.

The real aviation fuel sample II-B used in Example 11-20 was used as a test sample to perform online separation and detailed analysis of hydrocarbon components of the sample, the mass spectrum and chromatogram obtained are shown in the upper half and the lower half of Fig. 14, respectively, and the quantitative results of mass spectrometry for each type of compound are shown in Table 11-13, in which the reference contents are data obtained by the analysis method of the reference example. It should be noted that since the quantitative results of this example are obtained after the classification of each type of compound by mass spectrometry, there is a mass deducted associated with mass spectrometry, so that the results are slightly different from the unsaturated hydrocarbon content directly determined by gas chromatography in Example 11-20.

Table II-13 Detailed hydrocarbon composition results for sample II-B

| Hydrocarbon composition of the fraction | Measured content (m%) | Reference content (m%) |
|---|---|---|
| Paraffins | 50.6 | 51.4 |
| Noncondensed cycloparaffins | 24.8 | 25.6 |
| Condensed dicycloparaffins | 7.0 | 6.6 |
| Condensed Tricycloparaffins | 0.7 | 0.9 |
| Total naphthenes | 32.5 | 33.1 |
| Total saturated hydrocarbons | 83.1 | 84.5 |
| Olefins | 0.6 | -- |
| Alkylbenzenes | 12.3 | 12.0 |
| Indanes or tetralins, or both | 2.3 | 2.1 |
| Indenes | 0.1 | 0.2 |
| Total monocyclic aromatics | 14.7 | 14.3 |
| Naphthalene | 0.3 | 0.2 |
| Naphthalenes | 1.2 | 1.0 |
| Acenaphthenes | 0.1 | 0 |
| Acenaphthylenes | 0 | 0 |
| Total bicyclic aromatics | 1.6 | 1.2 |
| Tricyclic aromatics | 0 | 0 |
| Total aromatics | 16.3 | 15.5 |
| Total | 100.0 | 100 |

Examples II-22 to II-27 are provided to illustrate the system of the present application for separating and analyzing hydrocarbon components of hydrocarbon-based materials and the method of its use.

Example II-22

The device shown in Fig. 1 was used as the experimental equipment, the embedded packed column had an inner diameter of 3 mm, a length of 400 mm, the quartz capillary column 2 had an inner diameter of 0.53 mm and a length of 10m, the quartz capillary column 5 had an inner diameter of 0.32 mm and a length of 20m, the quartz capillary column 7 and the quartz capillary column 8 were configured at a split ratio of 5:1, and the enrichment filler used was the enrichment filler of Example 11-10.

A mixed solution containing decane, n-undecane, n-dodecane, n-tetradecane, n-hexadecane, hexylcyclohexane, pentylcyclohexane, decahydronaphthalene, dodecene, isopropylbenzene, butylbenzene, tetrahydronaphthalene and 1-methylnaphthalene was formulated, in which the mass content of saturated hydrocarbon was 81.05% and the mass content of unsaturated hydrocarbon was 18.95%.

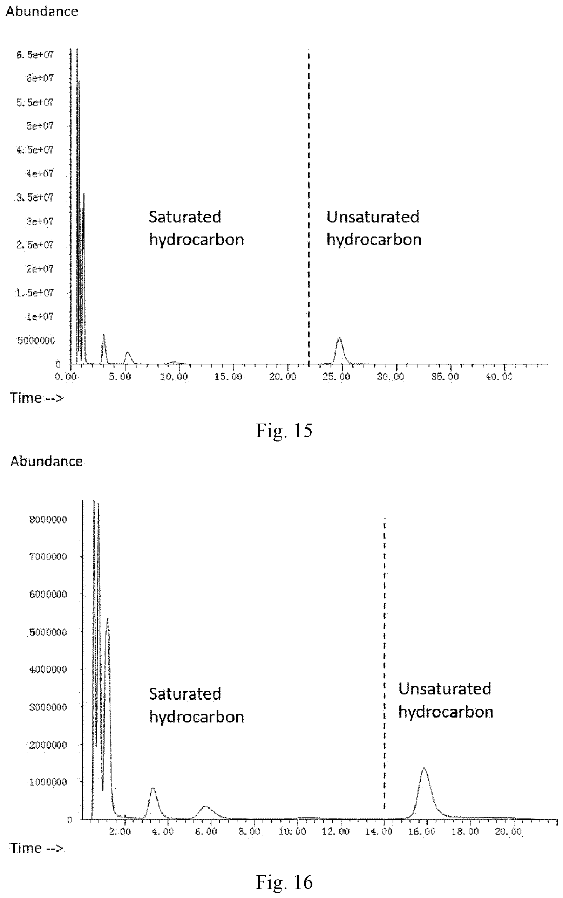

The solution obtained was used as a test object, the operating conditions of the system for separating and analyzing the hydrocarbon components in the hydrocarbon-based material include: a temperature of dividing/undividing sample inlet of 300 °C, a split ratio of 20:1, a carrier gas of helium, a flow rate of 10 mL/min, a temperature of the flame ionization detector of 300 °C, and a temperature of the mass spectrometer transmission line of 260 °C. The temperature of the valve box was 180 °C, the switching time of the six-way valve was 22 minutes, the initial temperature of the column box was 155 °C and maintained for 22 minutes, then the temperature was increased to 206 °C at a rate of 40 °C/min and maintained for 3 minutes, and the sample amount was 0.2 $\mu$L. The chromatogram obtained is shown in Fig. 15, in which the species eluted at a time before 22 minutes are saturated hydrocarbon components, and the species eluted at a time after 22 minutes are unsaturated hydrocarbon components. The device was used for determining the detailed hydrocarbon composition, which took 27 minutes. The detailed hydrocarbon composition results of the formulated sample are shown in Table 11-14.

Table 11-14 Detailed hydrocarbon composition data obtained by mass spectrometry

| Fractions | Measured content (m%) | Formulation content (m%) |
| --- | --- | --- |
| Paraffins | 40.1 | 42.77 |
| Noncondensed cycloparaffins | 27.7 | 26.47 |
| Condensed dicycloparaffins | 11.7 | 11.81 |
| Condensed Tricycloparaffins | 0 | 0 |
| Total naphthenes | 39.4 | 38.28 |
| Total saturated hydrocarbons | 79.5 | 81.05 |
| Olefins | 2.1 | 2.23 |
| Alkylbenzenes | 11.9 | 9.55 |
| Indanes or tetralins, or both | 5.6 | 6.54 |
| Indenes | 0 | 0 |
| Total monocyclic aromatics | 17.5 | 16.09 |
| Naphthalene | 0.2 | 0 |
| Naphthalenes | 0.7 | 0.63 |
| Acenaphthenes | 0 | 0 |
| Acenaphthylenes | 0 | 0 |
| Total bicyclic aromatics | 0.9 | 0.63 |
| Tricyclic aromatics | 0 | 0 |
| Total aromatics | 18.4 | 16.72 |

Example 11-23

An experiment was conducted using the device described in Example 11-22, with a difference that the embedded packed column had an inner diameter of 2.5 mm and a length of 200 mm, the quartz capillary column 2 had an inner diameter of 0.32 mm and a length of 5 m, the quartz capillary column 5 had an inner diameter of 0.32 mm and a length of 10 m, the quartz capillary column 7 and the quartz capillary column 8 were configured at a split ratio of 10:1, and the enrichment filler used was the enrichment filler of Example 11-10.

The device was used to determine the detailed hydrocarbon composition of the solution formulated in Example 11-22, and the operating conditions of the system for separating and analyzing hydrocarbon components in the hydrocarbon-based material were the same, except that the six-way valve switching time was 14 minutes and the initial temperature of the column box was maintained for 14 minutes. The chromatogram obtained is shown in Fig. 16, in which the species eluted at a time before 14 minutes are saturated hydrocarbon components, and the species eluted at a time after 14 minutes are unsaturated hydrocarbon components. The device was used for determining the detailed hydrocarbon composition, which took 19 minutes. The detailed hydrocarbon composition results for the formulated sample are shown in Table 11-15.

Table 11-15 Detailed hydrocarbon composition data obtained by mass spectrometry

| Fractions | Measured content (m%) | Formulation content (m%) |
| --- | --- | --- |
| Paraffins | 40.3 | 42.77 |
| Noncondensed cycloparaffins | 27.2 | 26.47 |
| Condensed dicycloparaffins | 11.9 | 11.81 |

(continued)

| Fractions | Measured content (m%) | Formulation content (m%) |
|---|---|---|
| Condensed Tricycloparaffins | 0 | 0 |
| Total naphthenes | 39.1 | 38.28 |
| Total saturated hydrocarbons | 79.4 | 81.05 |
| Olefins | 2.4 | 2.23 |
| Alkylbenzenes | 11.1 | 9.55 |
| Indanes or tetralins, or both | 6.1 | 6.54 |
| Indenes | 0 | 0 |
| Total monocyclic aromatics | 17.2 | 16.09 |
| Naphthalene | 0.2 | 0 |
| Naphthalenes | 0.8 | 0.63 |
| Acenaphthenes | 0 | 0 |
| Acenaphthylenes | 0 | 0 |
| Total bicyclic aromatics | 1 | 0.63 |
| Tricyclic aromatics | 0 | 0 |
| Total aromatics | 18.2 | 16.72 |

Example 11-24

An experiment was conducted using the device described in Example 11-22, with a difference that the embedded packed column had an inner diameter of 5 mm and a length of 500 mm, the quartz capillary column 2 had an inner diameter of 0.2 mm and a length of 1 m, the quartz capillary column 5 had an inner diameter of 0.25 mm and a length of 40 m, the quartz capillary column 7 and the quartz capillary column 8 were configured at a split ratio of 15:1, and the enrichment filler used was the enrichment filler of Example 11-10.

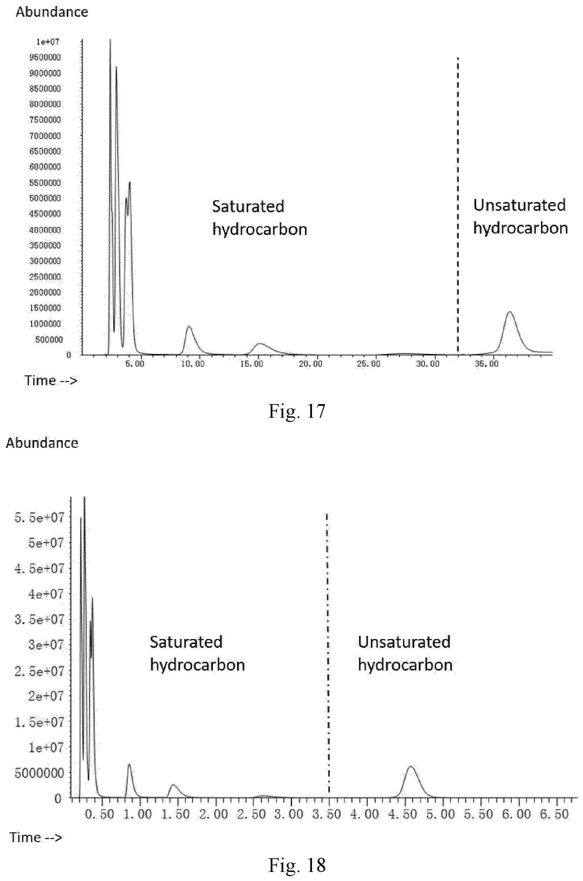

The device was used to determine the detailed hydrocarbon composition of the solution formulated in Example 11-22, and the operating conditions of the system for separating and analyzing hydrocarbon components in the hydrocarbon-based material were the same, except that the six-way valve switching time was 32 minutes and the initial temperature of the column box was maintained for 32 minutes. The chromatogram obtained is shown in Fig. 17, in which the species eluted at a time before 32 minutes are saturated hydrocarbon components, and the species eluted at a time after 32 minutes are unsaturated hydrocarbon components. The device was used for determining the detailed hydrocarbon composition, which took 40 minutes. The detailed hydrocarbon composition results for the formulated sample are shown in Table 11-16.

Table 11-16 Detailed hydrocarbon composition data obtained by mass spectrometry

| Fractions | Measured content (m%) | Formulation content (m%) |
|---|---|---|
| Paraffins | 39.1 | 42.77 |
| Noncondensed cycloparaffins | 28.3 | 26.47 |
| Condensed dicycloparaffins | 12.3 | 11.81 |
| Condensed Tricycloparaffins | 0 | 0 |
| Total naphthenes | 40.6 | 38.28 |
| Total saturated hydrocarbons | 79.7 | 81.05 |
| Olefins | 1.8 | 2.23 |
| Alkylbenzenes | 11.6 | 9.55 |
| Indanes or tetralins, or both | 5.9 | 6.54 |

(continued)

| Fractions | Measured content (m%) | Formulation content (m%) |
|---|---|---|
| Indenes | 0 | 0 |
| Total monocyclic aromatics | 17.5 | 16.09 |
| Naphthalene | 0.1 | 0 |
| Naphthalenes | 0.9 | 0.63 |
| Acenaphthenes | 0 | 0 |
| Acenaphthylenes | 0 | 0 |
| Total bicyclic aromatics | 1 | 0.63 |
| Tricyclic aromatics | 0 | 0 |
| Total aromatics | 18.5 | 16.72 |

Example II-25

The device shown in Fig. 2 was used as the experimental equipment, the embedded packed column had an inner diameter of 1.6 mm, and a length of 300 mm, the quartz capillary column 2 had an inner diameter of 0.25 mm and a length of 5 m, the quartz capillary column 5 had an inner diameter of 0.32 mm and a length of 0.1 m, the quartz capillary column 7 and the quartz capillary column 8 were configured at a split ratio of 10 : 1, and the enrichment filler used was the enrichment filler of Example 11-10.

The device was used to determine the detailed hydrocarbon composition of the solution formulated in Example 11-22, and the operating conditions of the system for separating and analyzing hydrocarbon components in the hydrocarbon-based material were the same, except that the six-way valve 11 was opened at 0.02 minutes so that the packed column was connected into the gas circuit; the six-way valve 3 was opened at 3.5 minutes so that a back flushing of the gas circuit was realized in the method, the initial temperature of the column box was 155 °C and maintained for 3.5 minutes, and then the temperature was raised to 206 °C at a rate of 40 °C/min, and maintained for 2 min; the six-way valve 3 was closed at 6.5 minutes so that the gas flow was restored to the positive direction, and the six-way valve 11 was closed at 6.6 minutes so that the packed column was isolated from the gas circuit of the system; and the sample amount was 0.2 μL. The chromatogram obtained is shown in Fig. 18, in which the species eluted at a time before 3.5 minutes are saturated hydrocarbon components, and the species eluted at a time after 3.5 minutes are unsaturated hydrocarbon components. The device was used for determining the detailed hydrocarbon composition, which took 8 minutes. The detailed hydrocarbon composition results for the formulated sample are shown in Table 11-17.

Table II-17 Detailed hydrocarbon composition data obtained by mass spectrometry

| Fractions | Measured content (m%) | Formulation content (m%) |
|---|---|---|
| Paraffins | 41.8 | 42.77 |
| Noncondensed cycloparaffins | 26.9 | 26.47 |
| Condensed dicycloparaffins | 12.1 | 11.81 |
| Condensed Tricycloparaffins | 0 | 0 |
| Total naphthenes | 39.0 | 38.28 |
| Total saturated hydrocarbons | 80.8 | 81.05 |
| Olefins | 2.1 | 2.23 |
| Alkylbenzenes | 10.2 | 9.55 |
| Indanes or tetralins, or both | 6.2 | 6.54 |
| Indenes | 0 | 0 |
| Total monocyclic aromatics | 16.4 | 16.09 |
| Naphthalene | 0.1 | 0 |

(continued)

| Fractions | Measured content (m%) | Formulation content (m%) |
|---|---|---|
| Naphthalenes | 0.6 | 0.63 |
| Acenaphthenes | 0 | 0 |
| Acenaphthylenes | 0 | 0 |
| Total bicyclic aromatics | 0.7 | 0.63 |
| Tricyclic aromatics | 0 | 0 |
| Total aromatics | 17.1 | 16.72 |

Example II-26

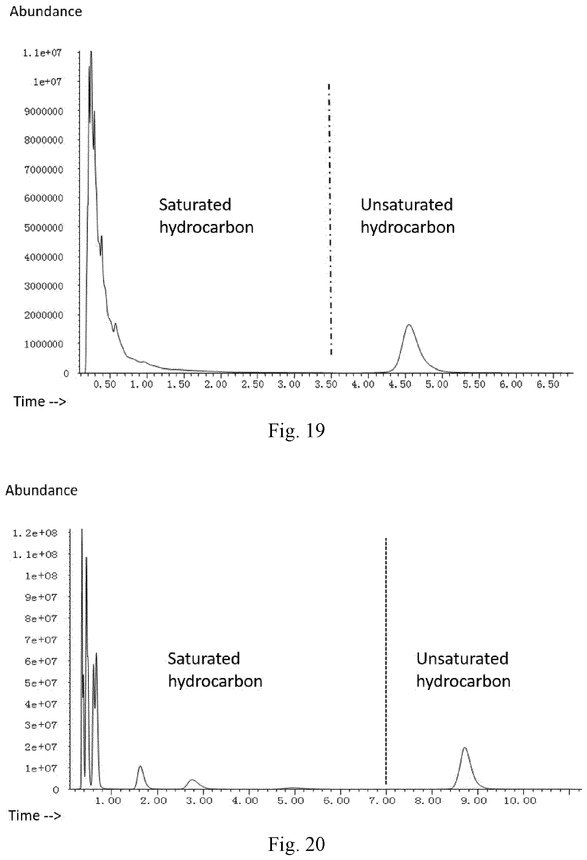

The hydrocarbon composition analyzing device used in Example 11-25 was used as the experimental equipment, a real aviation kerosene sample II-C was used as a test object, the test conditions were the same as those employed in Example 11-25. The chromatogram obtained is shown in Fig. 19, the time spent is 6 minutes, and the results of the detailed hydrocarbon composition analysis are shown in Table 11-18, in which the measured content is the detailed hydrocarbon composition including that for olefins obtained after the separation of olefins from naphthenes using the packed column of the present application, and the reference content is the detailed hydrocarbon composition determined by conventional mass spectrometry. As can be seen from the comparison of the test results, the system of the present application overcomes the defect of conventional mass spectrometry that it cannot distinguish olefins from naphthenes.

Table 11-18 Detailed hydrocarbon composition data obtained by mass spectrometry

| Fractions | Measured content (m%) | Reference content (m%) |
|---|---|---|
| Paraffins | 27.3 | 27.0 |
| Noncondensed cycloparaffins | 41.5 | 44.6 |
| Condensed dicycloparaffins | 13.6 | 14.8 |
| Condensed Tricycloparaffins | 1.8 | 2.0 |
| Total naphthenes | 56.9 | 61.4 |
| Total saturated hydrocarbons | 84.2 | 88.4 |
| Olefins | 3.6 | - |
| Alkylbenzenes | 7.4 | 7.7 |
| Indanes or tetralins, or both | 4.2 | 3.7 |
| Indenes | 0.1 | 0 |
| Total monocyclic aromatics | 11.7 | 11.4 |
| Naphthalene | 0 | 0.1 |
| Naphthalenes | 0.3 | 0.1 |
| Acenaphthenes | 0.1 | 0 |
| Acenaphthylenes | 0.1 | 0 |
| Total bicyclic aromatics | 0.5 | 0.2 |
| Tricyclic aromatics | 0 | 0 |
| Total aromatics | 12.2 | 11.6 |

Example 11-27

The device for analyzing detailed hydrocarbon composition of the hydrocarbon fuel used in Example 11-25 was used as the experimental equipment, and a real diesel oil sample II-D was used as a test object. The operating conditions of the system for separating and analyzing hydrocarbon components of the hydrocarbon-based material were the same as those employed in Example 11-25 except that the initial temperature of the column box was 165 °C, and the device was used for determining the detailed hydrocarbon composition of the sample II-D, which took 15 minutes. The results of the detailed hydrocarbon composition analysis are shown in Table 11-19, in which the measured content is the detailed hydrocarbon composition including that for olefins obtained after the separation of olefins from naphthenes using the packed column of the present application, and the reference content is the detailed hydrocarbon composition determined by conventional mass spectrometry. As can be seen from the comparison of the test results, the system of the present application overcomes the defect of conventional mass spectrometry that it cannot distinguish olefins from naphthenes.

Table 11-19 Detailed hydrocarbon composition data obtained by mass spectrometry

| Fractions | Measured content (m%) | Reference content (m%) |
|---|---|---|
| Paraffins | 43.9 | 44.3 |
| Noncondensed cycloparaffins | 24.7 | 26.7 |
| Condensed dicycloparaffins | 10.4 | 11.2 |
| Condensed Tricycloparaffins | 2.7 | 2.8 |
| Total naphthenes | 37.8 | 40.7 |
| Total saturated hydrocarbons | 81.7 | 85.0 |
| Olefins | 2.7 | - |
| Alkylbenzenes | 7.1 | 7.3 |
| Indanes or tetralins, or both | 4.2 | 4.6 |
| Indenes | 1.1 | 0.9 |
| Total monocyclic aromatics | 12.4 | 12.8 |
| Naphthalene | 0.4 | 0.1 |
| Naphthalenes | 1.5 | 0.9 |
| Acenaphthenes | 0.8 | 0.6 |
| Acenaphthylenes | 0.5 | 0.3 |
| Total bicyclic aromatics | 3.2 | 1.9 |
| Tricyclic aromatics | 0.3 | 0.3 |
| Total aromatics | 15.6 | 15.0 |

The following examples are provided to illustrate the method of the present application for separating and analyzing hydrocarbon components of hydrocarbon-based materials.

Example III-1

Diatomite starting material was mixed with water, slurried and calcined at a high temperature of 950 °C for 12 hours, the calcined diatomite was subjected to acid-washing treatment for 4 times, the temperature of each acid-washing was 135 °C, the time of each acid-washing was 8 hours, the mass ratio of the diatomite starting material on a dry basis to the acid solution was 1 : 5, the acid used was sulfuric acid, the mass concentration of the acid solution was 30%, the acid-washed diatomite was dried at 120 °C for 4 hours, and the change in the composition of the diatomite before and after treatment is shown in Table III-1.
The diatomite obtained (with a specific surface area of 17 $m^2$/g and a pore diameter mainly distributed in 50-1000 nm) was ground and screened. 24.8532g of diatomite with a particle size of 125-500 $\mu$m was taken, immersed in 20mL of a solution containing 5.5% by mass of silver nitrate, 3% by mass of ferric nitrate, 1.2% by mass of ammonium tungstate and 3% by mass of potassium nitrate, naturally evaporated to remove water, and dried in an oven at 150 °C for 1 h to obtain an enrichment filler having a mass content of silver nitrate of 4.4%, a mass content of ferric nitrate of 2.4%, a mass content of ammonium tungstate of 1% and a mass content of potassium nitrate of 2.4%, relative to the total amount of the carrier.

A U-shaped stainless steel column tube with an inner diameter of 3 mm and a length of 200 mm was subjected to a passivation treatment, in which the tube was soaked in a hot sodium hydroxide solution having a mass concentration of 10% for 30 minutes to remove oil stains on the tube wall, washed with deionised water to neutrality, further treated with a dilute hydrochloric acid aqueous solution having a concentration of 1:20, washed with water to neutrality, and dried for later use.

The enrichment material obtained was filled into the treated stainless steel column tube to make an enrichment column required for the method, and connected it into the device shown in Fig. 1, in which the quartz capillary column 2 had an inner diameter of 0.32 mm and a length of 10 m, the quartz capillary column 5 had an inner diameter of 0.32 mm and a length of 0.5 m, and the quartz capillary column 7 and the quartz capillary column 8 were configured at a split ratio of 10 : 1.

The experimental conditions employed in the method include: a temperature of the dividing/undividing sample inlet of 280 °C, a split ratio of 20:1, a carrier gas of helium, a flow rate of 10 mL/min, a temperature of the flame ionization detector (FID) of 260 °C, and a temperature of the mass spectrometer transmission line of 260 °C. The temperature of the valve box was 180 °C, the switching time of the six-way valve was 7 minutes, the initial temperature of the column box was 155 °C and maintained for 7min, then the temperature was increased to 206 °C at a rate of 40 °C/min and maintained for 3 minutes, and the sample amount was 0.2 $\mu$L.

The formlated sample III-A was used as a test object, and a detailed hydrocarbon composition analysis of the sample III-A was conducted using the method of the present application under the conditions as described above. The chromatogram obtained is shown in Fig. 20, in which the species eluted at a time before 7 minutes are saturated hydrocarbon components, and the species eluted at a time after 7 minutes are unsaturated hydrocarbon components.

The results of the total amount of unsaturated and saturated hydrocarbons of the formulated sample are shown in Table III-2, and the results of the detailed hydrocarbon composition analysis are shown in Table III-3.

Table III-1 Change in the composition of the diatomite before and after acid-washing

|  | Before treatment | After treatment |
|---|---|---|
| $SiO_2$ | 73.1 | 84.7 |
| $Al_2O_3$ | 9.7 | 7.9 |
| $Fe_2O_3$ | 5.2 | 3.4 |
| CaO | 3.6 | 0.9 |
| MgO | 3.1 | 1.4 |
| $K_2O$ | 2.5 | 0.8 |
| MnO | 1.2 | 0.7 |
| $P_2O_5$ | 1.6 | 0.2 |

Table III-2 Total amount of saturated and unsaturated hydrocarbons of sample III-A (FID data)

| Fractions | Measured content (m%) | Formulation content (m%) |
|---|---|---|
| Saturated hydrocarbon | 77.54 | 78.38 |
| Unsaturated hydrocarbon | 22.46 | 21.62 |

Table III-3 Detailed hydrocarbon composition data of sample III-A (mass spectrometry data)

| Fractions | Measured content (m%) | Formulation content (m%) |
|---|---|---|
| Paraffins | 38.8 | 41.36 |
| Noncondensed cycloparaffins | 26.8 | 25.6 |
| Condensed dicycloparaffins | 11.7 | 11.43 |
| Condensed Tricycloparaffins | 0 | 0 |
| Olefins | 4.3 | 5.45 |

(continued)

| Fractions | Measured content (m%) | Formulation content (m%) |
|---|---|---|
| Alkylbenzenes | 11.8 | 9.23 |
| Indanes or tetralins, or both | 5.6 | 6.32 |
| Indenes | 0 | 0 |
| Naphthalene | 0.3 | 0 |
| Naphthalenes | 0.7 | 0.61 |
| Acenaphthenes | 0 | 0 |
| Acenaphthylenes | 0 | 0 |
| Tricyclic aromatics | 0 | 0 |

Example III-2

$\gamma$-$Al_2O_3$ with a particle size of 150-450 $\mu$m was calcined at a high temperature of 500 °C for 5 hours, the calcined $\gamma$-$Al_2O_3$ was subjected to alkali-washing treatment using a sodium hydroxide solution, the mass ratio of the $\gamma$-$Al_2O_3$ starting material on a dry basis to the alkali solution was 1 : 15, the temperature of alkali-washing was 100 °C, the time of alkali-washing was 15 hours, the mass concentration of the alkali solution was 5%, and the alkali-washed $\gamma$-$Al_2O_3$ was dried at 120 °C for 3.5 hours.

3.3542g of the $\gamma$-$Al_2O_3$ obtained (with a specific surface area of 261 $m^2$/g, and a pore diameter mainly distributed in 2-50 nm) was taken, immersed in 10 mL of a solution containing 3% by mass of silver nitrate, 1% by mass of lithium nitrate, 2% by mass of manganese nitrate and 0.5% by mass of ferric nitrate, naturally evaporated to remove water, dried in an oven at 125 °C for 3 hours, to obtain a reversible enrichment filler having a mass content of silver nitrate of 9%, a mass content of lithium nitrate of 3%, a mass content of manganese nitrate of 6% and a mass content of ferric nitrate of 1.5%, relative to the total amount of the carrier.

An experiment was conducted using the above-described enrichment filler, using the method described in Example III-1, with a difference that the embedded packed column had an inner diameter of 3.2 mm, a length of 150 mm, the quartz capillary column 2 had an inner diameter of 0.32 mm and a length of 30 m, the quartz capillary column 5 had an inner diameter of 0.32 mm and a length of 1 m, and the quartz capillary column 7 and the quartz capillary column 8 were configured at a split ratio of 6 : 1.

The experimental conditions employed in the method include: a temperature of the dividing/undividing sample inlet of 280 °C, a split ratio of 20:1, a carrier gas of helium, a flow rate of 8 mL/min, a temperature of the flame ionization detector of 260 °C, and a temperature of the mass spectrometer transmission line of 260 °C. The temperature of the valve box was 180 °C, the switching time of the six-way valve was 7.5 minutes, the initial temperature of the column box was 160 °C and maintained for 7.5 minutes, then the temperature was raised to 206 °C at a rate of 40 °C/min and maintained for 2 minutes, and the sample amount was 0.2 $\mu$L.

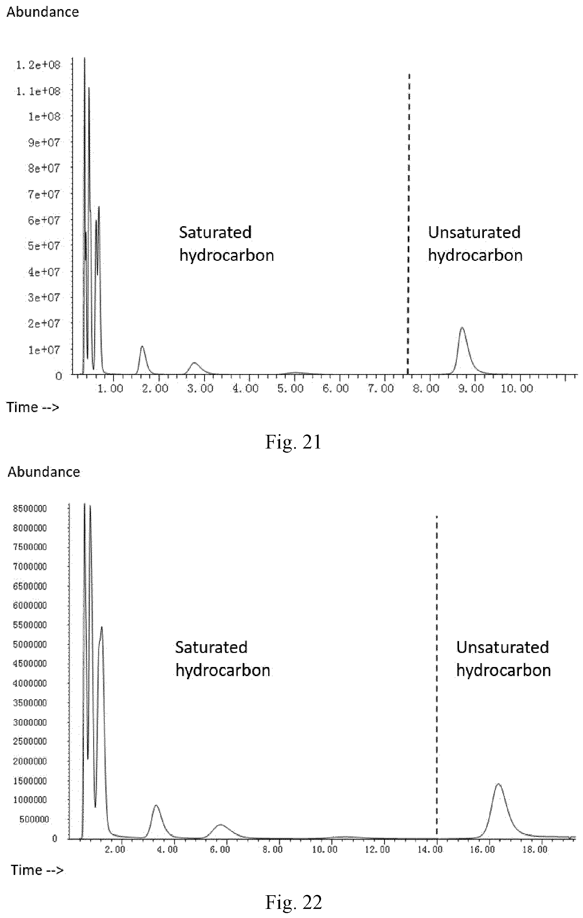

The formulated sample III-B was used as a test object, and a detailed hydrocarbon composition analysis of the sample III-B was conducted using the method of the present application under the conditions as described above. The chromatogram obtained is shown in Fig. 21, in which the species eluted at a time before 7.5 minutes are saturated hydrocarbon components, and the species eluted at a time after 7.5 minutes are unsaturated hydrocarbon components.

The results of the total amount of unsaturated and saturated hydrocarbons of the formulated sample are shown in Table III-4, and the results of the detailed hydrocarbon composition analysis are shown in Table III-5.

Table III-4 Total amount of saturated and unsaturated hydrocarbons of sample III-B (FID data)

| Fractions | Measured content (m%) | Formulation content (m%) |
|---|---|---|
| Saturated hydrocarbon | 79.99 | 80.93 |
| Unsaturated hydrocarbon | 20.01 | 19.07 |

Table III-5 Detailed hydrocarbon composition data of sample III-B (mass spectrometry data)

| Fractions | Measured content (m%) | Formulation content (m%) |
|---|---|---|
| Paraffins | 41.9 | 42.7 |
| Noncondensed cycloparaffins | 26.1 | 26.43 |
| Condensed dicycloparaffins | 11.6 | 11.8 |
| Condensed Tricycloparaffins | 0 | 0 |
| Olefins | 0 | 0 |
| Alkylbenzenes | 11.5 | 9.53 |
| Indanes or tetralins, or both | 5.4 | 6.53 |
| Indenes | 0 | 0 |
| Naphthalene | 0.1 | 0 |
| Naphthalenes | 3.4 | 3.01 |
| Acenaphthenes | 0 | 0 |
| Acenaphthylenes | 0 | 0 |
| Tricyclic aromatics | 0 | 0 |

Example III-3

The device shown in Fig. 2 and the enrichment material described in Example III-1 were used, the embedded packed column had an inner diameter of 4 mm and a length of 100 mm, the quartz capillary column 2 had an inner diameter of 0.25 mm and a length of 5 m, the quartz capillary column 5 had an inner diameter of 0.32 mm and a length of 5 m, and the quartz capillary column 7 and the quartz capillary column 8 were configured at a split ratio of 10 : 1.

The experimental conditions employed in the method include: a temperature of the dividing/undividing sample inlet of 280 °C, a split ratio of 20 : 1, a carrier gas of helium, a flow rate of 10 mL/min, a temperature of the flame ionization detector of 260 °C, and a temperature of the mass spectrometer transmission line of 260 °C. The temperature of the valve box was 180 °C, the six-way valve 11 was opened at 0.02 minutes so that the packed column was connected into the gas circuit; the six-way valve 3 was opened at 14 minutes so that a back flushing of the gas circuit was realized in the method, the initial temperature of the column box was 145 °C and maintained for 14 minutes, then the temperature was raised to 206 °C at a rate of 40 °C/min and maintained for 3 min; the six-way valve 3 was closed at 19 minutes so that the gas flow was restored to the positive direction, the six-way valve 11 was closed at 19.1 minutes so that the packed column was isolated from the gas circuit of the system; and the sample amount was 0.2 µL.

The formulated sample III-C was used as a test object, and a detailed hydrocarbon composition analysis of the sample III-C was conducted using the method of the present application under the conditions as described above. The chromatogram obtained is shown in Fig. 22, in which the species eluted at a time before 14 minutes are saturated hydrocarbon components, and the species eluted at a time after 14 minutes are unsaturated hydrocarbon components.

The results of the total amount of unsaturated and saturated hydrocarbons of the formulated sample are shown in Table III-6, and the results of the detailed hydrocarbon composition analysis are shown in Table III-7.

Table III-6 Total amount of saturated and unsaturated hydrocarbons of sample III-C (FID data)

| Fractions | Measured content (m%) | Formulation content (m%) |
|---|---|---|
| Saturated hydrocarbon | 73.08 | 73.55 |
| Unsaturated hydrocarbon | 26.92 | 26.45 |

Table III-7 Detailed hydrocarbon composition data of sample III-C (mass spectrometry data)

| Fractions | Measured content (m%) | Formulation content (m%) |
|---|---|---|
| Paraffins | 37.5 | 38.79 |
| Noncondensed cycloparaffins | 25.0 | 24.02 |

(continued)

| Fractions | Measured content (m%) | Formulation content (m%) |
|---|---|---|
| Condensed dicycloparaffins | 10.2 | 10.72 |
| Condensed Tricycloparaffins | 0 | 0 |
| Olefins | 0 | 0 |
| Alkylbenzenes | 10.3 | 8.68 |
| Indanes or tetralins, or both | 4.6 | 5.93 |
| Indenes | 0 | 0 |
| Naphthalene | 0 | 0 |
| Naphthalenes | 12.4 | 11.86 |
| Acenaphthenes | 0 | |
| Acenaphthylenes | 0 | |
| Tricyclic aromatics | 0 | |

Example III-4

An experiment was conducted using the device and enrichment material described in Example III-3, with a difference that the quartz capillary 2 had an inner diameter of 0.32 mm and a length of 5 m, the quartz capillary column 5 had an inner diameter of 0.53 mm and a length of 0.2 m, and the quartz capillary column 7 and the quartz capillary column 8 were configured at a split ratio of 10 : 1.

The experimental conditions employed in the method include: a temperature of the dividing/undividing sample inlet of 280 °C, a split ratio of 20:1, a carrier gas of helium, a flow rate of 10 mL/min, a temperature of the flame ionization detector of 260 °C, and a temperature of the mass spectrometer transmission line of 260 °C. The temperature of the valve box was 180 °C, the six-way valve 11 was opened at 0.02 minutes so that the packed column was connected into the gas circuit; the six-way valve 3 was opened at 14 minutes so that a back flushing of the gas circuit flow was realized in the method, the initial temperature of the column box was 155 °C and maintained for 14 minutes, then the temperature was raised to 206 °C at a rate of 40 °C/min and maintained for 3 min; the six-way valve 3 was closed at 19 minutes so that the gas flow was restored to the positive direction, the six-way valve 11 was closed at 19.1 minutes so that the packed column was isolated from the gas circuit of the system; and the sample amount was 0.2 μL.

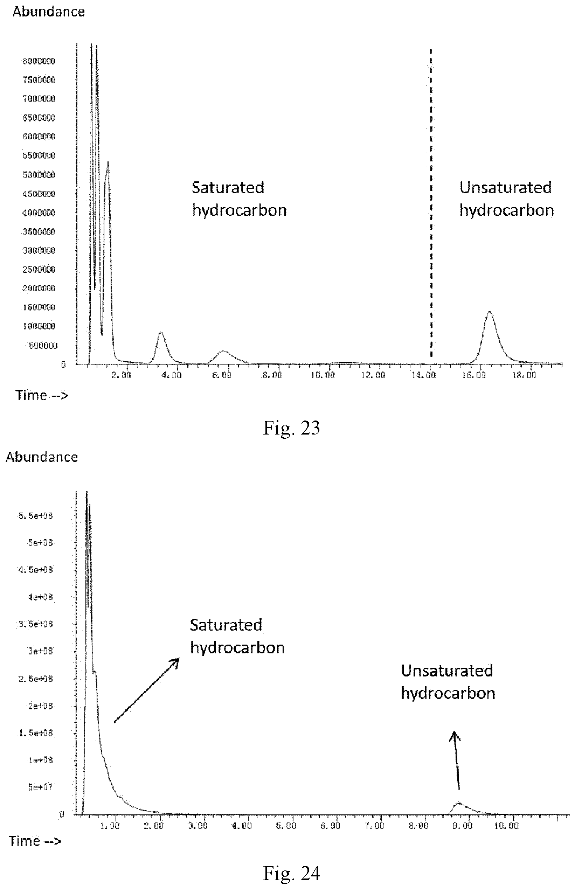

The formulated sample III-D was used as a test object, and a detailed hydrocarbon composition analysis of the sample III-D was conducted using the method of the present application under the conditions as described above. The chromatogram obtained is shown in Fig. 23, in which the species eluted at a time before 14 minutes are saturated hydrocarbon components, and the species eluted at a time after 14 minutes are unsaturated hydrocarbon components.

The results of the total amount of unsaturated and saturated hydrocarbons of the formulated sample are shown in Table III-8, and the results of the detailed hydrocarbon composition analysis are shown in Table III-9.

Table III-8 Total amount of saturated and unsaturated hydrocarbons of sample III-D (FID data)

| Fractions | Measured content (m%) | Formulation content (m%) |
|---|---|---|
| Saturated hydrocarbon | 69.79 | 69.62 |
| Unsaturated hydrocarbon | 30.21 | 30.38 |

Table III-9 Detailed hydrocarbon composition data of sample III-D (mass spectrometry data)

| Fractions | Measured content (m%) | Formulation content (m%) |
|---|---|---|
| Paraffins | 32.8 | 36.74 |
| Noncondensed cycloparaffins | 32.6 | 22.74 |
| Condensed dicycloparaffins | 4 | 10.15 |

(continued)

| Fractions | Measured content (m%) | Formulation content (m%) |
|---|---|---|
| Condensed Tricycloparaffins | 0 | 0 |
| Olefins | 0 | 0 |
| Alkylbenzenes | 9.8 | 8.2 |
| Indanes or tetralins, or both | 4.3 | 5.62 |
| Indenes | 0 | 0 |
| Naphthalene | 0 | 0 |
| Naphthalenes | 16.6 | 16.56 |
| Acenaphthenes | 0 | 0 |
| Acenaphthylenes | 0 | 0 |
| Tricyclic aromatics | 0 | 0 |

Example III-5

An experiment was conducted using the method described in Example III-2, with a difference that a real sample III-E was used as a test object, and a detailed hydrocarbon composition analysis of the real sample III-E was conducted using the method of the present application under the conditions as described. The chromatogram obtained is shown in Fig. 24, in which the species eluted at a time before 7 minutes are saturated hydrocarbon components, and the species eluted at a time after 7 minutes are unsaturated hydrocarbon components.

The results of the total amount of unsaturated and saturated hydrocarbons of the real sample III-E obtained are shown in Table III-10, and the results of the detailed hydrocarbon composition analysis are shown in Table III-11, in which the reference data are data obtained using the method described in the reference example.

Table III-10 Total amount of saturated and unsaturated hydrocarbons of sample III-E

| Fractions | Measured content (m%) | Reference data (m%) |
|---|---|---|
| Saturated hydrocarbon | 93.98 | 94.27 |
| Unsaturated hydrocarbon | 6.02 | 5.73 |

Table III-11 Detailed hydrocarbon composition data of sample III-E

| Fractions | Measured content (m%) | Reference data (m%) |
|---|---|---|
| Paraffins | 33.6 | 32.7 |
| Noncondensed cycloparaffins | 36.3 | 36.8 |
| Condensed dicycloparaffins | 20.5 | 21.2 |
| Condensed Tricycloparaffins | 3.2 | 2.9 |
| Olefins | 0 | - |
| Alkylbenzenes | 4.2 | 3.8 |
| Indanes or tetralins, or both | 2.0 | 2.3 |
| Indenes | 0 | 0 |
| Naphthalene | 0 | 0.1 |
| Naphthalenes | 0.2 | 0.3 |
| Acenaphthenes | 0 | 0 |
| Acenaphthylenes | 0 | 0 |

(continued)

| Fractions | Measured content (m%) | Reference data (m%) |
|---|---|---|
| Tricyclic aromatics | 0 | 0 |

Example III-6

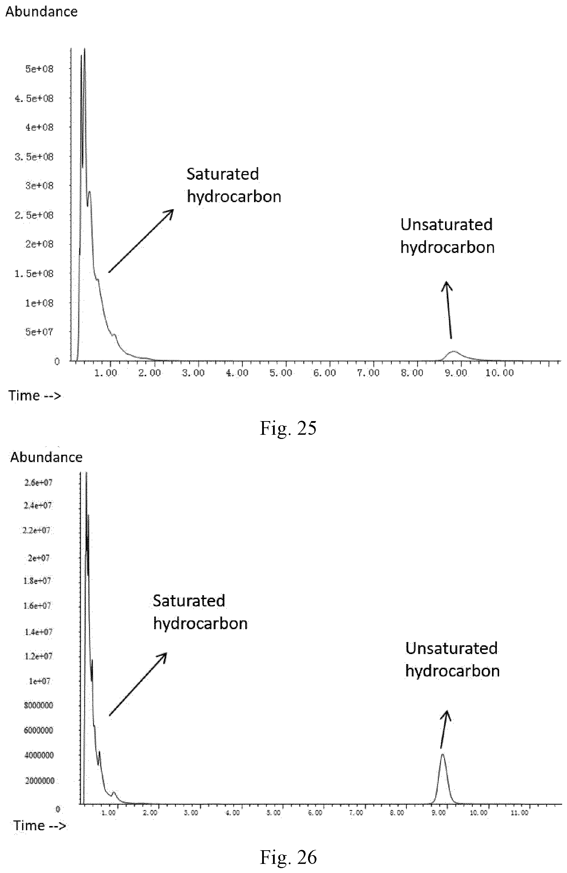

An experiment was conducted using the method described in Example III-2, with a difference that a real sample III-F was used as a test object, and a detailed hydrocarbon composition analysis of the real sample III-F was conducted using the method of the present application under the conditions as described. The chromatogram obtained is shown in Fig. 25, in which the species eluted at a time before 7 minutes are saturated hydrocarbon components, and the species eluted at a time after 7 minutes are unsaturated hydrocarbon components.

The results of the total amount of unsaturated and saturated hydrocarbons of the real sample III-F obtained are shown in Table 111-12, and the results of the hydrocarbon composition analysis are shown in Table 111-13, in which the reference data are data obtained using the method described in the reference example.

Table III-12 Total amount of saturated and unsaturated hydrocarbons of sample III-F

| Fractions | Measured content (m%) | Reference data (m%) |
|---|---|---|
| Saturated hydrocarbon | 95.26 | 96.17 |
| Unsaturated hydrocarbon | 4.74 | 3.83 |

Table III-13 Detailed hydrocarbon composition data of sample III-F

| Fractions | Measured content (m%) | Reference data (m%) |
|---|---|---|
| Paraffins | 32.2 | 32.9 |
| Noncondensed cycloparaffins | 36.1 | 37.3 |
| Condensed dicycloparaffins | 24.1 | 25.0 |
| Condensed Tricycloparaffins | 3.1 | 2.7 |
| Olefins | 0.2 | - |
| Alkylbenzenes | 3.3 | 2.1 |
| Indanes or tetralins, or both | 0.8 | 0 |
| Indenes | 0 | 0 |
| Naphthalene | 0 | 0 |
| Naphthalenes | 0.1 | 0 |
| Acenaphthenes | 0 | 0 |
| Acenaphthylenes | 0.1 | 0 |
| Tricyclic aromatics | 0 | 0 |

Example III-7

An experiment was conducted using the method described in Example III-2, with a difference that a real sample III-G was used as a test object, and a detailed hydrocarbon composition analysis of the real sample III-G was conducted using the method of the present application under the conditions as described. The chromatogram obtained is shown in Fig. 26, in which the species eluted at a time before 7 minutes are saturated hydrocarbon components, and the species eluted at a time after 7 minutes are unsaturated hydrocarbon components.

The results of the total amount of unsaturated and saturated hydrocarbons of the real sample III-G obtained are shown in Table 111-14, and the results of the detailed hydrocarbon composition analysis are shown in Table 111-15, in which the reference data are data obtained using the method described in the reference example.

Table III-14 Total amount of saturated and unsaturated hydrocarbons of sample III-G

| Fractions | Measured content (m%) | Reference data (m%) |
|---|---|---|
| Saturated hydrocarbon | 82.52 | 83.79 |
| Unsaturated hydrocarbon | 17.48 | 16.21 |

Table III-15 Detailed hydrocarbon composition data of sample III-G

| Fractions | Measured content (m%) | Reference data (m%) |
|---|---|---|
| Paraffins | 49.0 | 49.7 |
| Noncondensed cycloparaffins | 22.8 | 23.9 |
| Condensed dicycloparaffins | 9.2 | 10.7 |
| Condensed Tricycloparaffins | 1.0 | 0.8 |
| Olefins | 1.2 | - |
| Alkylbenzenes | 11.8 | 11.6 |
| Indanes or tetralins, or both | 2.8 | 2.4 |
| Indenes | 0.2 | 0.1 |
| Naphthalene | 0.4 | 0.1 |
| Naphthalenes | 1.5 | 0.7 |
| Acenaphthenes | 0.1 | 0 |
| Acenaphthylenes | 0 | 0 |
| Tricyclic aromatics | 0 | 0 |

Example III-8

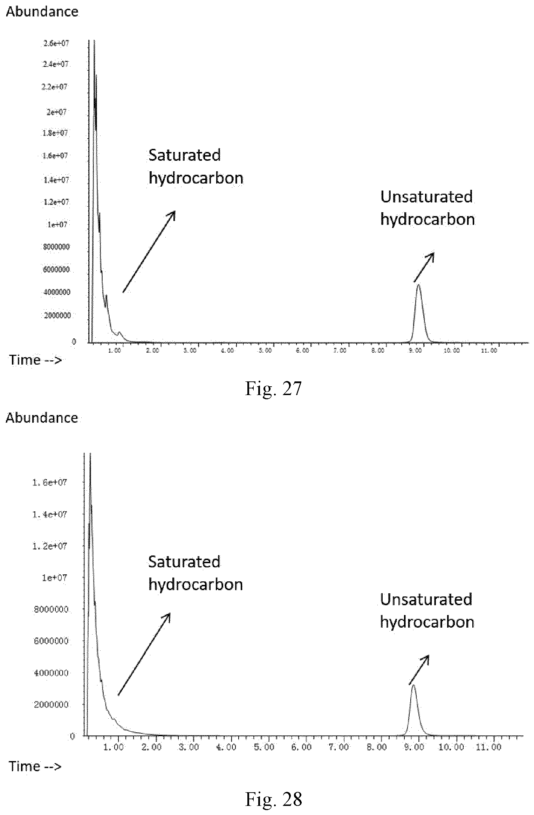

An experiment was conducted using the method described in Example III-2, with a difference that a real sample III-H was used as a test object, and a detailed hydrocarbon composition analysis of the real sample III-H was conducted using the method of the present application under the conditions as described. The chromatogram obtained is shown in Fig. 27, in which the species eluted at a time before 7 minutes are saturated hydrocarbon components, and the species eluted at a time after 7 minutes are unsaturated hydrocarbon components.

The results of the total amount of unsaturated and saturated hydrocarbons of the real sample III-H obtained are shown in Table 111-16, and the results of the detailed hydrocarbon composition analysis are shown in Table 111-17, in which the reference data are data obtained using the method described in the reference example.

Table III-16 Total amount of saturated and unsaturated hydrocarbons of sample III-H

| Fractions | Measured content (m%) | Reference data (m%) |
|---|---|---|
| Saturated hydrocarbon | 78.49 | 83.59 |
| Unsaturated hydrocarbon | 21.51 | 16.41 |

Table III-17 Detailed hydrocarbon composition data of sample III-H

| Fractions | Measured content (m%) | Reference data (m%) |
|---|---|---|
| Paraffins | 45.2 | 45.6 |
| Noncondensed cycloparaffins | 23.7 | 25.2 |
| Condensed dicycloparaffins | 7.7 | 8.6 |

(continued)

| Fractions | Measured content (m%) | Reference data (m%) |
|---|---|---|
| Condensed Tricycloparaffins | 1.1 | 0.9 |
| Olefins | 3.2 | - |
| Alkylbenzenes | 13.4 | 13.7 |
| Indanes or tetralins, or both | 3.3 | 3.5 |
| Indenes | 0.3 | 0.2 |
| Naphthalene | 0.2 | 0.5 |
| Naphthalenes | 1.9 | 1.7 |
| Acenaphthenes | 0 | 0.1 |
| Acenaphthylenes | 0 | 0 |
| Tricyclic aromatics | 0 | 0 |

Example III-9

An experiment was conducted using the method described in Example III-2, with a difference that a real sample III-I was used as a test object, and a detailed hydrocarbon composition analysis of the real sample III-I was conducted using the method of the present application under the conditions as described. The chromatogram obtained is shown in Fig. 28, in which the species eluted at a time before 7 minutes are saturated hydrocarbon components, and the species eluted at a time after 7 minutes are unsaturated hydrocarbon components.

The results of the total amount of unsaturated and saturated hydrocarbons of the real sample III-I obtained are shown in Table III-18, and the results of the detailed hydrocarbon composition analysis are shown in Table 111-19, in which the reference data are data obtained using the method described in the reference example.

Table III-18 Total amount of saturated and unsaturated hydrocarbons of sample III-I

| Fractions | Measured content (m%) | Reference data (m%) |
|---|---|---|
| Saturated hydrocarbon | 83.69 | 84.98 |
| Unsaturated hydrocarbon | 16.31 | 15.02 |

Table III-19 Detailed hydrocarbon composition data of sample III-I

| Fractions | Measured content (m%) | Reference data (m%) |
|---|---|---|
| Paraffins | 44.3 | 44.4 |
| Noncondensed cycloparaffins | 25.6 | 27.2 |
| Condensed dicycloparaffins | 10.9 | 11.8 |
| Condensed Tricycloparaffins | 1.4 | 1.1 |
| Olefins | 1.3 | - |
| Alkylbenzenes | 8.1 | 7.7 |
| Indanes or tetralins, or both | 7.3 | 6.9 |
| Indenes | 0.3 | 0.3 |
| Naphthalene | 0.2 | 0.1 |
| Naphthalenes | 0.6 | 0.5 |
| Acenaphthenes | 0 | 0 |
| Acenaphthylenes | 0 | 0 |

(continued)

| Fractions | Measured content (m%) | Reference data (m%) |
|---|---|---|
| Tricyclic aromatics | 0 | 0 |

Example III-10

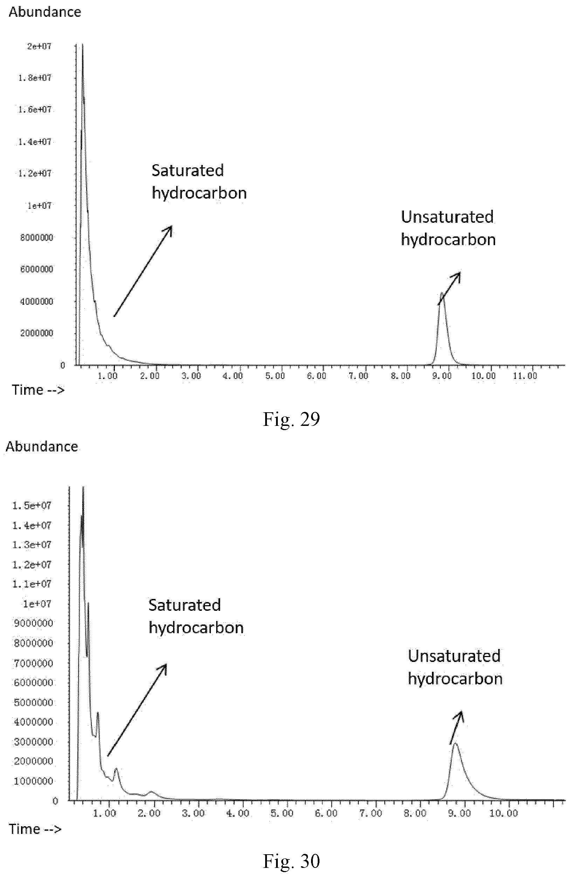

An experiment was conducted using the method described in Example III-2, with a difference that a real sample III-J were used as a test object, and a detailed hydrocarbon composition analysis of the real sample III-J was conducted using the method of the present application under the conditions as described. The chromatogram obtained is shown in Fig. 29, in which the species eluted at a time before 7 minutes are saturated hydrocarbon components, and the species eluted at a time after 7 minutes are unsaturated hydrocarbon components.

The results of the total amount of unsaturated and saturated hydrocarbons of the real sample III-J obtained are shown in Table 111-20, and the results of the detailed hydrocarbon composition analysis are shown in Table 111-21, in which the reference data are data obtained using the method described in the reference example.

Table 111-20 Total amount of saturated and unsaturated hydrocarbons of sample III-J

| Fractions | Measured content (m%) | Reference data (m%) |
|---|---|---|
| Saturated hydrocarbon | 78.46 | 81.8 |
| Unsaturated hydrocarbon | 21.54 | 18.2 |

Table 111-21 Detailed hydrocarbon composition data of sample III-J

| Fractions | Measured content (m%) | Reference data (m%) |
|---|---|---|
| Paraffins | 42.4 | 42.8 |
| Noncondensed cycloparaffins | 24.3 | 26.2 |
| Condensed dicycloparaffins | 10.5 | 11.4 |
| Condensed Tricycloparaffins | 0.9 | 1.4 |
| Olefins | 3.7 | - |
| Alkylbenzenes | 8.9 | 9.1 |
| Indanes or tetralins, or both | 8.4 | 8.0 |
| Indenes | 0.2 | 0.3 |
| Naphthalene | 0.2 | 0.2 |
| Naphthalenes | 0.5 | 0.6 |
| Acenaphthenes | 0 | 0 |
| Acenaphthylenes | 0 | 0 |
| Tricyclic aromatics | 0 | 0 |

Example III-11

An experiment was conducted using the method described in Example III-2, with a difference that a real sample III-K was used as a test object, and a detailed hydrocarbon composition analysis of the real sample III-K was conducted using the method of the present application under the conditions as described. The chromatogram obtained is shown in Fig. 30, in which the species eluted at a time before 7 minutes are saturated hydrocarbon components, and the species eluted at a time after 7 minutes are unsaturated hydrocarbon components.

The results of the total amount of unsaturated and saturated hydrocarbons of the real sample III-K obtained are shown in Table 111-22, and the results of the detailed hydrocarbon composition analysis are shown in Table 111-23, in which the reference data are data obtained using the method described in the reference example.

Table III-22 Total amount of saturated and unsaturated hydrocarbons of sample III-K

| Fractions | Measured content (m%) | Reference data (m%) |
|---|---|---|
| Saturated hydrocarbon | 79.67 | 81.34 |
| Unsaturated hydrocarbon | 20.33 | 18.66 |

Table III-23 Detailed hydrocarbon composition data of sample III-K

| Fractions | Measured content (m%) | Reference data (m%) |
|---|---|---|
| Paraffins | 50.2 | 50.5 |
| Noncondensed cycloparaffins | 22.1 | 24.2 |
| Condensed dicycloparaffins | 5.5 | 6.4 |
| Condensed Tricycloparaffins | 0.3 | 0.3 |
| Olefins | 1.4 | - |
| Alkylbenzenes | 15.5 | 14.7 |
| Indanes or tetralins, or both | 3.6 | 2.9 |
| Indenes | 0.1 | 0 |
| Naphthalene | 0.1 | 0.2 |
| Naphthalenes | 1.2 | 0.8 |
| Acenaphthenes | 0 | 0 |
| Acenaphthylenes | 0 | 0 |
| Tricyclic aromatics | 0 | 0 |

Example III-12

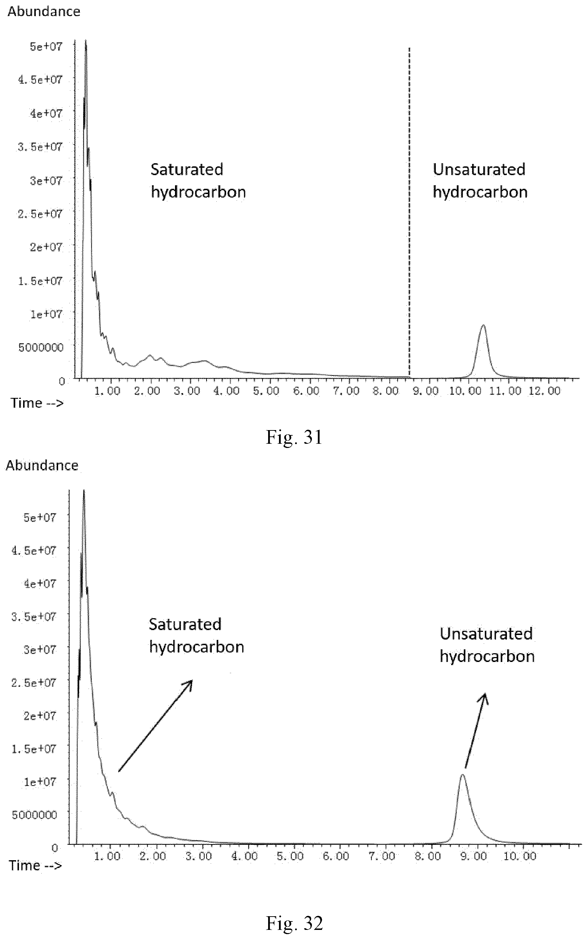

An experiment was conducted using the method described in Example III-1, with a difference that a biomass aviation fuel sample III-L was used as a test object, the switching time of the six-way valve was 8.5 minutes, and at the moment of 8.5 minutes the temperature of the column box began to raise and was finally raised to 206 °C and maintained for 3 minutes. A detailed hydrocarbon composition analysis of the real sample III-L was conducted using the method of the present application under the conditions as described. The chromatogram obtained is shown in Fig. 31, in which the species eluted at a time before 8.5 minutes are saturated hydrocarbon components, and the species eluted at a time after 8.5 minutes are unsaturated hydrocarbon components.

The results of the total amount of unsaturated and saturated hydrocarbons of the real sample III-L obtained are shown in Table 111-24, and the results of the detailed hydrocarbon composition analysis are shown in Table 111-25, in which the reference data are data obtained using the method described in the reference example.

Table 111-24 Total amount of saturated and unsaturated hydrocarbons of sample III-L

| Fractions | Measured content (m%) | Reference data (m%) |
|---|---|---|
| Saturated hydrocarbon | 87.91 | 88.33 |
| Unsaturated hydrocarbon | 12.09 | 11.67 |

Table 111-25 Detailed hydrocarbon composition data of sample III-L

| Fractions | Measured content (m%) | Reference data (m%) |
|---|---|---|
| Paraffins | 74.3 | 74.1 |
| Noncondensed cycloparaffins | 12.6 | 13.0 |

(continued)

| Fractions | Measured content (m%) | Reference data (m%) |
|---|---|---|
| Condensed dicycloparaffins | 3.5 | 3.2 |
| Condensed Tricycloparaffins | 0.5 | 0.8 |
| Olefins | 0 | - |
| Alkylbenzenes | 6.5 | 6.7 |
| Indanes or tetralins, or both | 1.8 | 1.4 |
| Indenes | 0.1 | 0.1 |
| Naphthalene | 0.1 | 0.3 |
| Naphthalenes | 0.5 | 0.4 |
| Acenaphthenes | 0.1 | 0 |
| Acenaphthylenes | 0 | 0 |
| Tricyclic aromatics | 0 | 0 |

Example III-13

An experiment was conducted using the method described in Example III-2, with a difference that a real sample III-M was used as a test object, and a detailed hydrocarbon composition analysis of the real sample III-M was conducted using the method of the present application under the conditions as described. The chromatogram obtained is shown in Fig. 32, in which the species eluted at a time before 7 minutes are saturated hydrocarbon components, and the species eluted at a time after 7 minutes are unsaturated hydrocarbon components.

The results of the total amount of unsaturated and saturated hydrocarbons of the real sample III-M obtained are shown in Table 111-26, and the results of the detailed hydrocarbon composition analysis are shown in Table 111-27, in which the reference data are data obtained using the method described in the reference example.

Table III-26 Total amount of saturated and unsaturated hydrocarbons of sample III-M

| Fractions | Measured content (m%) | Reference data (m%) |
|---|---|---|
| Saturated hydrocarbon | 82.55 | 84.17 |
| Unsaturated hydrocarbon | 17.45 | 15.83 |

Table 111-27 Detailed hydrocarbon composition data of sample III-M

| Fractions | Measured content (m%) | Reference data (m%) |
|---|---|---|
| Paraffins | 44.8 | 45.1 |
| Noncondensed cycloparaffins | 25.8 | 26.9 |
| Condensed dicycloparaffins | 10.4 | 10.7 |
| Condensed Tricycloparaffins | 1.4 | 1.3 |
| Olefins | 0.3 | - |
| Alkylbenzenes | 9.5 | 9.2 |
| Indanes or tetralins, or both | 6.5 | 6.0 |
| Indenes | 0.5 | 0.6 |
| Naphthalene | 0.1 | 0 |
| Naphthalenes | 0.5 | 0.2 |
| Acenaphthenes | 0 | 0 |

(continued)

| Fractions | Measured content (m%) | Reference data (m%) |
|---|---|---|
| Acenaphthylenes | 0 | 0 |
| Tricyclic aromatics | 0.1 | 0 |

**Reference Example**

The real sample III-E of Example III-5 was used as a representative example, and a detailed hydrocarbon composition analysis of the sample was conducted by using a method comprising sample pre-separation in combination with gas chromatography-mass spectrometry (according to SH/T0606-2005).

5g of silica gel (with a specific surface area of 300 m$^2$/g, a pore volume of 0.41 mL/g, and a particle size of 75-150 $\mu$m) was baked at 150 °C for 3 hours, and 5g of $\gamma$-alumina (with a specific surface area of 240 m$^2$/g, a pore volume of 0.62 mL/g, and a particle size of 75-150 $\mu$m) was calcined at 500 °C for 5 hours. The silica gel and the alumina was mixed at a ratio of 95 : 5 to obtain an adsorbent for the pre-separation of the sample, which was filled into a solid phase extraction column with an inner diameter of 1 cm and a length of 7 cm, and the column was plugged into a sieve plate and was wetted by 1 mL of n-pentane.

100 $\mu$L of the sample III-E was taken, placed on the sieve plate holding the solid phase extraction column, eluted with 2 mL of n-pentane, then eluted with 0.5 mL of a mixed solvent of dichloromethane and ethanol (with a volume ratio of 5 : 1), the effluent obtained being a mixture of saturated hydrocarbon and olefin (hereinafter referred to as saturated hydrocarbon component); and further eluted with 2.5 mL of a mixed solvent of dichloromethane and ethanol (with a volume ratio of 5 : 1), the effluent obtained being an unsaturated hydrocarbon component; 1 mL of n-triacontane was added using pipettes to the saturated hydrocarbon component and the unsaturated hydrocarbon component, respectively, as an internal standard substance to measure the total amount of the saturated hydrocarbon and the unsaturated hydrocarbon.

The saturated hydrocarbon component and the unsaturated hydrocarbon component obtained by the pre-separation were analyzed using a gas chromatograph-mass spectrometer, respectively, with the analyzing time of each component being 13 minutes. After the spectrums of the saturated hydrocarbon component and the unsaturated hydrocarbon component were collected respectively, and a data analysis was carried out. The whole analysis process took over 30 minutes, and since the olefin component and the saturated hydrocarbon component flowed out together, there was an interference between the analysis of the olefin component and the analysis of the naphthene component, and as a result the olefin component was grouped into the naphthene component. The data of the total amount of the saturated and unsaturated hydrocarbon components of the real sample III-E are shown in Table III-10, and the results of the detailed hydrocarbon composition analysis are shown in Table III-11.

The present application is illustrated in detail hereinabove with reference to preferred embodiments, but is not intended to be limited to those embodiments. Various modifications may be made following the inventive concept of the present application, and these modifications shall be within the scope of the present application.

It should be noted that the various technical features described in the above embodiments may be combined in any suitable manner without contradiction, and in order to avoid unnecessary repetition, various possible combinations are not described in the present application, but such combinations shall also be within the scope of the present application.

In addition, the various embodiments of the present application can be arbitrarily combined as long as the combination does not depart from the spirit of the present application, and such combined embodiments should be considered as the disclosure of the present application.

**Claims**

1. A reversible enrichment material, comprising:

   an inorganic carrier; and
   an active metal salt, a first metal salt promoter and a second metal salt promoter supported on the inorganic carrier;
   wherein the active metal salt is selected from the group consisting of soluble silver salts, soluble copper salts, or combinations thereof, the first metal salt promoter is one or more selected from the group consisting of soluble salts of Group IA, Group IIA, and Group IIIA metals, and the second metal salt promoter is one or more selected from the group consisting of soluble salts of transition metals other than Group IB metals,

in the reversible enrichment material, based on the total amount of the inorganic carrier, the active metal salt is present in an amount of about 0.1-80% by mass, preferably about 0.5-50% by mass; the first metal salt promoter is present in an amount of about 0.1-80% by mass, preferably about 0.2-40% by mass; the second metal salt promoter is present in an amount of about 0.5-80% by mass, preferably about 0.5-30% by mass; preferably, the ratio of the mass content of the first metal salt promoter to the mass content of the second metal salt promoter, calculated as metal oxide, is about 1 (0.05-50).

2.  The reversible enrichment material according to claim 1, wherein:

the soluble silver salt is silver nitrate and the soluble copper salt is selected from the group consisting of copper nitrate, copper sulfate or a combination thereof;
in the first metal salt promoter, the Group IA metal is one or more selected from the group consisting of lithium, sodium and potassium, the Group IIA metal is one or more selected from the group consisting of beryllium, magnesium, calcium and barium, and the Group IIIA metal is selected from the group consisting of aluminum, gallium or a combination thereof; and/or
in the second metal salt promoter, the transition metal is one or more selected from the group consisting of zinc, cadmium, vanadium, chromium, molybdenum, tungsten, manganese, iron, cobalt, nickel, ruthenium, platinum, rhodium and palladium.

3.  The reversible enrichment material according to any one of the preceding claims, wherein the second metal salt promoter comprises two soluble transition metal salts, preferably a first soluble transition metal salt selected from soluble salts of Group VIII transition metals, and a second soluble transition metal salt selected from soluble salts of Group IIB and Group VIB transition metals, preferably from soluble salts of Group IIB transition metals; preferably, the ratio of the mass content of the first soluble transition metal salt to the mass content of the second soluble transition metal salt, calculated as metal oxide, is about 1 : (0.05-40).

4.  The reversible enrichment material according to any one of the preceding claims, wherein the inorganic carrier is one or more selected from the group consisting of diatomite carrier, alumina carrier, titania carrier, zirconia carrier, mesoporous molecular sieve carrier, amorphous silica-alumina carrier, silica gel carrier, and controlled porous glass carrier;

preferably, the inorganic carrier has been subjected to a modification comprising sequentially a calcination treatment, an acid-washing and/or alkali-washing treatment and a drying treatment; and
further preferably, the inorganic carrier has a specific surface area of about 1-600 $m^2/g$, a pore size in a range of about 1-1000 nm, and a particle size in a range of about 80-800 $\mu$m.

5.  The reversible enrichment material according to claim 4, wherein:

the conditions of the calcination treatment include: a temperature of about 500-950 °C and a period of about 4-16 hours;
the conditions of the acid-washing treatment include: a temperature of about 100-200 °C, a period of about 10-40 hours, and a mass ratio of the starting material to be treated on a dry basis to the acid solution of about 1 : (1-20), wherein the acid solution used for the acid-washing treatment is one or more selected from the group consisting of nitric acid, hydrochloric acid and sulfuric acid, and the concentration of the acid solution used for the acid-washing treatment is about 1-90% by mass;
the conditions of the alkali-washing treatment include: a temperature of about 100-200 °C, a period of about 10-40 hours, and a mass ratio of the starting material to be treated on a dry basis to the alkali solution of about 1 : (1-20), wherein the alkali solution used for the alkali-washing treatment is one or more selected from the group consisting of sodium hydroxide solution, potassium hydroxide solution and ammonia water, and the concentration of the alkali solution used for the alkali-washing treatment is about 1-90% by mass; and/or
the conditions of the drying treatment include: a temperature of about 100-200 °C and a period of about 3-6 hours.

6.  A method for preparing the reversible enrichment material according to any one of the preceding claims, comprising the steps of:

1) providing an inorganic carrier;
2) loading the active metal salt, the first metal salt promoter and the second metal salt promoter onto the inorganic carrier; and

3) optionally, drying the material obtained in step 2),

preferably, the step 1) further comprises subjecting an inorganic starting material to a modification to obtain the inorganic carrier, wherein the modification comprises sequentially a calcination treatment, an acid-washing and/or alkali-washing treatment, and a drying treatment.

7. A packed column comprising a column tube and an enrichment filler filled in the column tube, wherein the enrichment filler is the reversible enrichment material according to any one of claims 1-5.

8. The packed column according to claim 7, wherein:

the column tube is a stainless steel column tube, a glass column tube or a quartz column tube;
the column tube is U-shaped or spiral-shaped; and/or
the column tube has an inner diameter of about 0.1-12 mm, preferably about 1-6 mm, and a length of about 20-5000 mm, preferably about 50-1000 mm.

9. The packed column according to claim 7 or 8, wherein the inner surface of the column tube has been subjected to a passivation treatment comprising an alkali treatment and an acid treatment,
preferably, the passivation treatment comprises: soaking the column tube in an alkali solution having a concentration of about 5-20% by mass at about 20-100 °C for about 30-120 minutes, washing with water to neutrality, immersing in an acid solution having a concentration of about 5-20% by mass at about 30-120 °C for about 30-100 minutes, washing with water to neutrality, and then drying.

10. A separation and analysis system, comprising the packed column according to any one of claims 7-9, preferably the system further comprises a gas chromatograph or a gas chromatograph-mass spectrometer in communication with the packed column.

11. The system according to claim 10, further comprising a gas chromatograph-mass spectrometer, wherein the gas chromatograph-mass spectrometer comprises a sample inlet and an analysis module, and the two ends of the packed column are respectively communicated with the sample inlet and an inlet of the analysis module;

the analysis module comprises a mass spectrometry unit and a gas chromatography unit, and inlets of the mass spectrometry unit and the gas chromatography unit are respectively communicated with the inlet of the analysis module through a splitter;
preferably, a first capillary column is connected between the sample inlet and the packed column, a second capillary column is connected between the packed column and the inlet of the analysis module, and/or
preferably, the system further comprises a multi-port valve through which the packed column is switchably connected into the gas chromatograph-mass spectrometer.

12. The system according to claim 11, wherein the multi-port valve has a plurality of working ports and has a first working position and a second working position;

in the first working position, the first end of the packed column is communicated with the sample inlet through at least one working port, and the second end of the packed column is communicated with the inlet of the analysis module through at least one working port;
in the second working position, the second end of the packed column is communicated with the sample inlet through at least one working port, and the first end of the packed column is communicated with the inlet of the analysis module through at least one working port;
preferably, the multi-port valve is a six-way valve having 6 working ports.

13. The system according to claim 11, further comprising a multi-port valve set having a first multi-port valve and a second multi-port valve, the first multi-port valve and the second multi-port valve are communicated with each other in a switchable manner, the packed column is connected into the gas chromatograph-mass spectrometer through the second multi-port valve;

the multi-port valve set has a switch-in working position for connecting the packed column into the system and a switch-out working position for removing the packed column out from the system;
in the switch-in working position, the sample inlet, the first multi-port valve, the packed column connected through the second multi-port valve and the inlet of the analysis module are communicated sequentially;
in the switch-out working position, the packed column is not connected into the system, and the sample inlet, the first multi-port valve, the second multi-port valve and the inlet of the analysis module are communicated sequentially.

14. A method for separating and analyzing hydrocarbon components of a hydrocarbon-based material comprising saturated hydrocarbon and unsaturated hydrocarbon, comprising the steps of:

S1) feeding the hydrocarbon-based material into a packed column from a first end thereof by purging with a carrier gas under a first separation condition for enrichment and separation, to obtain a first effluent comprising saturated hydrocarbon from a second end of the packed column; and
S2) purging the packed column from the second end thereof with the carrier gas under a second separation condition, to obtain a second effluent comprising unsaturated hydrocarbon from the first end of the packed column;
wherein the packed column is a packed column according to any one of claims 7-9 and the temperature of the first separation condition is lower than the temperature of the second separation condition.

15. The method according to claim 14, wherein:

the first separation condition include: a temperature of about 80-180 °C, and a flow rate of the carrier gas of about 1-60 mL/min; the second separation condition include: a temperature of about 190-400 °C, and a flow rate of the carrier gas of about 1-60 mL/min; the difference between the temperature of the second separation condition and the temperature of the first separation condition is about 20-200 °C; preferably, the method further comprises maintaining the first separation condition for about 1-50 minutes to perform step S1, then increasing the temperature to about 190-400 °C and maintaining for about 0.5-10 minutes to perform step S2, wherein the temperature raising rate is about 10-250 °C/min;
the carrier gas is at least one of helium, nitrogen and hydrogen; and/or
the sample amount of the hydrocarbon-based material is about 0.01-2 $\mu$L.

16. The method according to any one of claims 14-15, further comprising the steps of:

S3) after step S1, passing the first effluent to a gas chromatograph and a mass spectrometer to separate and analyze saturated hydrocarbon component therein; and/or,
S4) after step S2, passing the second effluent to a gas chromatograph and a mass spectrometer to separate and analyze unsaturated hydrocarbon component therein;
preferably:

the mass ratio of the portion of the first effluent entering the gas chromatograph and the portion of the first effluent entering the mass spectrometer is about (1-40) : 1;
the mass ratio of the portion of the second effluent entering the gas chromatograph to the portion of the second effluent entering the mass spectrometer is about (1-40) : 1;
the operating conditions of the gas chromatograph include: a temperature of the flame ionization detector of about 200-350 °C; and/or
the operating conditions of the mass spectrometer include: a temperature of the mass spectrometer transmission line of about 180-400 °C.

17. The method according to claim 16, further comprising: connecting the packed column between a sample inlet and an analysis module of a gas chromatograph-mass spectrometer, and passing the hydrocarbon-based material sequentially through the sample inlet, the packed column and the analysis module.

18. The method according to claim 17, further comprising: connecting the packed column into the gas chromatograph-mass spectrometer via a multi-port valve, which is configured to have a first working position and a second working position; and

in a first working position, the first end of the packed column is in communication with the sample inlet through at least one working port of the multi-port valve, and the second end of the packed column is in communication with the inlet of the analysis module through at least one working port, so that at least a portion of the hydrocarbon-based material flows in the packed column from the first end to the second end;

in a second working position, the second end of the packed column is in communication with the sample inlet through at least one working port, and the first end of the packed column is in communication with the inlet of the analysis module through at least one working port, so that at least a portion of the hydrocarbon-based material flows in the packed column from the second end to the first end;

wherein under the first separation condition, the multi-port valve is set in the first working position; while under the second separation condition, the multi-port valve is set in the second working position.

19. The method according to claim 17, further comprising: connecting the packed column into the gas chromatograph-mass spectrometer via a multi-port valve set having a first multi-port valve and a second multi-port valve, wherein the multi-port valve set is configured to have a switch-in working position for connecting the packed column into the system and a switch-out working position for removing the packed column out from the system;

in the switch-in working position, the sample inlet, the first multi-port valve, the packed column connected through the second multi-port valve and the inlet of the analysis module are communicated sequentially, so that at least a portion of the hydrocarbon-based material sequentially flows through the sample inlet, the first multi-port valve, the packed column and the analysis module;

in the switch-out working position, the packed column is not connected into the system, and the sample inlet, the first multi-port valve, the second multi-port valve and the inlet of the analysis module are communicated sequentially, so that at least a portion of the hydrocarbon-based material sequentially flows through the sample inlet, the first multi-port valve, the second multi-port valve and the analysis module;

wherein under the first and second separated conditions, the multi-port valve is set in the switch-in working position; and after the first effluent and the second effluent enter the analysis module for separation and analysis, the multi-port valve is set in the switch-out working position.

20. The method according to any one of claims 14-19, wherein the hydrocarbon-based material is a hydrocarbon fuel, preferably selected from the group consisting of aviation fuel, diesel fuel or a combination thereof; and the unsaturated hydrocarbon is selected from the group consisting of aromatic hydrocarbons, olefins, or combinations thereof, preferably aromatic hydrocarbons.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Abundance

Fig. 15

Abundance

Fig. 16

Abundance

Fig. 17

Abundance

Fig. 18

Abundance

Fig. 19

Abundance

Fig. 20

Abundance

Fig. 21

Abundance

Fig. 22

Abundance

Fig. 23

Abundance

Fig. 24

Abundance

Time -->

Fig. 25

Abundance

Time -->

Fig. 26

Abundance

Fig. 27

Abundance

Fig. 28

Abundance

Fig. 29

Abundance

Fig. 30

Fig. 31

Fig. 32

## INTERNATIONAL SEARCH REPORT

| International application No. |
|---|
| **PCT/CN2020/092590** |

**A. CLASSIFICATION OF SUBJECT MATTER**

B01J 20/08(2006.01)i; B01J 20/14(2006.01)i; B01J 20/16(2006.01)i; B01D 15/22(2006.01)i; G01N 3/02(2006.01)i

According to International Patent Classification (IPC) or to both national classification and IPC

**B. FIELDS SEARCHED**

Minimum documentation searched (classification system followed by classification symbols)

B01J, B01D, G01N

Documentation searched other than minimum documentation to the extent that such documents are included in the fields searched

Electronic data base consulted during the international search (name of data base and, where practicable, search terms used)

CNABS, CNTXT, VEN, CNKI: 富集, 吸附, 吸收, 活性, Cu, 铜, Ag, 银, 金属, 助剂, 载体, copper, silver, enrich+, adsorp+, absorp+, activat+, metal, agent, carrier

**C. DOCUMENTS CONSIDERED TO BE RELEVANT**

| Category* | Citation of document, with indication, where appropriate, of the relevant passages | Relevant to claim No. |
|---|---|---|
| X | CN 104383874 A (SHANGHAI LYUQIANG NEW MATERIALS CO., LTD. et al.) 04 March 2015 (2015-03-04)<br>description paragraphs 0011-0028, 0050 | 1-20 |
| X | CN 105289477 A (SHANGHAI LYUQIANG NEW MATERIALS CO., LTD. et al.) 03 February 2016 (2016-02-03)<br>description paragraphs 0012-0024, 0069 | 1-20 |
| A | CN 1521506 A (SINOPEC CORP. et al.) 18 August 2004 (2004-08-18)<br>entire document | 1-20 |
| A | US 2013146542 A1 (UNIV BROWN) 13 June 2013 (2013-06-13)<br>entire document | 1-20 |
| A | JP 2005017046 A (PENTAX CORP et al.) 20 January 2005 (2005-01-20)<br>entire document | 1-20 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 August 2020** | **27 August 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| CN | 104383874 | A | 04 March 2015 | None | | | |
| CN | 105289477 | A | 03 February 2016 | CN | 105289477 | B | 06 July 2018 |
| CN | 1521506 | A | 18 August 2004 | CN | 1226620 | C | 09 November 2005 |
| US | 2013146542 | A1 | 13 June 2013 | WO | 2012019134 | A2 | 09 February 2012 |
| | | | | WO | 2012019134 | A3 | 31 May 2012 |
| | | | | US | 10549255 | B2 | 04 February 2020 |
| | | | | EP | 2600967 | A4 | 26 July 2017 |
| | | | | EP | 2600967 | A2 | 12 June 2013 |
| | | | | US | 2020147582 | A1 | 14 May 2020 |
| JP | 2005017046 | A | 20 January 2005 | JP | 3723806 | B2 | 07 December 2005 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910471632
- CN 201910472985
- CN 201910472990